# FASCICULE DE BREVET EUROPEEN

(11) **EP 1 261 603 B1**
(45) Date de publication et mention de la délivrance du brevet: **16.08.2006**
(21) Numéro de dépôt: 01909906.8
(22) Date de dépôt: 27.02.2001
(51) Int. Cl.: C07D 405/04, C07D 409/14, C07D 405/14, C07D 413/14, A61K 31/453, A61P 35/00

(54) **DERIVES DE FLAVONES, LEUR PROCEDE DE PREPARATION, LEUR APPLICATION A TITRE DE MEDICAMENTS**
FLAVON DERIVATE, VERFAHREN ZU DEREN HERSTELLUNG UND DEREN VERWENDUNG ALS HEILMITTEL
FLAVONE DERIVATIVES, PREPARATION METHOD AND USE AS MEDICINES

(30) Priorité: 29.02.2000 FR 0002528
(43) Date de publication de la demande: 04.12.2002
(73) Titulaire: Aventis Pharma S.A., 92160 Antony (FR)
(72) Inventeur: HAESSLEIN, Jean-Luc, F-77181 Courtry (FR); LEFRANCOIS, Dominique, F-93340 Le Raincy (FR); URIDAT, Eric, F-69480 Pommiers (FR); ZHANG, Jidong, F-75013 Paris (FR)
(74) Mandataire: Vieillefosse, Jean-Claude
(86) Numéro de dépôt international: PCT/FR2001/000561
(87) Numéro de publication internationale: WO 2001/064673

(56) Documents cités:
- EP-A- 0 241 003
- EP-A- 0 366 061
- H.H. SEDLACEK ET AL.: "FLAVOPIRIDOL" INTERNAT. JOURNAL OF ONCOLOGY, vol. 9, 1996, pages 1143-68, XP002103774 USA
- CHEMICAL ABSTRACTS, vol. 130, no. 8, 1999 Columbus, Ohio, US; abstract no. 90187w, V. PATEL ET AL.: "FLAVOPIRIDOL" page 38; colonne 2; XP002154064 & J. CLIN. INVEST., vol. 102, no. 9, 1998, pages 1674-81, USA

## Description

La présente invention concerne de nouveaux dérivés de flavones, leur procédé de préparation, nouvelle méthode de déprotection des éthers méthyliques, les nouveaux intermédiaires obtenus, leur application à titre de médicaments, les compositions pharmaceutiques les renfermant et la nouvelle utilisation de tels dérivés de flavones. L'invention a ainsi pour objet de nouveaux dérivés de flavones possédant des propriétés anti prolifératives et notamment des dérivés de flavones dotés d'un effet inhibiteur vis-à-vis des protéines kinases cycline-dépendantes soit 'cdk' en abrégé que nous utiliserons dans la suite du texte. L'étude des mécanismes moléculaires qui contrôlent le cycle cellulaire a permis de mettre en évidence le rôle régulateur des cdk ainsi définies. Les cdk sont des protéines constituées d'au moins deux sous-unités, une sous-unité catalytique (dont cdk2 est le prototype) et une sous-unité régulatrice (cycline). On connaît ainsi un certain nombre de cdk. Les cdk forment donc des complexes protéiques dont chacun est impliqué dans une phase du cycle cellulaire. De nombreux documents de la littérature décrivent l'existence et le rôle des cdk et à titre d'exemple, on peut citer notamment le document WO 97/20842.

Le document EP-A-0 366 061 décrit des dérivés 4H-1-benzopyran-4-one utilisables pour le traitement de tumeurs.

Le document H.H. SEDLACEK ET AL. : 'FLAVOPIRIDOL' INTERNAT. JOURNAL OF ONCOLOGY, vol. 9, 1996, pages 1143-68 décrit le flavopiridol comme nouvel inhibiteur de kinase pour le traitement de tumeurs.

Le document EP-A-0 241 003 décrit des dérivés 4H-1-benzopyran-4-one comme analgésiques, immunosuppresseurs et anti-allergiques.
Plusieurs inhibiteurs de kinases ont été décrits comme la butyrolactone, le flavopiridol et la 2(2-hydroxyéthylamino)-6-benzylamino-9-méthylpurine appelée olomoucine.

La présente invention a ainsi pour objet les produits de formule (I) : 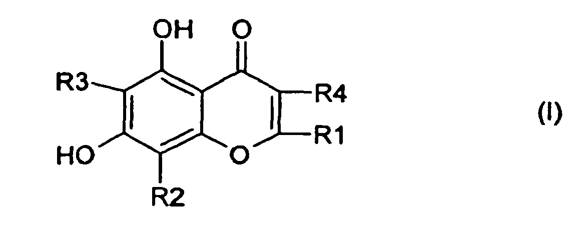 dans laquelle :
R2 et R3 sont tels que l'un représente un atome d'hydrogène et l'autre représente un radical pipéridinyle éventuellement substitué par un ou plusieurs radicaux hydroxyle et alkyle, R2 et R3 pouvant prendre alternativement les mêmes valeurs pour donner les isomères correspondants,
R4 représente un atome d'hydrogène, un radical alkyle ou phényle éventuellement substitués par un ou plusieurs atomes d'halogène,
R1 représente un radical phényle, éventuellement substitué par un ou plusieurs radicaux choisis parmi les atomes d'halogène ; le radical cyclohexyle ; cyano ; nitro ; hydroxyle ; carboxy libre, salifié ou estérifié ; tétrazolyle ; -NH2, -NH(alkyl), N(alkyl)(alkyl) ; SO2-NH-CO-NHR5 dans lequel R5 représente un radical alkyle ou phényle ; phényle ; alkyle, alcoxy ou phénoxy ; CF3 ; OCP3 ; pyrazolinyle lui-même éventuellement substitué par un ou plusieurs radicaux choisis parmi les radicaux alkyle et les atomes d'halogène, étant entendu que dans les radicaux ci-dessus, les radicaux alkyle et alcoxy sont linéaires ou ramifiés et renferment au plus 4 atomes de carbone,
caractérisé en ce que ces produits de formule (I) sont les suivants :
- le trifluoroacétate de 2-(2-chloro-4-fluorophényl)-5,7-dihydroxy-8-[(3S,4R)-3-hydroxy-1-méthyl-4-pipéridinyl]- 4H-benzopyran-4-one
- le trifluoroacétate de 2-(4-cyclohexylphényl)-5,7-dihydroxy-8-[(3S,4R)-3-hydroxy-1-méthyl-4-pipéridinyl]-4H-benzopyran-4-one
- le trifluoroacétate de 4-[5,7-dihydroxy-8-[(3S,4R)-3-hydroxy-1-méthyl-4-pipéridinyl]-4-axo-4H-benzopyran-2-yl]-benzonitrile
- le trifluoroacétate de 5,7-dihydroxy-8-[(3S,4R)-3-hydroxy-1-méthyl-4-pipéridinyl]-2-[4-(1H-tetrazol-5-yl)-phényl]-4H-benzopyran-4-one
- le trifluoroacétate de 5,7-dihydroxy-8-[(3S,4R)-3-hydroxy-1-méthyl-4-pipéridinyl]-2-[3-(phénoxy)-phényl]-4H-benzopyran-4-one
- le trifluoroacétate de 5,7-dihydroxy-6-[(3S,4R)-3-hydroxy-1-méthyl-4-pipéridinyl] -2- [3-(phénoxy) -phényl] -4H-benzopyran-4-one
- le 5,7-dihydroxy-8-[(3S,4R)-3-hydroxy-1-méthyl-4-pipéridinyl]-2-(3-nitrophényl)-4H-benzopyran-4-one
- le trifluoroacétate de 5,7-dihydroxy-2-[4-fluoro-3-(trifluorométhyl)phényl]-8-[(3S,4R)-3-hydroxy-1-méthyl-4-pipéridinyl]- 4H-benzopyran-4-one
- le trifluoroacétate de l'acide 4-[8-[(3S,4R)-3-acétyloxy-1-méthyl-4-pipéridinyl]- 5,7-dihydroxy-4-oxo-4H-benzopyran-2-yl]-2,5-dichloro-benzoique
- le chlorhydrate de 2,5-dichloro-4-[5,7-dihydroxy-8-[(3S,4R)-3-hydroxy-1-méthyl-4-pipéridinyl)-4-oxo-4H-benzopyran-2-yl]-benzoate de méthyle
- le trifluoroacétate de 2-[4-(diéthylamino)-phényl]-5,7-dihydroxy-8-[(3S,4R)-3-hydroxy-1-méthyl-4-pipéridinyl]- 4H-benzopyran-4-one
- le trifluoroacétate de 5,7-dihydroxy-8-[(3S,4R)-3-hydroxy-1-méthyl-4-pipéridinyl]-2-[3-(trifluorométhoxy)-phényl]-4H-benzopyran-4-one
- le trifluoroacétate de 2-[3,5-bis(trifluorométhyl)-phényl]-5,7-dihydroxy-8-[(3S,4R)-3-hydroxy-1-méthyl-4-pipéridinyl]- 4H-benzopyran-4-one
- le trifluoroacétate de 5,7-dihydroxy-8-[(3S,4R)-3-hydroxy-1-méthyl-4-pipéridinyl]-2-[4-(trifluorométhoxy)-phényl]-4H-benzopyran-4-one
- le trifluoroacétate de l'acide 4-[5,7-dihydroxy-8-[(3S,4R)-3-hydroxy-1-méthyl-4-pipéridinyl]-4-oxo-4H-benzopyran-2-yl]-benzoique
- le trifluoroacétate de 2-E3-[(4-bromo-3,5-diméthyl-1H-pyrazol-1-yl)méthyl]-phényl]-5,7-dihydroxy-8-[(3S,4R)-3-hydroxy-1-méthyl-4-pipéridinyl]-4H-benzopyran-4-one
- le trifluoroacétate de 5,7-dihydroxy-2-[3-[(3,5-diméthyl-1H-pyrazol-1-yl)méthyl]-phényl]-8-[(3S,4R)-3-hydroxy-1-méthyl-4-pipéridinyl]-4H-benzopyran-4-one
- le trifluoroacétate de 2-[2,5-bis(2,2,2-trifluoroéthoxy)-phényl]-5,7-dihydroxy-8-[(3S,4R)-3-hydroxy-1-méthyl-4-pipéridinyl]-4H-benzopyran-4-one
- le trifluoroacétate de 2-(2-chlorophényl)-5,7-dihydroxy-6-[(3S,9R)-3-hydroxy-1-méthyl-4-pipéridinyl)-4H-benzopyran-4-one
lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).

Dans les produits de formule (I) et dans ce qui suit :
- le terme radical alkyle linéaire ou ramifié désigne les radicaux méthyle, éthyle, propyle, isopropyle, butyle, isobutyle, sec-butyle, tert-butyle, pentyle, isopentyle, hexyle, isohexyle et également heptyle, octyle, nonyle et décyle ainsi que leurs isomères de position linéaires ou ramifiés,
- le terme radical alcoxy linéaire ou ramifié désigne les radicaux méthoxy, éthoxy, propoxy, isopropoxy, butoxy linéaire, secondaire ou tertiaire, pentoxy ou hexoxy ainsi que leurs isomères de position linéaires ou ramifiés
- le terme atome d'halogène désigne les atomes de chlore, de brome, d'iode ou de fluor et de préférence l'atome de chlore, de brome ou de fluor,
- les termes NH(alk) et N(alk)(alk) désigne un radical amino substitués respectivement par un ou deux radicaux alkyle, de tels radicaux alkyle étant linéaires ou ramifiés et renfermant de préférence au plus 4 atomes de carbone
- le terme acylamino désigne les radicaux -C(O)-NH2, -C(O)-NH(alk) et -C(O)-N(alk) (alk) : dans ces radicaux, NH(alk) et N(alk) (alk) ont les significations indiquées ci-dessus.

Le ou les radicaux carboxy des produits de formule (I) peuvent être salifiés ou estérifiés par les groupements divers connus de l'homme du métier parmi lesquels on peut citer, par exemple :
- parmi les composés de salification, des bases minérales telles que, par exemple, un équivalent de sodium, de potassium, de lithium, de calcium, de magnésium ou d'ammonium ou des bases organiques telles que, par exemple, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaine, la lysine, l'arginine; l'histidine, la N-méthylglucamine,
- parmi les composés d'estérification, les radicaux alkyle pour former des groupes alcoxy carbonyle tel que, par exemple, méthoxycarbonyle, éthoxycarbonyle, tert-butoxycarbonyle ou benzyloxycarbonyle, ces radicaux alkyles pouvant être substitués par des radicaux choisis par exemple parmi les atomes d'halogène, les radicaux hydroxyle, alcoxy, acyle, acyloxy, alkylthio, amino ou aryle comme, par exemple, dans les groupements chlorométhyle, hydroxypropyle, méthoxyméthyle, propionyloxyméthyle, méthylthiométhyle, diméthylaminoéthyle, benzyle ou phénéthyle.

Les sels d'addition avec les acides minéraux ou organiques des produits de formule (I) peuvent être, par exemple, les sels formés avec les acides chlorhydrique, bromhydrique, iodhydrique, nitrique, sulfurique, phosphorique, propionique, acétique, trifluoroacétique, formique, benzoïque, maléique, fumarique, succinique, tartrique, citrique, oxalique, glyoxylique, aspartique, ascorbique, les acides alcoylmonosulfoniques tels que par exemple l'acide méthanesulfonique, l'acide éthanesulfonique, l'acide propanesulfonique, les acides alcoyldisulfoniques tels que par exemple l'acide méthanedisulfonique, l'acide alpha, bêta-éthanedisulfonique, les acides arylmonosulfoniques tels que l'acide benzènesulfonique et les acides aryldisulfoniques.

On peut rappeler que la stéréoisomérie peut être définie dans son sens large comme l'isomérie de composés ayant mêmes formules développées, mais dont les différents groupes sont disposés différemment dans l'espace, tels que notamment dans des cyclohexanes monosubstitués dont le substituant peut être en position axiale ou équatoriale, et les différentes conformations rotationnelles possibles des dérivés de l'éthane. Cependant, il existe un autre type de stéréoisomérie, dû aux arrangements spatiaux différents de substituants fixés, soit sur des doubles liaisons, soit sur des cycles, que l'on appelle souvent isomérie géométrique ou isomérie cis-trans. Le terme stéréoisomères est utilisé dans la présente demande dans son sens le plus large et concerne donc l'ensemble des composés indiqués ci-dessus.

La présente invention a tout particulièrement pour objet les produits de formule (I) telle que définie ci-dessus, dans laquelle R1 a la signification indiquée ci-dessus, R2 et R3 sont tels que l'un représente l'atome d'hydrogène et l'autre représente un radical pipéridinyle éventuellement substitué par un radical hydroxyle sur un chaînon carboné et un radical alkyle sur l'atome d'azote et R4 représente un atome d'hydrogène,
lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).

La présente invention a encore plus particulièrement pour objet les produits de formule (I) telle que définie ci-dessus, répondant aux formules suivantes :
- le trifluoroacétate de 2-(2-chloro-4-fluorophényl)-5,7-dihydroxy-8-[(3S,4R)-3-hydroxy-1-méthyl-4-piperidinyl]-4H-benzopyran-4-one
- le trifluoroacétate de 2-(2,5-dichloro-3-thiényl)-5,7-dihydroxy-8-[(3S,4R)-3-hydroxy-1-méthyl-4-piperidinyl]-4H-benzopyran-4-one
- le trifluoroacétate de 5,7-dihydroxy-8-[(3S,4R)-3-hydroxy-1-méthyl-4-piperidinyl]-2- (5-méthyl-3-isoxazolyl) -4H-benzopyran-4-one
- le trifluoroacétate de 5,7-dihydroxy-8-[(3S,4R)-3-hydroxy-1-méthyl-4-piperidinyl] -2- [5- (trifluorométhyl) -1-phényl-1H-pyrazol-4-yl]- 4H-benzopyran-4-one
- le trifluoroacétate de 5,7-dihydroxy-6-[(3S,4R)-3-hydroxy-1-méthyl-4-piperidinyl]-2-[3-(phénoxy)-phényl]-4H-benzopyran-4-one

La présente invention a encore pour objet un procédé de préparation des produits de formule (I), telle que définie ci-dessus, caractérisé en ce que l'on soumet le composé de formule (II) : 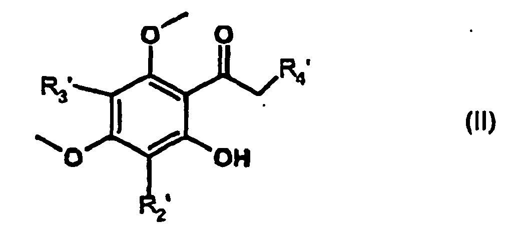 dans laquelle R2', R3' et R4' ont les significations indiquées ci-dessus respectivement pour R2, R3 et R4, dans lesquelles les éventuelles fonctions réactives sont éventuellement protégées par des groupements protecteurs. à une réaction avec un composé de formule (III) :

R1'COX (III)

dans laquelle R1' a la signification indiquée ci-dessus pour R¹, dans laquelle les éventuelles fonctions réactives sont éventuellement protégées par des groupements protecteurs et X représente un atome d'halogène ou un radical alcoxy renfermant au plus 6 atomes de carbone, pour obtenir le produit de formule (IV) : 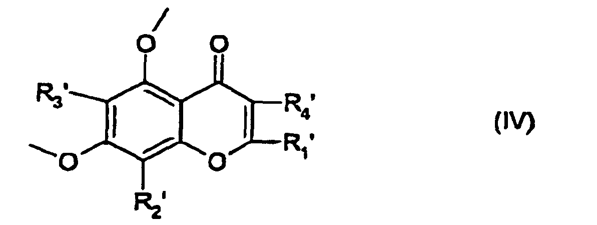 dans laquelle R1', R2', R3' et R4' ont les significations indiquées ci-dessus,
produit de formule (IV) que l'on soumet à une réaction de déprotection des radicaux hydroxyle
pour obtenir un produit de formule (I') 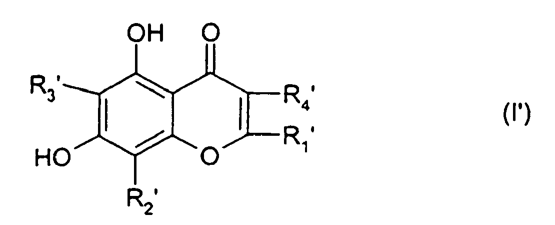 dans laquelle R1', R2', R3' et R4' ont les significations indiquées ci-dessus,
produits de formule (I') qui peuvent être des produits de formule (I) et que, pour obtenir des ou d'autres produits de formule (I), l'on peut soumettre, si désiré et si nécessaire, à l'une ou plusieurs des réactions de transformations suivantes, dans un ordre quelconque :
a) une réaction d'estérification de fonction acide,
b) une réaction de saponification de fonction ester en fonction acide,
c) une réaction d'oxydation de groupement alkylthio en sulfoxyde ou sulfone correspondant,
d) une réaction de transformation de fonction cétone en fonction oxime,
e) une réaction de réduction de la fonction carboxy libre ou estérifié en fonction alcool,
f) une réaction de transformation de fonction alcoxy en fonction hydroxyle, ou encore de fonction hydroxyle en fonction alcoxy,
g) une réaction d'oxydation de fonction alcool en fonction aldéhyde, acide ou cétone,
h) une réaction de transformation de radical nitrile en tétrazolyle,
i) une réaction d'élimination des groupements protecteurs que peuvent porter les fonctions réactives protégées,
j) une réaction de salification par un acide minéral ou organique ou par une base pour obtenir le sel correspondant,
k) une réaction de dédoublement des formes racémiques en produits dédoublés,
lesdits produits de formule (I) ainsi obtenus étant sous toutes les formes isomères possibles racémiques, énantiomères et diastéréoisomères.

On peut noter que de telles réactions de transformation de substituants en d'autres substituants peuvent également être effectuées sur les produits de départ ainsi que sur les intermédiaires tels que définis ci-dessus avant de poursuivre la synthèse selon les réactions indiquées dans le procédé décrit ci-dessus.

La présente invention a notamment pour objet un procédé de déprotection des fonctions hydroxyle d'un produit de formule (IV) telle que définie ci-dessus : 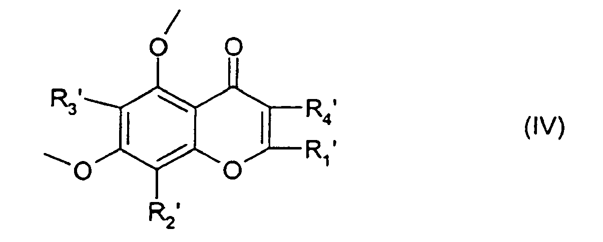 dans laquelle R1', R2', R3' et R4' ont les significations indiquées ci-dessus,
caractérisé en ce que l'on soumet le produit de formule (IV) soit à l'action de l'acide iodhydrique supporté par une résine dans un solvant polaire anhydre,
soit à l'action d'un sel de l'acide iodhydrique tel que HI-Py dans un solvant polaire anhydre,
soit à l'action de l'acide iodhydrique concentré, pour obtenir un produit de formule (I') : 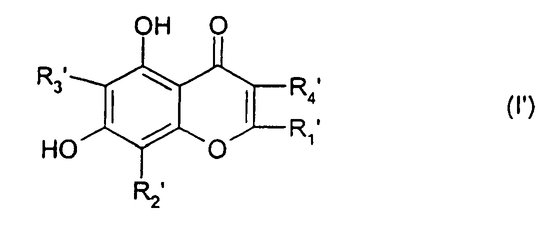 dans laquelle R1', R2', R3' et R4' ont les significations indiquées ci-dessus,
produits de formule (I') qui peuvent être des produits de formule (I) et que, pour obtenir des ou d'autres produits de formule (I), l'on peut soumettre, si désiré et si nécessaire, à l'une ou plusieurs des réactions de transformations suivantes, dans un ordre quelconque :
a) une réaction d'estérification de fonction acide,
b) une réaction de saponification de fonction ester en fonction acide,
c) une réaction d'oxydation de groupement alkylthio en sulfoxyde ou sulfone correspondant, correspondant,
d) une réaction de transformation de fonction cétone en fonction oxime,
e) une réaction de réduction de la fonction carboxy libre ou estérifié en fonction alcool,
f) une réaction de transformation de fonction alcoxy en fonction hydroxyle, ou encore de fonction hydroxyle en fonction alcoxy,
g) une réaction d'oxydation de fonction alcool en fonction aldéhyde, acide ou cétone,
h) une réaction de transformation de radical nitrile en tétrazolyle,
i) une réaction d'élimination des groupements protecteurs que peuvent porter les fonctions réactives protégées,
j) une réaction de salification par un acide minéral ou organique ou par une base pour obtenir le sel correspondant,
k) une réaction de dédoublement des formes racémiques en produits dédoublés,
lesdits produits de formule (I) ainsi obtenus étant sous toutes les formes isomères possibles racémiques, énantiomères et diastéréoisomères.

Dans le procédé indiqué ci-dessus, on soumet le produit de formule (IV) à l'action de l'acide iodhydrique supporté par une résine telle que par exemple la Reillex-pyridine TM -402 polymer (poly 4-vinylpyridine) ou encore la poly-2-vinylpyridine.

Dans des conditions préférentielles de mise en oeuvre de l'invention, le procédé décrit ci-dessus peut-être réalisé comme indiqué sur le schéma de la figure 1 décrit ci-après : un tel procédé peut ainsi être réalisé de la façon suivante : La réaction du produit de formule (II) avec un produit de formule (III) pour donner un produit de formule (IV) peut être réalisée notamment selon quatre méthodes différentes dites A1, B1, C1 et D1 :
Les trois premières utilisent le produit de formule (III) dans laquelle X représente un radical alcoxy et peuvent être réalisées de la façon suivante :
pour A1 le produit de formule (II) est d'abord soumis 1) à l'action de KOtBu (terbutylate de potassium) dans un solvant tel que le DMF, puis 2) à l'action du composé de formule (III) et enfin 3) à l'action d'acide chlorhydrique concentré pour B1 le produit de formule (II) est d'abord soumis 1) à l'action d'une base telle que l'hydrure de sodium NaH dans un solvant tel que le DMSO ou le DMF, puis 2) à l'action du composé de formule (III) et enfin 3) à l'action d'acide chlorhydrique concentré
pour C1 le produit de formule (II) est d'abord soumis 1) à l'action d'une base telle que l'hydrure de sodium NaH dans un solvant tel que le THF et en présence d'éthanol en quantité catalytique (2 gouttes) et de l'éther de couronne dibenzo-18-C-6, puis 2) à l'action du composé de formule (III) et enfin 3) à l'action d'acide chlorhydrique concentré.

La quatrième méthode D1 utilise le produit de formule (III) dans laquelle X représente un atome d'halogène et peut être réalisée de la façon suivante :
le produit de formule (II) est 1) d'abord soumis à l'action d'une base telle que l'hydrure de sodium NaH dans un solvant tel que le THF ou encore le DMF, puis 2) à l'action du composé de formule (III) dans lequel X représente un atome d'halogène ensuite 3) de nouveau à l'action d'une base telle que l'hydrure de sodium NaH dans un solvant tel que le THF ou encore le DMF et enfin 4) à l'action de l'acide chlorhydrique concentré dans l'acide acétique (en proportion de 1 : 10).

Le produit de formule (IV) ainsi obtenu peut être soumis à une réaction de déprotection des radicaux hydroxyle pour donner un produit de formule (I') notamment selon trois méthodes différentes dites A2, B2 et C2 :
- pour A2 le produit de formule (IV) est soumis à l'action de l'acide iodhydrique concentré à 130°C pendant une heure
- pour B2 le produit de formule (IV) est soumis, à 130°C dans un solvant tel que le DMF ou encore le DMA pendant 12 à 48 heures, à l'action de l'acide iodhydrique supporté par une résine comme par exemple la résine Reillex-pyridine TM-402 préparée comme indiqué dans la partie expérimentale,
- pour C2 le produit de formule (IV) est soumis à l'action d'un sel d'acide iodhydrique tel que HI-Py (pyridine iodhydrate) à 130°C dans un solvant tel que le DMA ou le DMF pendant 3 à 12 heures.

Les produits de formule (IV) peuvent donc constituer des produits de formule (I) dans laquelle les deux fonctions hydroxyle sont protégées et qui donnent donc après réaction selon A2, B2 ou C2 un produit de formule (I) telle que définie ci-dessus.

Les produits de formule (IV) peuvent également constituer des produits de formule (I) dans laquelle les deux fonctions hydroxyle sont protégées mais également d'autres fonctions éventuellement réactives peuvent être protégées : les produits de formule (IV), après déprotection des radicaux hydroxyle après réaction selon A2, B2 ou C2, peuvent constituer alors des produits de formule (I') dans laquelle des fonctions éventuellement réactives peuvent être protégées.

Selon les valeurs de R1', R2', R3' et R4', les produits de formule (I') constituent ou non des produits de formule (I) et peuvent donner des produits de formule (I), ou être transformés en d'autres produits de formule (I) en étant soumis à une ou plusieurs des réactions a) à k) indiquées ci-dessus.

Ainsi les diverses fonctions réactives que peuvent porter certains composés des réactions définies ci-dessus peuvent, si nécessaire, être protégées : il s'agit par exemple des radicaux hydroxyle, acyle, carboxy libres ou encore amino et monoalkylamino qui peuvent être protégés par les groupements protecteurs appropriés.

La liste suivante, non exhaustive, d'exemples de protection de fonctions réactives peut être citée :
- les groupements hydroxyle peuvent être protégés par exemple par les radicaux alkyle tels que tert-butyle, triméthylsilyle, tert-butyldiméthylsilyle, méthoxyméthyle, tétrahydropyrannyle, benzyle ou acétyle,
- les groupements amino peuvent être protégés par exemple par les radicaux acétyle, trityle, benzyle, tert-butoxycarbonyle, benzyloxycarbonyle, phtalimido ou d'autres radicaux connus dans la chimie des peptides,
- les groupements acyles tel que le groupement formyle peuvent être protégés par exemple sous forme de cétals ou de thiocétals cycliques ou non cycliques tels que le diméthyl ou diéthylcétal ou l'éthylène dioxycétal, ou le diéthylthiocétal ou l'éthylènedithiocétal,
- les fonctions acide des produits décrits ci-dessus peuvent être, si désiré, amidifiées par une amine primaire ou secondaire par exemple dans du chlorure de méthylène en présence, par exemple, de chlorhydrate de 1-éthyl-3-(diméthylaminopropyl) carbodiimide à la température ambiante
- les fonctions acide peuvent être protégées par exemple sous forme d'esters formés avec les esters facilement clivables tels que les esters benzyliques ou ter butyliques ou des esters connus dans la chimie des peptides.

Les réactions auxquelles les produits de formules (IV) et (I') telles que définies ci-dessus peuvent être soumis, si désiré ou si nécessaire, peuvent être réalisées, par exemple, comme indiqué ci-après.
a) Les produits décrits ci-dessus peuvent, si désiré, faire l'objet, sur les éventuelles fonctions carboxy, de réactions d'estérification qui peuvent être réalisées selon les méthodes usuelles connues de l'homme du métier.
b) Les éventuelles transformations de fonctions ester en fonction acide des produits décrits ci-dessus peuvent être, si désiré, réalisées dans les conditions usuelles connues de l'homme du métier notamment par hydrolyse acide ou alcaline par exemple par de la soude ou de la potasse en milieu alcoolique tel que, par exemple, dans du méthanol ou encore par de l'acide chlorhydrique ou sulfurique.
c) Les éventuels groupements alkylthio des produits décrits ci-dessus peuvent être, si désiré, transformés en les fonctions sulfoxyde ou sulfone correspondantes dans les conditions usuelles connues de l'homme du métier telles que par exemple par les peracides comme par exemple l'acide peracétique ou l'acide métachloroperbenzoïque ou encore par l'ozone, l'oxone, le périodate de sodium dans un solvant tel que par exemple le chlorure de méthylène ou le dioxanne à la température ambiante.
   L'obtention de la fonction sulfoxyde peut être favorisée par un mélange équimolaire du produit renfermant un groupement alkylthio et du réactif tel que notamment un peracide. L'obtention de la fonction sulfone peut être favorisée par un mélange du produit renfermant un groupement alkylthio avec un excès du réactif tel que notamment un peracide.
d) La réaction de transformation d'une fonction cétone en oxime peut être réalisée dans les conditions usuelles connues de l'homme de métier, telle que notamment une action en présence d'une hydroxylamine éventuellement O-substituée dans un alcool tel que par exemple l'éthanol, à température ambiante ou en chauffant.
e) Les éventuelles fonctions carboxy libre ou estérifié des produits décrits ci-dessus peuvent être, si désiré, réduites en fonction alcool par les méthodes connues de l'homme de métier : les éventuelles fonctions carboxy estérifié peuvent être, si désiré, réduites en fonction alcool par les méthodes connues de l'homme du métier et notamment par de l'hydrure de lithium et d'aluminium dans un solvant tel que par exemple le tétrahydrofuranne ou encore le dioxane ou l'éther éthylique. Les éventuelles fonctions carboxy libre des produits décrits ci-dessus peuvent être, si désiré, réduites en fonction alcool notamment par de l'hydrure de bore.
f) Les éventuelles fonctions alcoxy telles que notamment méthoxy des produits décrits ci-dessus peuvent être, si désiré, transformées en fonction hydroxyle dans les conditions usuelles connues de l'homme du métier et notamment selon les conditions décrites ci-après dans la partie expérimentale
g) Les éventuelles fonctions alcool des produits décrits ci-dessus peuvent être, si désiré, transformées en fonction aldéhyde ou acide par oxydation dans les conditions usuelles connues de l'homme du métier telles que par exemple par action de l'oxyde de manganèse pour obtenir les aldéhydes ou du réactif de Jones pour accéder aux acides.
h) Les éventuelles fonctions nitrile des produits décrits ci-dessus peuvent être, si désiré, transformées en tétrazolyle dans les conditions usuelles connues de l'homme du métier telles que par exemple par cycloaddition d'un azidure métallique tel que par exemple l'azidure de sodium ou un azidure de trialkylétain sur la fonction nitrile ainsi qu'il est indiqué dans la méthode décrite dans l'article référencé comme suit :
   J. Organometallic Chemistry., 33, 337 (1971) KOZIMA S.& coll. On peut noter que la réaction de transformation d'un carbamate en urée et notamment d'un sulfonylcarbamate en sulfonylurée, peut être réalisée par exemple au reflux d'un solvant comme par exemple le toluène en présence de l'amine adéquate. Il est entendu que les réactions décrites ci-dessus peuvent être effectuées comme indiqué ou encore, le cas échéant, selon d'autres méthodes usuelles connues de l'homme du métier.
i) L'élimination de groupements protecteurs tels que par exemple ceux indiqués ci-dessus peut être effectuée dans les conditions usuelles connues de l'homme de métier notamment par une hydrolyse acide effectuée avec un acide tel que l'acide chlorhydrique, benzène sulfonique ou para-toluène sulfonique, formique ou trifluoroacétique ou encore par une hydrogénation catalytique.
   Le groupement phtalimido peut être éliminé par l'hydrazine. On trouvera une liste de différents groupements protecteurs utilisables par exemple dans le brevet BF 2 499 995.
j) Les produits décrits ci-dessus peuvent, si désiré, faire l'objet de réactions de salification par exemple par un acide minéral ou organique ou par une base minérale ou organique selon les méthodes usuelles connues de l'homme du métier.
k) Les éventuelles formes optiquement actives des produits décrits ci-dessus peuvent être préparées par dédoublement des racémiques selon les méthodes usuelles connues de l'homme du métier.

Des illustrations de telles réactions définies ci-dessus sont données dans la préparation des exemples décrits ci-après. Les produits de formule (I) tels que définis ci-dessus ainsi que leurs sels d'addition avec les acides présentent d'intéressantes propriétés pharmacologiques.

Les produits de la présente invention tels que définis ci-dessus, possèdent des propriétés inhibitrices de kinases d'une grande sélectivité.

Les cdk jouent un rôle central dans l'initiation, le développement et l'achèvement des évènements du cycle cellulaire et ainsi, les molécules inhibitrices de cdk sont susceptibles de limiter des proliférations cellulaires non désirées telles que celles observées dans les cancers, psoriasis, croissance de champignons, de parasites (animaux, protistes) : de telles molécules inhibitrices de cdk sont ainsi également susceptibles d'intervenir dans la régulation de maladies neurodégénératives telles que la maladie d'alzheimer.

Des kinases particulièrement sensibles aux effets inhibiteurs des dérivés de la présente invention sont notamment les cdk1, cdk2, cdk4, cdk5 et cdk7.

Les produits de la présente invention sont donc doués de propriétés antimitotiques.

Les produits de la présente invention possèdent en plus de leurs propriétés inhibitrices spécifiques de kinases, des effets cellulaires intéressants tels que des propriétés antiprofilératives et notamment des effets sur l'apoptose. On sait par des travaux décrits dans la littérature tel que dans WO 97/20842, que des rapports existent entre le cycle cellulaire et l'apoptose. Parmi les voies conduisant à l'apoptose, certaines sont dépendantes de kinases.

Les produits de la présente invention sont notamment utiles pour la thérapie de tumeurs.

Les produits de l'invention peuvent également ainsi augmenter les effets thérapeutiques d'agents anti-tumoraux couramment utilisés.

Les produits de formule (I) de la présente invention possèdent donc tout particulièrement des propriétés antimitotiques et anti-neurodégénératives.

Ces propriétés justifient leur application en thérapeutique et l'invention a particulièrement pour objet à titre de médicaments, les produits de formule (I) telle que définie ci-dessus, lesdits produits de formule (I) étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de formule (I).

L'invention a ainsi plus particulièrement pour objet à titre de médicaments, les produits de formule (I) telle que définie ci-dessus , ainsi que les sels d'addition avec les acides minéraux et organiques ou avec les bases minérales et organiques pharmaceutiquement acceptables desdits produits de formule (I).

L'invention a tout particulièrement pour objet, à titre de médicaments, les produits décrits ci-après dans les exemples et notamment les produits de formule (I) telle que définie ci-dessus, répondant aux formules suivantes :
- le trifluoroacétate de 2-(2-chloro-4-fluorophényl)-5,7-dihydroxy-8-((3S,4R)-3-hydroxy-1-méthyl-4-piperidinyl)-4H-benzopyran-4-one
- le trifluoroacétate de 2-(2,5-dichloro-3-thiényl)-5,7-dihydroxy-8-[(3S,4R)-3-hydroxy-1-méthyl-4-piperidinyl]-4H-benzopyran-4-one
- le trifluoroacétate de 5,7-dihydroxy-8-((3S, 4R)-3-hydroxy-1-méthyl-4-piperidinyl]-2-(5-méthyl-3-isoxazolyl)-4H-benzopyran-4-one
- le trifluoroacétate de 5,7-dihydroxy-8-((3S, 4R)-3-hydroxy-1-méthyl-4-piperidinyl]-2-[5-(trifluorométhyl)-1-phényl-1H-pyrazol-4-yl]- 4H-benzopyran-4-one
- le trifluoroacétate de 5,7-dihydroxy-6-((3S, 4R)-3-hydroxy-1-méthyl-4-piperidinyl]-2-[3-(phénoxy)-phényl]-4H-benzopyran-4-one,
ainsi que les sels d'addition avec les acides minéraux et organiques ou avec les bases minérales et organiques pharmaceutiquement acceptables desdits produits de formule (I).

Les médicaments, objet de l'invention, trouvent, par exemple, comme antimitotiques, leur emploi dans la chimiothérapie des cancers, ou encore dans le traitement de psoriasis, de parasitoses telles que celles dues à des protistes ou à des champignons ou encore dans le traitement de la maladie d'Alzheimer ou dans le traitement de l'apoptose neuronale. L'invention s'étend aux compositions pharmaceutiques contenant à titre de principe actif l'un au moins des médicaments tels que définis ci-dessus.

De telles compositions pharmaceutiques de la présente invention peuvent également, le cas échéant, renfermer des principes actifs d'autres médicaments antimitotiques tels que notamment ceux à base de taxol, cis-platine, les agents intercalants de l'ADN et autres.

Ces compositions pharmaceutiques peuvent être administrées par voie buccale, par voie parentérale ou par voie locale en application topique sur la peau et les muqueuses ou par injection par voie intraveineuse ou intramusculaire.

Ces compositions peuvent être solides ou liquides et se présenter sous toutes les formes pharmaceutiques couramment utilisées en médecine humaine comme, par exemple, les comprimés simples ou dragéifiés, les pilules, les tablettes, les gélules, les gouttes, les granulés, les préparations injectables, les pommades, les crèmes ou les gels ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie usuelle, variable selon le produit utilisé, le sujet traité et l'affection en cause, peut être, par exemple, de 0,05 à 5 g par jour chez l'adulte, ou de préférence de 0,1 à 2 g par jour.

Parmi les produits de départ de formule (II), certains sont connus et peuvent être obtenus commercialement ou peuvent être préparés selon les méthodes usuelles connues de l'homme du métier.

Certains produits de départ de formule (II) peuvent être préparés comme indiqué dans la demande de brevet numéro FR9807677 : on peut citer notamment le produit de départ de formule (II) dans lequel R3 et R4 représentent tous deux un atome d'hydrogène et R2 représente le radical pipéridinyle substitué par un radical hydroxyle sur un chaînon carboné et méthyle sur l'atome d'azote un tel produit est notamment le 1-(2-hydroxy-4,6-diméthoxy-3-[(3S,4R)-3-hydroxy-1-méthyl-4-piperidinyl]-phényl]-éthanone nommé également (-)-cis-acétoflocino-pipéridinol ou encore Acétoflocinopipéridol préparé comme indiqué dans la demande de brevet numéro FR 9807677

Parmi les produits de départ de formule (III), certains sont connus et peuvent être obtenus commercialement ou peuvent être préparés selon les méthodes usuelles connues de l'homme du métier.

Parmi les produits de départ commerciaux de formule (III) dans laquelle X représente un radical alcoxy, on peut citer par exemple, les produits de formule (III) suivants :
le 2-chloro-4-fluorobenzoate de méthyle,
le 2-5-bis(2,2,2-trifluoroéthoxy)benzoate de méthyle,
le 2,5-dichloroterephtalate de diméthyle
Le 2,5-diméthylfuran-3-carboxylate de méthyle.

Parmi les produits de départ commerciaux de formule (III) dans laquelle X représente un atome d'halogène, on peut citer par exemple, les produits de formule (III) suivants :
le chlorure de 3-(trifluorométhoxy)benzoyle
le chlorure de 3-(trifluorométhyl)-4-(fluoro)benzoyle.

On peut encore notamment préparer certains produits de départ à partir de produits de commerciaux par exemple en les soumettant à une ou plusieurs des réactions décrites ci-dessus en a) à k), réalisées dans les conditions également décrites ci-dessus.

La partie expérimentale ci-après donne des exemples de tels produits de départ.

La présente invention a enfin pour objet à titre de produits industriels nouveaux, les produits de formule (IV) tels que définis ci-dessus.

L'invention a ainsi particulièrement pour objet les compositions pharmaceutiques contenant à titre de principe actif, l'un au moins des médicaments tels que définis ci-dessus.

L'invention a tout particulièrement pour objet les compositions pharmaceutiques telles que définies ci-dessus caractérisées en ce qu'elles sont utilisées comme médicaments antimitotiques, en particulier pour la chimiothérapie de cancers ou encore pour le traitement de psoriasis, de parasitoses telles que celles dues à des champignons ou à des protistes ou de la maladie d'Alzheimer.

L'invention a également tout particulièrement pour objet les compositions pharmaceutiques telles que définies ci-dessus caractérisées en ce qu'elles sont utilisées comme médicaments antineurodégénératifs notamment anti-apoptose neuronale.

La présente invention a notamment pour objet l'utilisation des produits de formule (I) telle que définie ci-dessus pour la préparation de médicaments destinés à la prévention ou au traitement de maladies liées à un dérèglement de la sécrétion et/ou de l'activité de protéines tyrosines kinases. L'invention a notamment pour objet l'utilisation des produits de formule (I) telle que définie ci-dessus pour la préparation de médicaments destinés à la chimiothérapie de cancers, au traitement de psoriasis, de parasitoses telles que celles dues à des champignons ou à des protistes, au traitement de la maladie d'Alzheimer ou au traitement d'affections neurodégénératives notamment l'apoptose neuronale.

Les exemples suivants illustrent l'invention sans toutefois la limiter.

### 1) Préparation du réactif Pyridine iodhydrate (Py-HI)

On introduit à TA 500 cm3 d'éther éthylique anhydre, 10 cm3 de pyridine (m=79.10) (d=0.978, 123.6 mmol, 1.2eq.), 13.5 cm3 d'HI (acide iodhydrique)(57 %) (M=127.91) (d=1.7, 102.3 mmol) goutte à goutte.

A la fin de l'introduction de l'HI, on élimine la phase éthérée par transvasement, rince le précipité pâteux à l'éther puis recristallise par 30 ml d'EtOH absolu au reflux. On obtient après filtration, et séchage sous vide le produit attendu sous forme de cristaux blancs. Poids : 16.87 g

### 2) Préparation du réactif Reillex-Py-HI

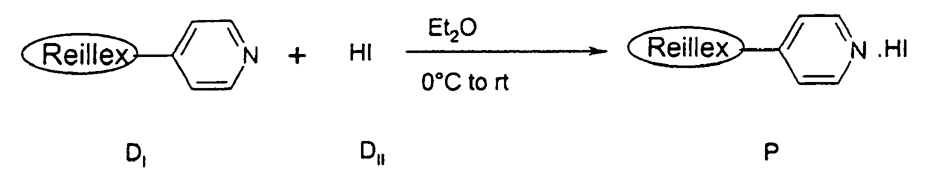

Les composés utilisés sont DI et DII définis comme suit :
DI : Reillex TM 402 polymer cross-linked with 2 % DVB -4-pyridine (-7 mmol/g).
DII : acide iodhydrique HI(57 %) d 1.70 (-7. 6M)

Dans 350 ml d'éther Et2O contenant 10 g (-70 mmol)de DI, on ajoute à 0°C 18.42 ml (-140 mmol)de DII sous argon. La température remonte lentement jusqu'à température ambiante et on laisse une nuit.

La solution obtenue est ensuite filtrée, la résine est alors lavée avec de l'éther (3x), éthanol (1x), puis de nouveau de l'éther (lx). Après passage sous vide, on obtient 21 g de produit attendu soit Reillex-Py.HI : le produit obtenu est tel que 11 g de HI (acide iodhydrique) sont fixés sur DI et la molarité du produit obtenu est donc : (11/128)/21 = 0.004 M/g = 4 mmol/g.

### Exemple 1 : trifluoroacétate de 2-(2-chloro-4-fluorophényl)-5,7-dihydroxy-8-[(3S,4R)-3-hydroxy-1-méthyl-4-piperidinyl]-4H-benzopyran-4-one

### Stade a) : 2-(2-chloro-4-fluorophény]-5,7-diméthoxy-8-[(3S,4R)-3-hydroxy-1-méthyl-4-piperidinyl]- 4H-benzopyran-4-one

### 1) Condensation : 1-(2-chloro-4-fluorophényl)-3-[2-hydroxy-4,6-diméthoxy-3-[(3S,4R)-3-hydroxy-1-méthyl-4-piperidinyl] phényl]-1,3-propanedione

Sous N2, on introduit 380 mg de NaH à 50 % dans l'huile (M=24) (8 mmol- 4 eq.) et 9.5 cm3 de DMSO/NK20, 618 mg de 1-[2-hydroxy-4,6-diméthoxy-3-[(3S, 4R)-3-hydroxy-1-méthyl-4-piperidinyl]-phényl]-éthanone appelé aussi Acétoflocinopipéridol (2 mmol) préparé comme indiqué dans la demande de brevet numéro FR 9807677. On agite 1h à température ambiante puis introduit 1.13 g de 2-chloro-4-fluoro-benzoate de méthyle (M= 188.59) (6 mmol-3 eq.) et maintient sous agitation pendant 20 heures à t. amb.

### 2) Cyclisation : 2-(2-chloro-4-fluorophény)-5,7-diméthoxy-8-[(3S,4R)-3-hydroxy-1-méthyl-4-piperidinyl]-4H-benzopyran-4-one

On ajoute au milieu réactionnel obtenu ci-dessus en 1), 3 cm3 de HCl à 36 % (36 mmol-18 eq.) et porte alors à 50°C pendant 2 h. On revient à température ambiante, verse dans l'eau glacée, puis on amène à pH basique par addition de soude concentrée, extrait par 3 fois 50 cm3 de CH2Cl2/MeOH : 9/1, lave à l'eau, sèche sur MgSO4 anhydre et porte à sec sous vide. On obtient 1.5 g d'une huile marron que l'on chromatographie sur silice 0,04-0,06 mm dans CH2Cl2/MeOH : 90/10. On isole le produit attendu sous forme de solide jaune de poids 90 mg.

### Analyses :

Spectre ¹H RMN : CDC13 δ (ppm)
1,57(dl), 3(masqué) : 2H ; 2,11 (t,1), 3,05(masqué) : 2H ; 2,28(m), 3,05(m) : 2H ; 2,37(s) : CH3-N ; 3,50(ddd) : 1H ; 3,95(s,1) : 1H(équatorial) ; 3,97(s), 4,00(s) : 6H ; 6,43(s) : 1H ; 6,46(s) : 1H ; 7,14(ddd) : 1H ; 7,30(dd) : 1H ; 7,60(dd) : 1H.

### Stade b) : trifluoroacétate de 2-(2-chloro-4-fluorophényl)-5,7-dihydroxy-8-[(3S,4R)-3-hydroxy-1-méthyl-4-piperidinyl]-4H-benzopyran-4-one

Dans un ballon de 30 cm3 avec réfrigérant à reflux, sous N2, on introduit 90 mg du diméthoxy (0.2 mmol) obtenu au stade a) ci-dessus, 1.8 cm3 de DMF anhydre et 550 mg de Reillex-pyridine- HI (≈ 4 mmol/g)(22 mmol-11 eq.) obtenu comme indiqué à la préparation 2 ci-dessus. On porte à 130°C pendant 24h et à 150°C pendant 4h. On revient ensuite à température ambiante, dilue par CH3CN, filtre sur Iéna, rince par CH3CN puis ajoute 1.5 g de résine PTBD (soit 1,5,7 Triazabicyclo-[4,4,0]dec-5-ene) (2.6 mmol/g) (4 mmol-20eq.). On agite 20h à température ambiante, filtre sur Iéna, lave par CH3CN puis décroche par 20 cm3 de CH3CN/TFA : 95/05 sous agitation. On filtre sur Iéna, rince par CH3CN/TFA : 95/05 puis porte à sec sous vide. On récupère 160 mg d'un produit amorphe orange que l'on reprend par 5 cm3 d'éluant HPLC décrit ci-après. On filtre sur toupie-0.45 µm puis injecte en 3 fois en HPLC préparative : Kromasil C18 - 10 µm - 500x22 mm - λ= 225 nm - 15 ml/min avec pour éluant : CH3CN/H2O/TFA : 35/65/0.1. On regroupe les fractions propres que l'on concentre sous vide puis lyophilise. On isole le produit attendu sous forme de solide jaune de poids 42 mg.

### Analyses :

Spectre ¹H RMN : DMSO D⁶ δ(ppm)
1,83(dl), 2,98(m) : 2H ; 3,08(m), 3,36(masqué) : 2H ; 3,29(m) : 2H ; 3,44(m) : 1H (axial) ; 2,72(d) : CH3-N ; 4,06(sl) : 1H (équatorial) ; 6,00 (s1) : OH ; 6,39(s) : 1H ; 6,60 (s) : 1H ; 7,47(ddd) : 1H ; 7,75 (dd) : 1H ; 7,92 (dd) : 1H ; 9,30 (s1) : N⁺R; 11,30(sl), 12,97 (s) : 2 OH.

### Exemple 2 : trifluoroacétate de 2-(2,5-dichloro-3-thiényl)-5,7-dihydroxy-8-[(3S,4R)-3-hydroxy-1-méthyl-4-piperidinyl]-4H-benzopyran-4-one

### Stade a) : 2-(2,5-dichloro-3-thiényl)-5,7-diméthoxy-8-[(3S,4R)-3-hydroxy-1-méthyl-4-piperidinyl]-4H-benzopyran-4-one

### 1) Condensation : 1-(2,5-dichloro-3-thiényl)-3-[2-hydroxy-4,6-diméthoxy-3-[(3S,4R)-3-hydroxy-1-méthyl-4-piperidinyl] phényl]-1,3-propanedione

Dans un ballon de 30 cm3, sous N2, on introduit 310 mg de NaH à 50 % dans l'huile (M=24) (6.5 mmol-4eq.), 10 cm3 de THF anhydre, 500 mg d'Acétoflocinopipéridol (1.6 mmol) par fractions, 2 gouttes d'EtOH et 12 mg de Dibenzo-18-Crown-6 (M=360.41) (0.03 mmol-2 % molaire). On agite 5 min à température ambiante, puis introduit 1.02 g de 2,5-dichloro-3-thiophénecarboxylate de méthyle (M=211.07) (4.8 mmol-3eq.) et porte à reflux pendant 4h30.

### 2) Cyclisation : 2-(2,5-dichloro-3-thiényl)-5,7-diméthoxy-8-[(3S,4R)-3-hydroxy-1-méthyl-4-piperidinyl)-4H-benzopyran-4-one

On ajoute au milieu réactionnel obtenu ci-dessus en 1), 10 cm3 de HCl à 36 % (116 mmol-72eq.) et maintient à reflux pendant 2h. On revient à température ambiante, verse dans 100 cm3 de NaOH(2N), extrait par 2x100 cm3 de CH2Cl2/MeOH 8/2, lave par une solution saturée en NaCl, sèche sur Na2SO4 anhydre et porte à sec sous vide. On obtient 0.76 g d'une résine noire que l'on chromatographie sur silice 0,04-0,06 mm dans CH2Cl2/MeOH : 80/20. On isole ainsi le produit attendu de poids : 147 mg.

### Analyses :

Spectre IR : Absorption région OH/NH
-C=O : 1644 cm-1 ; Système conjugué aromatique : 1594, 1567, 1538, 1493 cm-1.
Spectre ¹H RMN : DMSO D⁶ δ (ppm)
1,51(dl), 3,03(masqué) : 2H ; 2,11(t,1), 2,95(masqué) : 2H ; 2,27(masqué), 2,94 (masqué) : 2H ; 2,28(s) : CH3-N ; 3,36 (masqué) : 1H(axial) ; 3,76(s,l) : 1H(équatorial) ; 3, 89 (s), 3,93 (s) : 6H ; 4,49 (sl) : OH ; 6,66 (s) : 1H ; 6,51(s) : 1H ; 7, 76 (s) : 1H.

### Stade b) : trifluoroacétate de 2-(2,5-dichloro-3-thiényl)-5,7-dihydroxy-8-[(3S,4R)-3-hydroxy-1-méthyl-4-piperidinyl]-4H-benzopyran-4-one

Dans un ballon de 20 cm3 avec réfrigérant à reflux, sous N2, on introduit 115 mg du diméthoxy obtenu au stade a) ci-dessus (0.24 mmol), 2.4 cm3 de DMA et 506 mg de Pyridine-HI (M=207.01) (2.4 mmol-10 eq.) obtenu comme indiqué à la préparation 1 ci-dessus. On porte à 130°C pendant 18h. On revient ensuite à température ambiante, dilue par CH3CN, puis ajoute 1.9 g de résine PTBD (2.6 mmol/g) (4.9 mmol-20eq.) On agite 1h30 à température ambiante, filtre sur Iéna, lave par CH3CN puis décroche par 10 cm3 de CH3CN/TFA : 95/05 sous agitation. On filtre sur Iéna, rince par CH3CN/TFA : 95/05 puis porte à sec sous vide. On récupère 217 mg d'une résine marron que l'on reprend par 10 cm3 de MeOH et que l'on saponifie par 1 cm3 de NaOH (2N) (2 mmol-8eq.).
On porte alors à 60°C pendant 5h30 et revient à température ambiante 1 nuit. On porte à sec sous vide. On obtient 350 mg d'un solide marron que l'on reprend par 6.5 cm3 d'éluant HPLC décrit ci-après. On filtre sur toupie-0.45 *µ*m puis injecte en 4 fois en HPLC préparative : Kromasil C18 - 10 *µ*m - 500x22 mm - λ= 225 nm avec pour éluant : CH3CN/H2O/TFA : 40/60/0.1 - 15 ml/min. On regroupe les fractions propres que l'on concentre sous vide puis lyophilise. On isole le produit attendu sous forme de solide jaune de poids 28 mg.

### Analyses :

Spectre ¹H RMN : DMSO D⁶ δ (ppm)
1,83(d), 3,03(m) : 2H ; 2,79(s) : CH3-N ; 3,13(m), 3,42(masqué) : 2H ; 3,26(masqué), 3,36(masqué) : 2H ; 3,54(d) : 1H(axial) ; 4,13(s) : 1H(équatorial) ; 6,35(s) : 1H ; 6,73(s) : 1H ; 7,62(s) : 1H ; 9, 37 (s1), 11, 31 (s1), 12,9(s) : 3H (mobiles).

### Exemple 3 : trifluoroacétate de 5,7-dihydroxy-8-[(3S,4R)-3-hydroxy-1-méthyl-4-piperidinyl)-2-(5-méthyl-3-isoxazolyl)-4H-benzopyran-4-one

### Stade a) : 5,7-diméthoxy-8-[(3S,4R)-3-hydroxy-1-méthyl-4-piperidinyl]-2-(5-méthyl-3-isoxazolyl)-4H-benzopyran-4-one

### 1) Condensation : 1-[2-hydroxy-4,6-diméthoxy-3-[(3S,4R)-3-hydroxy-1-méthyl -4-piperidinyl]phényl]-3-(5-méthyl-3-isoxazolyl) 1,3-propanedione

Dans un ballon de 30 cm3, sous N2, on introduit 310 mg de NaH à 50 % dans l'huile (M=24) (6.5 mmol-4eq.), 10 cm3 de THF anhydre, 500 mg d'Acétoflocinopipéridol (1.6 mmol) par fractions, 2 gouttes d'EtOH et 12 mg de Dibenzo-18-Crown-6 (M=360.41) (0.03 mmol-2 % molaire). On agite ½h à température ambiante, puis introduit 684 mg de 5-méthyl-3-isoxazolecarboxylate de méthyle (M=141.13) (4.8 mmol-3eq.) On maintient sous agitation à température ambiante, pendant 18h.

### 2) Cyclisation : 5,7-diméthoxy-8-[(3S,4R)-3-hydroxy-1-méthyl-4-piperidinyl]-2-(5-méthyl-3-isoxazolyl)-4H-benzopyran-4-one

On ajoute au milieu réactionnel obtenu ci-dessus en 1), 10 cm3 de HCl à 36 % (116 mmol-72eq.) et porte alors à 50°C pendant 2h. On revient à température ambiante, verse dans 100 cm3 de NaOH(2N), extrait par 2x200 cm3 de CH2Cl2/MeOH : 8/2, lave par une solution saturée en NaCl, sèche sur Na2SO4 anhydre et porte à sec sous vide. On obtient 650 mg d'un solide beige que l'on chromatographie sur silice 0,04-0,06 mm dans CH2Cl2/MeOH : 90/10 puis CH2Cl2/MeOH/NH40H : 90/10/1. On isole le produit attendu : sous forme de solide beige de poids 418 mg.

### Analyses :

Spectre IR Absorption région OH/NH
-C=O : 1658 cm-1 ; Système conjugué + aromatique : 1628, 1595, 1568 cm-1.
Spectre ¹H RMN : DMSO D⁶ δ (ppm)
1,47(d1), 3,14(m) : 2H ; 1, 97 (t, l), 2,93(d1) : 2H ;
2,20(masqué), 2,88(d1) 2H ; 2,23(s) : CH3-N , 3,29(d1) : 1H (axial) ; 3,94(masqué) : 1H(OH) ; 3,69(s,1) : 1H
(équatorial) ; 3,90(s), 3,94(s) : 6H ; 6, 66 (s) : 1H ; 6,72(s), 6,93(s) : 2H ; 2,51(masqué) : 3H.

### Stade b) : trifluoroacétate de 5,7-dihydroxy-8-[(3S, 4R)-3-hydroxy-1-méthyl-4-piperidinyl]-2-(5-méthyl-3-isoxazolyl)-4H-benzopyran-4-one

Dans un ballon de 30 cm3 avec réfrigérant à reflux, sous N2, on introduit 90 mg du diméthoxy obtenu au stade a) ci-dessus (0.22 mmol), 2.5 cm3 de DMF anhydre et 538 mg de Reillex-pyridine -HI (≈ 4 mmol/g) (2.15 mmol-10 eq.) : on porte à 130°C pendant 18h45 et à 150°C pendant 5h30. On revient ensuite à température ambiante, dilue par CH3CN, filtre sur Iéna, rince par CH3CN puis ajoute 1.7 g de résine PTBD (2.6 mmol/g) (4.4 mmol-20eq.) On agite 1h à température ambiante, filtre sur Iéna, lave par CH3CN puis décroche par 20 cm3 de CH3CN/TFA : 95/05 sous agitation. On filtre sur Iéna, rince par CH3CN/TFA : 95/05 puis porte à sec sous vide. On récupère 205 mg d'un vernis jaune que l'on reprend par 3 cm3 d'éluant HPLC décrit ci-après. On filtre sur toupie-0.45 *µ*m puis injecte en 3 fois en HPLC préparative : Kromasil C18 - 10 *µ*m - 500x22 mm - λ= 225 nm - 15 ml/min.avec pour éluant CH3CN/H2O/TFA : 30/70/0.1. On regroupe les fractions propres que l'on concentre sous vide puis lyophilise. On isole le produit attendu sous forme de solide crème de poids 43 mg.

### Analyses :

Spectre ¹H RMN : DMSO D⁶ δ (ppm)
1,85(dl), 3,35(masqué) : 2H ; 2,55(s) : 3H ; 2,80(s) : N-CH3 ; 3,14(m), 3,47 (m) : 2H ; 3,36 (masqué) : 2H ; 3,47 (m) : 1H (axial) ; 4,07(sl) : 1H(équatorial) ; 6,39(s) : 1H ; 6,94, 7,00 : 2H ; 5,77(sl), 9,40(sl), 11,32(sl), 12,87(s) : 4H (mobiles).

### Exemple 4 : trifluoroacétate de 5,7-dihydroxy-8-[(3S, 4R)-3-hydroxy-1-méthyl-4-piperidinyl]-2-[5-(trifluorométhyl)1-phényl-1H-pyrazol-4-yl]-4H-benzopyran-4-one

### Stade a) : 5,7-diméthoxy-8-[(3S, 4R)-3-hydroxy-1-méthyl-4-piperidinyl]-2-[5-(trifluorométhyl)1-phényl-1H-pyrazol-4-yl]-4H-benzopyran-4-one

### 1) Condensation : 1-[2-hydroxy-4,6-diméthoxy-3-[(3S, 4R)-3-hydroxy-1-méthyl-4-piperidinyl]phényl]-3-[5-(trifluorométhyl) 1-phényl-1H-pyrazol-4-yl]-1,3-propanedione

Dans un ballon sec de 50 cm3, sous N2, on introduit 310 mg de NaH à 50 % dans l'huile (M=24) (6.5 mmol-4eq.), 10 cm3 de THF anhydre, 500 mg d'Acétoflocinopipéridol (1.6 mmol) par fractions, 2 gouttes d'EtOH et 12 mg de Dibenzo-18-Crown-6 (M=360.41) (0.03 mmol-2 % molaire). On agite ½h à température ambiante, puis introduit 1.3 g de 5-(trifluorométhyl)1-phényl-1H-pyrazole-4-carboxylate de méthyle (M=270.21) (4.8 mmol-3eq.) On porte à reflux pendant 2h30.

### 2) Cyclisation : 5,7-diméthoxy-8-[(3S,4R)-3-hydroxy-1-méthyl-4-piperidinyl]-2-[5-(trifluorométhyl)1-phényl-1H-pyrazol-4-yl]-4H-benzopyran-4-one

On ajoute au milieu réactionnel obtenu ci-dessus en 1), 10 cm3 de HCl à 36 % (116 mmol-72eq.) et porte alors à 80°C pendant 1h. On revient à température ambiante, verse dans 100 cm3 de NaOH (2N), extrait par 2x200 cm3 de CH2Cl2/MeOH 8/2, lave par une solution saturée en NaCl, sèche sur Na2SO4 anhydre et porte à sec sous vide. On obtient 3.72 g d'une huile orange que l'on chromatographie sur silice 0,04-0,06 mm dans CH2Cl2/MeOH/NH4OH : 90/10/0.2 puis 90/10/1. On isole le produit attendu sous forme de mousse beige de poids 454 mg.

### Analyses :

Spectre IR : Absorption région OH/NH
-C=O : 1656 cm-1 ; Système conjugué + aromatique : 1627, 1596, 1570, 1500 cm-1.
Spectre ¹H RMN : DMSO D⁶ δ (ppm)
1,47(dl), 3,03(m) : 2H ; 1, 97 (t, l), 2,87(masqué) : 2H ; 2,87(masqué), 2,18(masqué) : 2H ; 2,21(s) : CH3-N ; 3,29(masqué) : 1H (axial) ; 3,71(s, l) : 1H (équatorial) ; 3,90(s), 3,94(s) : 6H ; 4,32 (dl) : 1H(OH) ; 6,31(s) : 1H ; 6,67 (s) : 1H ; 7,63 (sl) : 5H ; 8,55(s) : 1H.

### Stade b) : trifluoroacétate de 5,7-dihydroxy-8- [(3S,4R)-3-hydroxy-1-méthyl-4-piperidinyl]-2-[5-(trifluorométhyl)1-phényl-1H-pyrazol-4-yl]-4H-benzopyran-4-one

Dans un ballon de 30 cm3 avec réfrigérant à reflux, sous N2, on introduit 117 mg du diméthoxy obtenu ci-dessus au stade a) (0.22 mmol), 2.5 cm3 de DMF anhydre, 538 mg de Reillex-pyridine -HI (≈ 4 mmol/g) (2.15 mmol-10 eq.).

On porte à 130°C pendant 37h et à 150°C pendant 5h.

On revient ensuite à température ambiante, dilue par CH3CN, filtre sur Iéna, rince par CH3CN puis ajoute 1.7 g de résine PTBD (2.6 mmol/g) (4.4 mmol-20eq.). On agite 4h30 à température ambiante, filtre sur Iéna, lave par CH3CN puis décroche par 20 cm3 de CH3CN/TFA : 95/05 sous agitation. On filtre sur Iéna, rince par CH3CN/TFA : 95/05 puis porte à sec sous vide. On récupère 195 mg d'une résine jaune que l'on reprend par 5 cm3 d'éluant HPLC décrit ci-après. On filtre sur toupie-0.45 *µ*m puis injecte en 2 fois en HPLC préparative : Kromasil C18 - 10 *µ*m - 500x22 mm λ= 225 nm- 15 ml/min. avec pour éluant : CH3CN/H2O/TFA : 40/60/0.1. On regroupe les fractions propres que l'on concentre sous vide puis lyophilise. On isole le produit attendu sous forme de solide légèrement jaune de poids 83 mg.

### Analyses :

Spectre ¹H RMN : DMSO D⁶ δ (ppm)
1, 79 (dl), 3,08(m) : 2H ; 2,79(d) : CH3-N ; 3,10(m), 3,45(m) : 2H ; 3,28(tl), 3,38(m) : 2H ; 3,51(m) : 1H(axial) ;
4,08(sl) : 1H (équatorial) ; 6,39(s) : 1H ; 6,55(s) : 1H ; 6,27(s1) : 1H (OH) ; 7,64(s1) : 5H ; 8,54(s) : 1H ; 9,43(s1): 1H NH⁺ ; 11, 34 (sl), 12,94(s) : 2H(OH).

### Exemple 5 : trifluoroacétate de 4-[5,7-dihydroxy-8-[(3S,4R)-3-hydroxy-1-méthyl-4-piperidinyl]-4-oxo-4H-benzopyran-2-yl]-cyclohexanecarboxylate de méthyle

### Stade a) : 4-[5,7-diméthoxy-8-[(3S,4R)-3-hydroxy-1-méthyl-4-piperidinyl] -4-oxo-4H-benzopyran-2-yl]cyclohexanecarboxylate de méthyle

### 1) Condensation : 4-[3-(2-hydroxy-4,6-diméthoxy-3-[(3S,4R)-3-hydroxy-1-méthyl-4-piperidinyl]phényl]1,3-dioxopropyl]-cyclohexanecarboxylate de méthyle

Dans un ballon de 30 cm3, sous N2, on introduit 390 mg de NaH à 50 % dans l'huile M=24 (8 mmol-4eq.), 10 cm3 de DMSO/NK20 et ajoute 618 mg d'Acétoflocinopipéridol (2 mmol). On agite 1h à température ambiante, puis introduit 1.1 cm3 (600 mg)de 1,4-cyclohexanedicarboxylate de diméthyle (M=200.24) (3 mmol-3eq.). On maintient sous agitation pendant 20h. On traite la réaction en versant dans l'eau glacée et amène à pH 7 par addition de HCl 2N. On extrait par un mélange CH2Cl2/MeOH : 90/10. On lave à l'eau, sèche sur MgSO4, filtre et amène à sec sous vide au rotavapor. Le produit brut obtenu de poids 3.58 g, est chromatographié sur silice 0.04-0.06 mm dans CH2Cl2/MeOH : 80/20. On isole les fractions contenant le produit attendu que l'on amène à sec et sèche sous vide : on obtient ainsi le produit attendu sous forme de résine de poids 300 mg.

### 2) Cyclisation : 4-[5,7-diméthoxy-8-[(3S,4R)-3-hydroxy-1-méthyl-4-piperidinyl]-4-oxo-4H-benzopyran-2-yl] cyclohexanecarboxylate de méthyle

Dans un ballon sous agitation, on met 300 mg de produit ci-dessus en 1) (0.62 mmol), 1.5 cm3 de DMSO anhydre et 0.063 cm3 d'HCl conc à 36 % (0.75 mmol-1.2 eq). On porte le milieu à 50°C pendant 1h. La réaction est terminée, on verse dans l'eau glacée, amène à pH 9-10 par addition de soude conc. (1.5 cm3). On extrait par 3 fois 20 cm3 de CH2Cl2/MeOH : 90/10, lave à l'eau, sèche sur MgSO4, filtre et amène à sec.

On chromatographie sur colonne de silice 0.04-0.06 mm dans l'éluant CH2Cl2/MeOH : 80/20 et isole le produit attendu sous forme de solide jaune de poids 145 mg : le produit TRANS est majoritaire.

### Analyses :

Spectre ¹H RMN : CDCl3 δ (ppm)
2,50(tt) 1H ; 2,35(tt) : 1H ; 1,45(m), 2,11(m), 2,14(m), 1,59(m) : 8H ; 2,48(s) : N-CH3 ; 2,41, 3,22 : 2H ; 2,25, 3,22 : 2H ; 1,66, 3,22 : 2H ; 3,92(1) : 1H(éq) ; 3,41(ddd) : 1H(axial) ; 2,80 (1) : OH ; 3,70(s), 3,94(s), 3,96(s) : 9H ; 6,00 (s) : 1H ; 6,40 (s) : 1H.

### Stade b) : trifluoroacétate de 4-[5,7-dihydroxy-8-[(3S,4R)-3-hydroxy-1-méthyl-4-piperidinyl]-4-oxo-4H-benzopyran-2-yl]-cyclohexanecarboxylate de méthyle

### A) Déblocage : trifluoroacétate de l'acide 4-[5,7-dihydroxy-8-[(3S,4R)-3-(formyloxy)-1-méthyl-4-piperidinyl]-4-oxo-4H-benzopyran-2-yl]-cyclohexanecarboxylique

Dans un ballon de 30 cm3 avec réfrigérant à reflux, sous N2, on introduit 120 mg du diméthoxy obtenu ci-dessus au stade a) (0.26 mmol), 2.6 cm3 de DMA anhydre et 540 mg de HI Pyridine M= 207.01 (2.6 mmol-10 eq.).On porte à 130°C pendant 20h. On revient ensuite à température ambiante, dilue par CH3CN, puis ajoute 1.9 g de résine PTBD (2.6 mmol/g) (5.2 mmol-20eq.). On agite 2h à température ambiante, filtre sur Iéna, lave par CH3CN puis décroche par 10 cm3 de CH3CN/TFA : 95/05 sous agitation. On filtre sur Iéna, rince par CH3CN/TFA : 95/05 puis porte à sec sous vide. On obtient ainsi 160 mg de produit attendu.

### B) Estérification : trifluoroacétate de 4-[5,7-dihydroxy-8-[(3S,4R)-3-hydroxy-1-méthyl-4-piperidinyl]-4-oxo-4H-benzopyran-2-yl]-cyclohexanecarboxylate de méthyle

Dans un ballon sous agitation, et argon, on met 160 mg de produit obtenu en A) ci-dessus (0.26 mmol supposées), 1.6 cm3 de méthanol et 0.25 cm3 de ClSiMe3 (M=108.64 d=0.856). On laisse à 50°C pendant 48h. On amène à sec sous vide au rotavapor. On obtient ainsi 150 mg de produit que l'on purifie par HPLC : les 150 mg de produit ainsi obtenus sont dissous dans 1 cm3 de solution de CF3CO2Na à 0.5 M/l auxquels on ajoute 2 cm3 d'éluant HPLC : CH3CN/H2O/TFA : 35/65/0.1. On filtre sur toupie-0.45 µm puis injecte en 3 fois en HPLC préparative : Kromasil C18 - 10 µm - 500x22 mm - λ= 225 nm - 15 ml/min. On regroupe les fractions propres que l'on concentre sous vide puis lyophilise. On isole ainsi le produit attendu sous forme de solide de poids 48 mg.

### Analyses :

Spectre ¹H RMN : DMSO D⁶ δ (ppm)
2,79 : N-CH3 ; 3,35 : 2H ; 5,74 : 1H(OH) ; 4,00(s1) : 1H(éq) ; 3,40(masqué) : 1H(axial) ; 3,13, 1,79 : 2H ; 3,47, 3,12 : 2H ; 3,63(s) : 3H ; 2,59, 1,96, 1,65, 1,48, 2,03, 2,44 : 10 H ; 6,14 : 1H ; 6,30 : 1H ; 9,34(sl), 11,1 : 3H.

### Exemple 6 : trifluoroacétate de 2-(4-cyclohexylphényl)-5,7-dihydroxy-8-[(3S, 4R)-3-hydroxy-1-méthyl-4-piperidinyl]-4H-benzopyran-4-one

### Stade a) : 2-(4-cyclohexylphényl)-5,7-diméthoxy-8-[(3S,4R)-3-hydroxy-1-méthyl-4-piperidinyl]-4H-benzopyran-4-one

### 1) Condensation : 1-(4-cyclohexylphényl)-3-[2-hydroxy-4,6-diméthoxy-3-[(3S,4R)-3-hydroxy-1-méthyl-4-piperidinyl] phényl]-1,3-propanedione

Dans un ballon de 30 cm3, sous N2, on introduit 288 mg de NaH à 50 % dans l'huile (M=24) (4.5 mmol-4 eq), 4.5 cm3 de DMSO/NK20 et 0.464 mg d'Acétoflocinopipéridol (1.5 mmol) On agite 1h à température ambiante, puis introduit 0.982 mg de 4-cyclohexyl-benzoate de méthyle (M=218.3) (4.5 mmol-3 eq.) et maintient sous agitation pendant 20h.

### 2) Cyclisation : 2-(4-cyclohexylphényl)-5,7-diméthoxy-8-[(3S,4R)-3-hydroxy-1-méthyl-4-piperidinyl]-4H-benzopyran-4-one

On ajoute au milieu réactionnel obtenu ci-dessus en 1), 3.25 cm3 de HCl à 36 % (39 mmol-25 eq) et porte alors à 50°C pendant 3 h. On revient à température ambiante, traite en diluant avec 5 cm3 d'eau et ajoute 3.5 cm3 de NaOH 12 N extrait par 3 fois 20 cm3 de CH2Cl2/MeOH : 9/1, lave à l'eau, sèche sur Na2SO4 anhydre et porte à sec sous vide. On obtient 1.4 g d'une huile marron que l'on chromatographie sur silice 0,04-0,06 mm dans CH2Cl2/MeOH : 90/10. On isole le produit attendu sous forme de solide jaune de poids 300 mg.

### Analyses :

Spectre ¹H RMN : CDCl3 δ (ppm)
1,2, 1,95 : 10 H ; 2,58(tt) : 1H ; 2,41(s) : N-CH3 ; 3,96(s), 3,99(s) : 6H ; 2, 33 (dl), 3,15 : 2H ; 4,09 (sl) : 1H(éq) ; 3,61(ddd) : 1H(axial) ; 3,12, 1,64 : 2H ; 2,15, 3,09 : 2H ; 6,44(s) : 1H ; 6,64(s) : 1H ; 7,37 , 7,74 : 4H.

### Stade b) : trifluoroacétate de 2-(4-cyclohexylphényl)-5,7-dihydroxy-8-[(3S,4R)-3-hydroxy-1-méthyl-4-piperidinyl]-4H-benzopyran-4-one

Dans un ballon de 20 cm3 avec réfrigérant à reflux, sous N2, on introduit 113 mg du diméthoxy obtenu au stade a) ci-dessus (0.24 mmol) et 1,3 cm3 d'HI (57 %). On porte à 130°C pendant 1h30. On revient ensuite à température ambiante, dilue par 3 cm3 de MeOH, amène à pH 10 par addition de 2 cm3 de solution de soude 6M/l. On filtre sur toupie-0.45 µm puis injecte en 4 fois en HPLC préparative : Kromasil C18 - 10 µm - 500x22 mm - λ= 225 nm - 15 ml/min. avec pour éluant : MeOH/H2O/TFA : 75/25/0.1. On regroupe les fractions propres que l'on concentre sous vide puis lyophilise. On isole ainsi le produit attendu sous forme de solide jaune de poids 41 mg.

### Analyses :

Spectre ¹H RMN : DMSO D⁶ δ (ppm)
1,28, 1,73 : 2H ; 1,82, 1,41 : 4H ; 1,45, 1,82 : 4H ; 2,62(m) : 1H ; 2,82(s) : CH3-N ; 1,83, 3,20 : 2H ; 3,20, 3,47 : 2H ; 3,40 : 2H ; 3,54(dl) : 1H(axial) ; 4,10(sl) : 1H(équatorial) ; 6,35(s) : 1H ; 6,89(s) : 1H ; 8,02 : 2H ; 7,45 : 2H ; 5,98(1) : 1H(OH) ; 9, 44 (1), 11, 17 (1), 13,20 (1) : 3H mobiles.

### Exemple 7 : trifluoroacétate de 4-[5,7-dihydroxy-8-[(3S,4R)-3-hydroxy-1-méthyl-4-piperidinyl]-4-oxo-4H-benzopyran-2-yl]-benzonitrile

### Stade a) : 4-[5,7-diméthoxy-8-[(3S,4R)-3-hydroxy-1-méthyl-4-piperidinyl]-4-oxo-4H-benzopyran-2-yl]-benzonitrile

### 1) Condensation : 4-[3-[2-hydroxy-4,6-diméthoxy-3-[(3S,4R) 3-hydroxy-1-méthyl-4-piperidinyl]phényl]-1,3-dioxopropyl]-benzonitrile.

Dans un ballon de 20 cm3, sous N2, on introduit 392 mg de NaH à 50 % dans l'huile (M=24) (8 mmol-4eq.), 10 cm3 de DMSO/NK20 et 618 mg d'Acétoflocinopipéridol (2 mmol). On agite 1h à température ambiante, puis introduit 966 mg de 4-cyanobenzoate de méthyle (M= 161.16) (6 mmol-3eq.). On maintient sous agitation pendant 20h à température ambiante.

### 2) Cyclisation : 4-[5,7-diméthoxy-8-[(3S,4R)-3-hydroxy-1-méthyl-4-piperidinyl]-4-oxo-4H-benzopyran-2-yl]-benzonitrile

On ajoute au milieu réactionnel obtenu ci-dessus en 1), 3 cm3 de HCl à 36 % (36 mmol-18eq.) et porte alors à 50°C pendant 1h30. On revient à température ambiante. Le produit attendu précipite dans le milieu. On filtre le précipité, lave à l'éther, et sèche sous vide. Le produit obtenu est sous forme de chlorhydrate sous forme de solide jaune de poids 1.4 g. Le produit obtenu est libéré de son sel par traitement à la soude 1N et extrait par un mélange de CH2Cl2/MeOH : 90/10, 3 fois 20 cm3. On obtient ainsi 600 mg de produit attendu.

### Analyses :

Spectre IR : - Absorption région OH/NH
-CN : 2228 cm-1 ; C=O : 1650 cm-1 ; Système conjugué + aromatique : 1619, 1594, 1568, 1557, 1497 cm-1
Spectre ¹H RMN : CDCl3 δ (ppm)
7,93, 7, 82AA' BB' . 4H ; 6,69 (s) : 1H ; 6,47 (s) : 1H ;
4,01(s), 3,98(s) : 6H ; 4,0 : 1H ; 2,39(s) : CH3-N ; 3,10(m), 1,61 : 2H ; 3,09(m), 2,11(t) : 2H ; 3,15(m), 3,28 : 2H ; 1,80 : 1H(OH) ; 3,51(m) : 1H.

### Stade b) : trifluoroacétate de 4-[5,7-dihydroxy-8-[(3S,4R)-3-hydroxy-1-méthyl-4-piperidinyl]-4-oxo-4H-benzopyran-2-yl]-benzonitrile

Dans un ballon de 30 cm3 avec réfrigérant à reflux, sous N2, on introduit 112 mg du diméthoxy obtenu ci-dessus au stade a) (0.26 mmol), 2.6 cm3 de DMF anhydre et 732 mg de Reillex-pyridine -HI (≈ 4 mmol/g) (2.86 mmol-10 eq.)obtenu à la préparation 2) ci-dessus. On porte à 130°C pendant 40h. On revient ensuite à température ambiante, dilue par MeOH, filtre sur Iéna, rince par MeOH puis amène à sec sous vide au rotavapor. Poids de produit brut obtenu : 500 mg. Ce produit est mis en solution dans 6 cm3 de solution de soude 6M/l de MeOH (pH du milieu- 10). On filtre sur toupie-0.45 µm puis injecte en 6 fois en HPLC préparative : Kromasil C18 - 10 µm - 500x22 mm - λ= 225 nm - 15 ml/min avec pour éluant :
MeOH/H2O/TFA : 55/45/0.1. On regroupe les fractions propres que l'on concentre sous vide puis lyophilise. On isole ainsi le produit attendu sous forme de solide jaune de poids 75 mg.

### Analyses :

Spectre ¹H RMN : DMSO D⁶ δ (ppm)
3,16, 1,81(dl) : 2H ; 2,83 : CH3-N ; 3,16, 3,47(masqué) : 2H ; 3,40(masqué) : 2H ; 3,53(tl) : 1H(éq) ; 4,09(sl) :
1H (axial) ; 6,39(s) : 1H ; 7,09(s) : 1H ; 8,30, 8,05AA'BB' : 4H ; 5,97, 11,31, 13,03, 9,42 : 4H mobiles.

### Exemple 8 : trifluoroacétate de 5,7-dihydroxy-8-[(3S,4R)-3-hydroxy-1-méthyl-4-piperidinyl]-2-[4-(1H-tetrazol-5-yl)-phényl]-4H-benzopyran-4-one

### Stade a) : 5,7-diméthoxy-8-[(3S,4R)-3-hydroxy-1-méthyl-4-piperidinyl]-2-[4-(1H-tetrazol-5-yl)-phényl]-4H-benzopyran-4-one

Dans un ballon de 30 cm3, sous N2,muni d'un réfrigérant, on introduit 200 mg de produit nitrile obtenu au stade a) de l'exemple 7 (M= 420.47) (0.47 mmol), 4 cm3 de toluène sec et 180 mg d'azidure de triméthylétain (M=205,81) (0,87 mmol - 1.85 eq). On porte à reflux pendant 43h puis on refroidit et filtré le précipité formé. On lave et on sèche sous vide pendant une nuit. On obtient ainsi 300 mg de produit brut. Le produit brut est chromatographié sur silice 0,04-0,06 dans CH2Cl2/MeOH/NH4OH : 78/20/02. On isole ainsi le produit attendu sous forme de solide de poids 174 mg.

### Analyses :

Spectre ¹H RMN : DMSO D⁶ δ (ppm)
3,38(masqué) : 2H ; 4,06(sl) : 1H(équatorial) ; 3,60(dl) : 1H(axial) ; 3,33(masqué), 1,83(dl) : 2H ; 3,27, 3,38(masqués) : 2H ; 2,81(s) : N-CH3 ; 6,70(s) : 1H ; 6,67(s) : 1H ; 3,91(s) : 3H ; 3,94(s) : 3H ; 5, 53 (sl) 1H(OH) ; 8,06, 8,20 AA'BB' : 4H.

### Stade b) : trifluoroacétate de 5,7-dihydroxy-8-[(3S,4R)-3-hydroxy-1-méthyl-4-piperidinyl}-2-[4-(1H-tetrazol-5-yl)-phényl]-4H-benzopyran-4-one

Dans un ballon de 30 cm3 avec réfrigérant à reflux, sous N2, on introduit 120 mg du diméthoxy obtenu au stade a) ci-dessus (0.26 mmol), 2.4 cm3 de DMF anhydre et 700 mg de Reillex-pyridine-HI (≈ 4 mmol/g) (2.86 mmol-11eq.) obtenu à la préparation 2) ci-dessus. On porte à 130°C pendant 40h. On revient ensuite à température ambiante, filtre sur Iéna, rince par MeOH puis par DMF. Le produit solide obtenu est lavé à l'eau et séché sous vide. On obtient 209 mg de produit brut. Ce produit brut est mis en solution dans environ 4 cm3 de l'éluant HPLC : CH3CN/H2O/TFA : 25/75/0.1. Le milieu est amené à pH 10 par addition de soude 2N (0.45 cm3). On filtre sur toupie-0.45 *µ*m puis injecte en 5 fois en HPLC préparative : Kromasil C18 - 10 *µ*m - 500x22 mm - λ= 225 nm - 15 ml/min -.

On regroupe les fractions propres que l'on concentre sous vide puis lyophilise. On isole ainsi le produit attendu sous forme de solide jaune de poids 88 mg.

### Analyses :

Spectre ¹H RMN : DMSO D⁶ δ (ppm)
3,43(s1) : 2H ; 4,11(s1) : 1H(éq) ; 3,57(d1) : 1H(ax) ; 3,20(masqué), 1,84 (d1) : 2H ; 3,21(masqué), 3,49(d1) : 2H ; 2,84(s) : N-CH3 ; 7,08(s) : 1H ; 6,38(s) : 1H ; 8,29, 8,35 AA' BB' : 4H ; 5,99(s1) : 1H(OH) ; 9,36(sl), 11,25(s1) ; 13,11(s) : 3H(mobiles).

### Exemple 9 : trifluoroacétate de 5,7-dihydroxy-8-[(3S,4R)-3-hydroxy-1-méthyl-4-piperidinyl]-2-[3-(phénoxy)-phényl]-4H-benzopyran-4-one

### Stade a) : 5,7-diméthoxy-8-[(3S,4R)-3-hydroxy-1-méthyl-4-piperidinyl]-2-[3-(phénoxy)phényl]-4H-benzopyran-4-one

### 1) Condensation : 1-[2-hydroxy-4,6-diméthoxy-3-[(3S,4R)-3-hydroxy-1-méthyl-4-piperidinyl]phényl]-3-[3-(phénoxy)phényl] -1,3-propanedione

Dans un ballon de 10 cm3, sous N2, on introduit 196 mg de NaH à 50 % dans l'huile (M=24) (4 mmol-4eq.), 5 cm3 de DMSO/NK20 et 309 mg d'Acétoflocinopipéridol (1 mmol). On agite 1h à température ambiante, puis introduit 685 mg de 3-(phénoxy)-benzoate de méthyle (M=228.2) (3 mmol-3eq.) On maintient sous agitation pendant 20 heures. On traite le milieu par 3.5 cm3 de HCl 1N jusqu'à pH 6-7, on extrait avec 3 fois 10 cm3 de chlorure de méthylène, puis on lave à l'eau, sèche sur MgSO4, filtre et amène à sec sous vide. On obtient ainsi 1.5 g de produit brut.

### 2) Cyclisation : 5,7-diméthoxy-8-[(3S,4R)-3-hydroxy-1-méthyl-4-piperidinyl]-2-[3-(phénoxy)phényl]-4H-benzopyran-4-one

Dans un ballon de 10 cm3, on met 474 mg de produit brut (0.32 mmoles) obtenu ci-dessus en 1) et 0.5 cm3 de HCl à 36 % (6 mmol-18eq.). On porte alors à 50°C pendant 2h. On revient à température ambiante, on dilue par 5 cm3 d'eau, ajoute 0.55 cm3 de NaOH à 32 %, extrait par 3 fois 10 cm3 de CH2Cl2/MeOH : 9/1, lave à l'eau, sèche sur Na2SO4 anhydre et porte à sec sous vide. On obtient 150 mg d'une huile marron que l'on chromatographie sur silice 0,04-0,06 mm dans CH2Cl2/MeOH : 95/05. On obtient ainsi le produit attendu sous forme de solide jaune de poids 220 mg.

### Analyses :

Spectre IR : Absorption région OH/NH
-C=O : 1646 cm-1 ; Système conjugué + aromatique : 1620, 1597, 1578, 1489 cm-1.
Spectre ¹H RMN : CDCl3 δ (ppm)
2,33(s) : N-CH3 ; 3,95(s), 3,98(s) : 6H ; 6,44(s) : 1H ; 6,62(s) : 1H ; 1,57(m), 3,10(m) : 2H ; 2,10(m), 2,98(m) : 2H ; 2,03(m), 3,02(m) : 2H ; 3,48(ddd) : 1H(ax) ; 3,93(1) : 1H(éq) ; 7,06 : 2H ; 7,16 : 1H ; 7,38 : 2H ; 7,19(ddd) : 1H ; 7,45(dd) : 1H ; 7,50(t) : 1H ; 7,56(ddd) : 1H.

### Stade b) : trifluoroacétate de 5,7-dihydroxy-8-[(3S,4R)-3-hydroxy-1-méthyl-4-piperidinyl]-2-[3-(phénoxy)-phényl]-4H-benzopyran-4-one

Dans un ballon de 30 cm3 avec réfrigérant à reflux, sous N2, on introduit 200 mg du diméthoxy obtenu au stade a) ci-dessus (0.41 mmol) et 3 cm3 de HI à 47 %(1604 mmol-40 eq.) et porte à 130°C pendant 1h30. On arrête la réaction et on amène à sec sous vide au rotavapor. On récupère 340 mg d'un produit amorphe orange que l'on reprend par 6 cm3 d'éluant HPLC décrit ci-après. On filtre sur toupie-0.45 *µ*m puis injecte en 4 fois en HPLC préparative : Kromasil C18 - 10 *µ*m - 500x22 mm - λ = 225 nm - 15 ml/min. avec pour éluant MeOH/H2O/TFA : 65/35/0.1. On regroupe les fractions propres que l'on concentre sous vide puis lyophilise. On isole le produit attendu sous forme de solide jaune de poids 25 mg.

### Analyses :

Spectre ¹H RMN : DMSO D⁶ δ (ppm)
2,78(s) : N-CH3 ; 3,23, 3,33 : 2H ; 4,07 : 1H ; 3,49 : 1H ; 3,14, 1,80 : 2H ; 3,14, 3,37 : 2H ; 5,99 : 1H(OH) ; 6,93 : 1H ; 6,36 : 1H ; 13,11, 11,23 : 2H(mobiles) ; 7,68 : 1H ; 7,26 : 1H ; 7,69 : 1H ; 7,91 : 1H ; 7,11 : 2H ; 7,45 : 2H ; 7,21 : 1H.

### Exemple 10 : trifluoroacétate de 5,7-dihydroxy-6-[(3S,4R)-3-hydroxy-1-méthyl-4-piperidinyl]-2-[3-(phénoxy)-phényl] -4H-benzopyran-4-one

Au stade b) de l'exemple 9, un second produit est isolé sous forme de solide jaune de poids 16 mg.

### Analyses :

Spectre ¹H RMN : DMSO D⁶ δ (ppm)
2,74 : N-Me ; 3,29 : 2H ; 4,07 : 1H ; 3,40 : 1H ; 1,62, 3,05 : 2H ; 3,06, 3,36 : 2H ; 6,96 : 1H ; 6,45 : 1H ; 7,71 : 1H ; 7,18 : 1H ; 7,57 : 1H ; 7,84 : 1H ; 7,09 : 2H ; 7,44 : 2H ; 7,19 : 1H ; 13,58 : 1H(mobile) ; 9,08(sl) : 2H mobiles.

### Exemple 11 . 5,7-dihydroxy-8-[(3S,4R)-3-hydroxy-1-méthyl-4-piperidinyl]-2-(3-nitrophényl)-4H-benzopyran-4-one

### Stade a) : 1-[4,6-diméthoxy-3-[(3S,4R)-3[(éthoxycarbonyl)oxy] -1-méthyl-4-piperidinyl]-2-hydroxy phényl]-éthanone

Dans un ballon de 30 cm3, sous N2, on introduit 19 ml de CH2Cl2 et 1.13 g d'Acétoflocinopipéridol (3.65 mmol) puis à - 78°C, on introduit goutte à goutte, 348µl de chloroformate d'éthyle (C3H5ClO2, M=108.52, d=1.139, 3.65 mmol, 1.0eq.). On laisse le milieu réactionnel revenir à température ambiante lentement et laisse 48h à cette température. Le milieu réactionnel est dilué dans 100 ml de CH2C12, lavé ensuite deux fois avec H2O à 10 % de NaHCO3 et une fois avec H2O saturée en NaCl. Après séchage sur MgSO4, filtration et évaporation, on obtient 1.23 g de produit attendu.

### Analyses :

Spectre ¹H RMN : CDCl3 δ(ppm)
1, 19 (t, J=7) : 3H ; 4, 02 (qd) : 2H ; 1, 54 (m), 3, 10 (m) : 2H ; 2,08(m), 3, 01(m) : 2H ; 2, 31 (s1) : N-CH3 ; 3,09(m), 2,32 (masqué) : 2H ; 4,90(1) : 1H(éq) ; 3,35(ddd, J=2,5- 4,0-13,0) : 1H(ax) ; 2,61 (s) : 3H ; 3,84(s), 3, 89 (s) : 6H ; 5,90(s) : 1H ; 14,34(s) : 1H (OH).

Spectre IR : OH sous forme chélatée
-C=O : 1733 cm-1 ; C=O + aromatique : 1612, 1598 (max), 1582 (ep), 1503 cm-1 (f) .

### Stade b) : 5,7-diméthoxy-8-[(3S,4R)-3-[(éthoxycarbonyl)oxy]-1-méthyl-4-piperidinyl]-2-(3-nitrophényl)-4H-benzopyran-4-one

Dans un ballon de 60 cm3, sous N2, on introduit 1.09 g du produit obtenu au stade a) ci-dessus (2.87 mmol) et 15 cm3 de THF anhydre puis à 0°C, on introduit rapidement 462 mg de tBuOK (M=112) (4.12 mmol-1.4eq.)

Après 1h à cette température, on introduit 692 mg de chlorure de 3-nitro-benzoyle (M=185.57) (3.73 mmol-1.3eq.). On laisse le milieu revenir à TA et agit pendant 12h. On introduit ensuite à TA 398 mg de tBuOK (M=112) (3.45 mmol-1.2eq.). On porte le milieu à 65°C et laisse pendant 4h. On introduit alors 15 ml d'AcOH et 1.5 ml d'HCl concentré et porte à 65°C pendant 4h. On revient à température ambiante, et évapore les solvants sous vide. Les résidus sont repris par 200 cm3 de CH2Cl2/MeOH : 9/1, lavés par une solution saturée en NaHCO3, et H2O saturée en NaCl. On sèche sur MgSO4 anhydre et porte à sec sous vide. On obtient 3.7 g d'un solide marron que l'on chromatographie sur silice 0,04-0,06 mm dans CH2C12/MeOH : 90 : 10. On isole le produit attendu sous forme de solide jaune de poids 205 mg.

### Analyses :

Spectre ¹H RMN : CDCl3 δ(ppm)
3,25, 2,49 : 2H ; 2,39(s) : CH3-N ; 3,12, 3,18 : 2H ; 2,73, 3,22 : 2H ; 3,63(td) : 1H(ax) ; 3,96(s), 4,01(s) : 6H ; 1,06 : 3H ; 3,94 : 2H ; 5,0(sl) : 1H(éq) ; 6,44(s) : 1H ; 6,72(s) : 1H ; 8,78(s) : 1H ; 8,38 (dd) : 1H ; 8,40, 8,30 : 1H ; 7, 78 (t) : 1H.

### Stade c) : 5,7-diméthoxy-8-[(3S,4R)-3-hydroxy-1-méthyl-4-piperidinyl]-2-(3-nitrophényl)-4H-benzopyran-4-one

Dans un ballon de 10 cm3, sous N2, on introduit 200 mg de produit obtenu au stade b) ci-dessus (0.34 mmol), 2.4 ml d'éthanol et 0.4 ml de NaOH (2N) (0.8 mmol, 2 ; 35 eq).

On agite le milieu à TA pendant 2h et dilue dans 4.8 ml d'H2O et on laisse évapore l'éthanol pendant la nuit. On filtre les précipités sur fritté et lave avec H2O (3x2 ml), et de l'éther (5 ml). Après séchage sous vide, on obtient le produit attendu sous forme de solide jaune de poids 132 mg.

### Stade d) : 5,7-dihydroxy-8-[(3S,4R)-3-hydroxy-1-méthyl-4-piperidinyl]-2-(3-nitrophényl)-4H-benzopyran-4-one

Dans un ballon de 30 cm3 avec réfrigérant à reflux, sous N2, on introduit 100 mg du diméthoxy obtenu au stade c) ci-dessus (0.23 mmol), 2.3 cm3 de DMF anhydre et 624 mg de Reillex-pyridine -HI (≈ 4 mmol/g) (2.5 mmol-11 eq) obtenu à la préparation 2). On porte à 130°C pendant 24h. On revient ensuite à température ambiante, dilue par MeOH (5 ml), filtre sur Iéna, rince par MeOH (2x5 ml). On évapore sous vide le méthanol, les résidus sont repris dans 2.0 ml d'une solution de MeOH/H2O/CF3COONa (50/50, 0.5M), le pH du milieu de ce mélange est ajusté à 8 - 9 avec NaOH (N). On filtre sur Iéna, lave avec H2O (3x3 ml). On isole après séchage sous vide, le produit attendu sous forme de solide jaune = 80 mg.

### Analyses :

Spectre ¹H RMN : DMSO D⁶ δ (ppm)
1,52, 2,81 : 2H ; 2,84, 3,30 : 2H ; 2,96, 3,24 : 2H ; 2,62 (s) : CH3-N ; 3,43 : 1H ; 4,08(sl) : 1H ; 5,78 (s) : 1H ; 6,97(s) : 1H ; 7,86(t) : 1H ; 8,40, 8,46 : 2H ; 8,72 : 1H ; 12,76 : 1H (OH).

### Exemple 12 : trifluoroacétate de 5,7-dihydroxy-2-[4-fluoro-3-(trifluorométhyl)phényl]-8-[(3S,4R)-3-hydroxy-1-méthyl-4-piperidinyl]- 4H-benzopyran-4-one

### Stade a) : 5,7-diméthoxy-2-[4-fluoro-3(trifluorométhyl) phényl]-8-[(3S,4R)-3-hydroxy-1-méthyl-4-piperidinyl]-4H-benzopyran-4-one

Dans un ballon de 60 cm3, sous N2, on introduit 52 mg de NaH à 50 % dans l'huile (M=24) (1.08 mmol-1.2eq.) et 9.0 cm3 de THF anhydre puis à -78°C, on introduit 344 mg du produit obtenu au stade a) de l'exemple 11 (0.90 mmol). Après 1h à cette température, on introduit 225 *µ*l de chlorure de 4-fluoro-3-(triflurométhyl)-benzoyle(M=226.56) (d=1.493, 1.50 mmol, 1.7 éq). On laisse le milieu revenir à TA et agit pendant 12h. On introduit ensuite à 0°C 129 mg de NaH à 50 % dans l'huile (M=24) (2.69 mmol-3.0eq.) On laisse le milieu sous agitation à TA pendant 72h. On introduit alors 9 ml d'AcOH et 1 ml d'HCl concentré. On porte alors à 65°C pendant 3h. On revient à température ambiante, et évapore les solvants sous vide. Les résidus sont repris par 100 cm3 de CH2Cl2/MeOH : 9/1, lavés par une solution saturée en NaHC03, et H2O saturée en NaCl. On sèche sur MgSO4 anhydre et porte à sec sous vide. On obtient 703 mg d'une huile que l'on chromatographie sur silice 0,04-0,06 mm dans CH2Cl2/MeOH : 90/10. On isole ainsi le produit attendu sous forme de solide jaune = 148 mg.

### Analyses :

Spectre ¹H RMN : CDCl3 δ(ppm)
2,60(m), 3,46(m) : 2H ; 4,17 (sl) : 1H ; 3,52 (m) : 1H ; 1,67(dl), 3,31(m) : 2H ; 3,29(m), 2,41(m) : 2H ; 3,48 (sl) : 6H ; 6, 42 (sl), 6,52 (s) : 2H ; 7,37 (t) : 1H ; 8,16 (sl) : 1H ; 7,94 (sl) : 1H.

### Stade b) : trifluoroacétate de 5,7-dihydroxy-2-[4-fluoro-3-(trifluorométhyl)phényl]-8-[(3S,4R)-3-hydroxy-1-méthyl-4-piperidinyl]-4H-benzopyran-4-one

Dans un ballon de 30 cm3 avec réfrigérant à reflux, sous N2, on introduit 139 mg du diméthoxy obtenu au stade a) ci-dessus (0.29 mmol), 2.9 cm3 de DMF anhydre et 725 mg de Reillex-pyridine -HI (≈ 4 mmol/g) (2.9 mmol-10 eq.) obtenue comme indiqué à la préparation 2) ci-dessus. On porte à 130°C pendant 42h. On revient ensuite à température ambiante, dilue par CH3CN (10 ml), filtre sur Iéna, rince par CH3CN (2x10 ml) puis ajoute 2.23 g de résine PTBD (2.6 mmol/g) (5.8 mmol-20eq.). On agite 1h à température ambiante, filtre sur Iéna, lave par CH3CN (100 ml) puis décroche par 20 cm3 de CH3CN/TFA : 95/05 sous agitation. On filtre sur Iéna, rince par CH3CN/TFA : 95/05 (2x10 ml) puis porte à sec sous vide. On récupère 144 mg d'un produit amorphe orange que l'on reprend par 3 cm3 d'éluant HPLC : CH3CN/H2O/TFA : 35/65/0.1. On filtre sur toupie-0.45 µm puis injecte en 2 fois en HPLC préparative : Kromasil C18 - 10 *µ*m - 500x22 mm - λ= 225 nm - 15 ml/min. On regroupe les fractions propres que l'on concentre sous vide puis lyophilise. On isole ainsi le produit attendu sous forme de solide jaune de poids 76.2 mg.

### Analyses :

Spectre ¹H RMN : DMSO D⁶ δ (ppm)
1,83, 3,09 : 2H ; 2,80(s) : N-Me ; 3,13, 3,41 : 2H ; 3,34 : 2H ; 3,53(dl), 4,11 (s1) : 1H(ax), 1H(éq) ; 6,32 : 1H ; 7,13 : 1H ; 7,47(t) : 1H ; 8,39(dd) : 1H ; 8,43(d) : 1H.

### Exemple 13 : trifluoroacétate de 2-(2- chloro-3-pyridinyl)-5,7-dihydroxy-8-[(3S,4R)-3-hydroxy-1-méthyl-4-piperidinyl]-4H-benzopyran-4-one

### Stade a) : 2-chloro-3-pyridinecarboxylate de (3S,4R)-4-[2-(2-chloro-3-pyridinyl)-5,7-diméthoxy-4-oxo-4H-benzopyran-8-yl]-1-méthyl-3-piperidinyle

Dans un ballon de 60 cm3, sous N2, on introduit 586 mg d'Acétoflocinopipéridol (DI) (1.89 mmol) et 19.0 cm3 de THF anhydre. Ensuite à 0°C, on introduit 400 mg de NaH à 50 % dans l'huile (M=24) (8.3 mmol-4.4eq.) Après 1h à cette température, on introduit 1.70 g de chlorure de 2-chloro-3-pyridinecarbonyle (M=176.56) (5.67 mmol, 3éq). Le milieu réactionnel est laissé 1h à 0°C, 4h à TA, et 12h à 65°C. On laisse le milieu revenir à TA et introduit ensuite 110 mg de NaH à 50 % dans l'huile (M=24) (2.27 mmol-1.2eq.) On agite le milieu à TA pendant 1h à TA et 4h à 65°C. On introduit alors 19 ml d'AcOH et 1.9 ml d'HCl concentré et porte à 80°C pendant 12h. On revient à température ambiante, et évapore les solvants sous vide. Les résidus sont repris par 150 cm3 de CH2Cl2/MeOH : 9/1, lavés deux fois par une solution de NaOH (1N) et H2O saturée en NaCl. On sèche sur MgSO4 anhydre et porte à sec sous vide. On obtient 2.5 g de produits bruts que l'on chromatographie sur silice 0,04-0,06 mm dans CH2Cl2/MeOH/NH3.H2O : 90 : 10 : 01. On isole le produit attendu sous forme de solide jaune de poids 315 mg.

### Analyses :

Spectre ¹H RMN : CDCl3 δ(ppm)
3,23(dl), 2,37(dl) : 2H ; 3,07(tl), 1,79(dl) : 2H ; 3,05, 2,09(tl) : 2H ; 2,30(s) : CH3-N ; 3,62 (td) : 1H(ax) ; 5,39(sl) : 1H(éq) ; 3,99(s), 3,85(s) : 6H ; 6,40(s) : 1H ; 6,50(s) : 1H ; 7,23(dd) : 1H ; 7,97(dd) : 1H ; 8,45 (dd) : 1H ; 7,39(dd) : 1H ; 7,85(dd) : 1H ; 8,53(dd) : 1H.

### Stade b) : 2-(2- chloro-3-pyridinyl)-5,7-diméthoxy-8-[(3S,4R)-3-hydroxy-1-méthyl-4-piperidinyl]-4H-benzopyran-4-one

Dans un ballon de 10 cm3, sous N2, on introduit 300 mg de produit obtenu au stade a) ci-dessus (0.53 mmol), 1.06 ml d'éthanol et 0.53 ml de NaOH (2N) (1.06 mmol, 2 eq.). On agite le milieu à TA pendant 4h, et le dilue dans 20 ml d'H2O et extrait ensuite deux fois avec CH2Cl2/MeOH : 9/1 (2x25 ml).Les phases organiques sont réunies et lavées avec de l'eau saturée en NaCl, séchées sur MgSO4 filtrées et évaporées à sec. On obtient le produit attendu sous forme de solide jaune de poids 166 mg.

### Analyses :

Spectre ¹H RMN : CDC13 δ (ppm)
2,99, 1,57(dd) : 2H ; 2,06(tl), 3,0 : 2H ; 2,23(dl), 3,0 : 2H ; 2,32(s) : CH3-N ; 3,98(s), 4,01(s) : 6H ; 3,50(ddd) : 1H(ax) ; 3,92(sl) : 1H(éq) ; 3,26 : 1H(OH) ; 6,48 : 1H ; 6,48 : 1H ; 8,57(dd) : 1H ; 7,44(dd) : 1H ; 7,92(dd) : 1H. Spectre SM : MH+=431+ ; 413+
Spectre IR :
OH 3532 cm-1 ; C=O 1653 cm-1 ; Système conjugué + aromatique : 1631, 1598, 1574, 1561, 1494 cm-1.

### Stade c) : trifluoroacétate de 2-(2-chloro-3-pyridinyl)-5,7-dihydroxy-8-[(3S,4R)-3-hydroxy-1-méthyl-4-piperidinyl]-4H-benzopyran-4-one

Dans un ballon de 30 cm3 avec réfrigérant à reflux, sous N2, on introduit 144 mg du diméthoxy obtenu au stade b) (0.33 mmol), 3.3 cm3 de DMF anhydre et 725 mg de Reillex-pyridine-HI (≈ 4 mmol/g) (2.9 mmol-10 eq.) obtenu comme indiqué à la préparation 2) ci-dessus. On porte à 130°C pendant 42h. On revient ensuite à température ambiante, dilue par CH3CN (10 ml), filtre sur Iéna, rince par CH3CN (2x10 ml) puis ajoute 2.6 g de résine PTBD (2.6 mmol/g) (6.7 mmol-20 eq.). On agite 1h à température ambiante, filtre sur Iéna, lave par CH3CN (100 ml) puis décroche par 20 cm3 de CH3CN/TFA : 95/05 sous agitation. On filtre sur Iéna, rince par CH3CN/TFA : 95/05 (2x10 ml) puis porte à sec sous vide. On récupère 156 mg d'un produit amorphe orange que l'on reprend par 6 cm3 d'éluant HPLC CH3CN/H2O/TFA : 25/75/0.1. On filtre sur toupie-0.45 µm puis injecte en 3 fois en HPLC préparative : Kromasil C18 - 10 µm - 500x22 mm - λ= 225 nm - 15 ml/min. On regroupe les fractions propres que l'on concentre sous vide puis lyophilise. On isole ainsi le produit attendu sous forme de solide jaune de poids 30 mg.

### Analyses :

Spectre ¹H RMN : DMSO D⁶ δ(ppm)
1,82, 2,95 : 2H ; 2,73(sl) : N-CH3 ; 3,07, 3,36 : 2H ; 3,29 : 2H ; 3,45(masqué) : 1H(ax) ; 4,07(sl) : 1H(éq) ; 6,01 : 1H(OH) ; 6,39(s) : 1H ; 6,71(s) : 1H ; 7,68(dd) : 1H ; 8,28(dd) : 1H ; 8,65(dd) : 1H ; 9,30, 11, 34 (s), 12,32(s) : 3H(mobiles).
Spectre SM : MH+=403+

### Exemple 14 : trifluoroacétate de l'acide 4-[8-[(3S,4R)-3-acétyloxy-1-méthyl-4-piperidinyl]- 5,7-dihydroxy-4-oxo-4H-benzopyran-2-yl]-2,5-dichloro-benzoique

### Stade a) : 2,5-dichloro-4-[5,7-diméthoxy-8-[(3S,4R)-3-hydroxy-1-méthyl-4-piperidinyl]-4-oxo-4H-benzopyran-2-yl]-benzoate de méthyle

### 1) Condensation : 2,5-dichloro-4-[3-[2-hydroxy-4,6-diméthoxy-3-[(3S,4R)-3-hydroxy-1-méthyl-4-piperidinyl)phényl]1,3-dioxopropyl]-benzoate de méthyle

Dans un ballon de 30 cm3, sous N2, on introduit 550 mg de tBuOK(M=112.22) (4.5 mmol-3 eq.) et 7 cm3 de DMF/NK20. On refroidit à 0°C et on ajoute 460 mg d'Acétoflocinopipéridol (1.5 mmol). On agite 1h à température ambiante, puis introduit 1079 mg de 2,5-dichloro-1,4-benzenedicarboxylate de diméthyle (M=263.08) (4,5 mmol-3eq.). On maintient sous agitation pendant 17h à t amb. On traite la réaction en versant dans l'eau, amène à pH=7 par addition d'HCl 1N. On extrait par 3 fois 20 cm3 d'un mélange CH2Cl2/MeOH : 90/10, lave à l'eau et sèche sous vide. On obtient ainsi 1,34 g de produit brut.

### 2) Cyclisation : 2,5-dichloro-4-[5,7-diméthoxy-8-[(3S,4R)-3-hydroxy-1-méthyl-4-piperidinyl]-4-oxo-4H-benzopyran-2-yl] - benzoate de méthyle

Dans un ballon sous agitation, on met 1.3 g de produit brut obtenu ci-dessus en 1), 6.5 cm3 de DMF et 0.625 cm3 de HCl à 36 % (7.5 mmol-5 eq.). On porte alors à 50°C pendant 2h. On revient à température ambiante, verse dans l'eau et on ajoute 0.65 cm3 de NaOH(12N), extrait par 3 fois 20 cm3 de CH2Cl2/MeOH : 9/1, lave par de l'eau, sèche sur MgSO₄ anhydre et porte à sec sous vide. On obtient 1.3 g d'une huile marron que l'on chromatographie sur silice 0,04-0,06 mm dans CHCl3/MeOH : 90/10. On isole le produit attendu sous forme de solide jaune de poids 210 mg.

### Analyses :

Spectre ¹H RMN : CDCl3 δ (ppm)
1,56, 2,99 : 2H ; 3,99(s) : 3H ; 2,35(s) : N-CH3 ; 3,97(s), 3,99 (s) : 6H ; 2,03, 2,97 : 2H ; 2,20, 3,00 : 2H ; 3,91 : 1H(éq) ; 3,45(ddd) : 1H(ax) ; 3,19 : 1H(OH) ; 6,45(s) : 1H ; 6,54(s) : 1H ; 7,30(s) : 1H ; 7,87(s) : 1H.

### Stade b) : trifluoroacétate de l'acide 4-[8-[(3S,4R)-3-acétyloxy-1-méthyl-4-piperidinyl]-5,7-dihydroxy-4-oxo-4H-benzopyran-2-yl]-2,5-dichloro-benzoique

Dans un ballon de 30 cm3 avec réfrigérant à reflux, sous N2, on introduit 130 mg du diméthoxy obtenu au stade a) (0,25 mmol), 2.5 cm3 de Diméthylacétamide anhydre et 517 mg de HI-Pyridine (M= 207.1) (2.5 mmol-10 eq.) obtenu comme indiqué ci-dessus à la préparation 1). On porte à 130°C pendant 20h. On revient ensuite à température ambiante, ajoute 2.5 cm3 de CH3CN, puis on ajoute au milieu 1.9 g de résine PTBD (2.6 mmol/g) (5 mmol-20eq.). On agite 2 h à température ambiante, filtre sur Iéna, lave par CH3CN puis décroche par 10 cm3 de CH3CN/TFA : 95/05 sous agitation. On filtre sur Iéna, rince par CH3CN/TFA : 95/05 puis porte à sec sous vide. On récupère 180 mg d'un produit amorphe orange que l'on reprend par 7 cm3 d'éluant HPLC CH3CN/H2O/TFA : 27/73/0.1. On filtre sur toupie-0.45 µm puis injecte en 4 fois en HPLC préparative : Kromasil C18 - 10 µm - 500x22 mm - λ= 225 nm.- 15 ml/min. On regroupe les fractions propres que l'on concentre sous vide puis lyophilise. On isole le produit attendu sous forme de solide jaune de poids 32 mg.

### Analyses :

Spectre ¹H RMN : DMSO D⁶ δ (ppm)
2(dl), 3,07(m) : 2H ; 3, 16 (m), 3,47(m) : 2H ; 3,50 (m) : 2H ; 2,74(d) : CH3-N⁺H ; 5, 11 (s1), 1,78 (s) : 1H(éq) ; 1,78 (s) : 3H ; 3,59(masqué) : 1H(ax) ; 6,37(s) : 1H ; 6,68(s) : 1H ; 8,07(s), 8,03(s) : 2H ; 9, 32 (s1) : N⁺H ; 11, 39 (s), 12, 89 (s), 14,0(s1) : 3H(mobiles).

### Exemple 15 : chlorhydrate de 2,5-dichloro-4-[5,7-dihydroxy-8-[(3S,4R)-3-hydroxy-1-méthyl-4-piperidinyl]-4-oxo-4H-benzopyran-2-yl]-benzoate de méthyle

Dans un ballon de 30 cm3, sous N2, on introduit 20 mg du produit de l'exemple 14 (0.032 mmol), 0.5 cm3 de MeOH anhydre et 0.01 cm3 de chlorure de triméthylsilyle (M= 108.64 d=0.856) (2.5 eq 0.08 mmol). On laisse 24h à t. amb. On rajoute 0.05 cm3 de chlorure de triméthylsilyle. On porte le milieu réactionnel à 50°C pendant 20h. On amène à sec sous vide au rotavapor et reprend le produit par 20 cm3 d'eau. On met à lyophiliser. Le produit attendu est obtenu sous forme de solide jaune de poids 9.7 mg.

### Exemple 16 : trifluoroacétate de 5,7-dihydroxy-2-(2,5-diméthyl-3-furanyl)-8-[(3S,4R)-3-hydroxy-1-méthyl-4-piperidinyl]-4H-benzopyran-4-one

### Stade a) : 5,7-diméthoxy-2-(2,5-diméthyl-3-furanyl)-8-[(3S,4R)-3-hydroxy-1-méthyl-4-piperidinyl]-4H-benzopyran-4-one

### 1) Condensation : 1-3-[2-hydroxy-4,6-dimethoxy-3-[(3S,4R)-3-hydroxy-1-méthyl-4-piperidinyl]phényl]-1,3-propanedione

Dans un ballon de 30 cm3, sous N2, on introduit 310 mg de NaH à 50 % dans l'huile (M=24) (6.5 mmol-4eq.), 10 cm3 de THF anhydre, 500 mg d'Acétoflocinopipéridol (1.6 mmol) par fractions, 2 gouttes d'EtOH et 12 mg de Dibenzo-18-Crown-6 (M=360.41) (0.03 mmol-2 % molaire). On agite 5 min à température ambiante, puis introduit 685 µl de 2,5-diméthyl-3-furancarboxylate de méthyle (M=154.17, d=1.092) (4.8 mmol-3eq.). On agite 17h à température ambiante, puis on porte à reflux pendant 4h30.

### 2) Cyclisation : 5,7-diméthoxy-2-(2,5-diméthyl-3-furanyl)-8-[(3S,4R)-3-hydroxy-1-méthyl-4-piperidinyl]-4H-benzopyran-4-one

On ajoute au milieu réactionnel obtenu ci-dessus en 1), 10 cm3 de HCl à 36 % (116 mmol-72eq.) et maintient à reflux pendant 2h. On revient à température ambiante, verse dans 100 cm3 de NaOH(2N), extrait par 2x100 cm3 de CH2Cl2/MeOH : 8/2, lave par une solution saturée en NaCl, sèche sur Na2SO4 anhydre et porte à sec sous vide. On obtient 2.13 g d'une huile marron que l'on chromatographie sur silice 0,04-0,06 mm successivement dans CH2Cl2/MeOH : 80/20 puis dans CH2Cl2/MeOH : 90/10. On isole le produit attendu sous forme de résine marron de poids 89 mg.

### Analyses :

Spectre ¹H RMN : DMSO D⁶ δ (ppm)
1, 46 (d) , 3,05(m) : 2H ; 1, 98 (t) , 2,90(m) : 2H ; 2, 17 (d) , 2,80(m) : 2H ; 2,22(s), 2,28(s) : 6H ; 2,52 (s) : N-CH3 ; 3,32 (masqué) : 1H ; 3,73 (sl) : 1H(éq) ; 3,87(s), 3,91(s) : 6H ; 6, 14 (s) : 1H ; 6,49(s) : 1H ; 6,61 (s) : 1H.

### Stade b) : trifluoroacétate de 5,7-dihydroxy-2-(2,5-diméthyl-3-furanyl)-8-[(3S,4R)-3-hydroxy-1-méthyl-4-piperidinyl]-4H-benzopyran-4-one

Dans un ballon de 30 cm3 avec réfrigérant à reflux, sous N2, on introduit 89 mg du diméthoxy obtenu ci-dessus au stade a) (0.22 mmol), 2.5 cm3 de DMF anhydre et 538 mg de Reillex-pyridine -HI (≈ 4 mmol/g) (2.15 mmol-10 eq.) obtenu à la préparation 2) ci-dessus. On porte à 130°C pendant 20h30 puis à 150°C pendant 3h. On revient ensuite à température ambiante, dilue par CH3CN, filtre sur Iéna, rince par CH3CN puis ajoute 1.7 g de résine PTBD (2.6 mmol/g) (4.4 mmol-20eq.). On agite 2h30 à température ambiante, filtre sur Iéna, lave par CH3CN puis décroche par 20 cm3 de CH3CN/TFA : 95/05 sous agitation. On filtre sur Iéna, rince par CH3CN/TFA : 95/05 puis porte à sec sous vide. On récupère 135 mg d'une huile marron que l'on reprend par 3 cm3 d'eau et quelques gouttes de CH3CN puis que l'on dépose sur une cartouche de 2 g de C18. On élue la cartouche successivement par 10 cm3 de CH3CN/H2O : 5/95, 20 cm3 de CH3CN/H2O : 20/80, 40 cm3 de CH3CN/H2O : 50/50 et 20 cm3 de CH3CN pur. On regroupe les fractions propres que l'on concentre sous vide puis lyophilise. On isole le produit attendu sous forme de solide jaune pâle de poids 49 mg.

### Analyses :

Spectre ¹H RMN : DMSO D⁶ δ (ppm)
1,80(d), 3,11(masqué) : 2H ; 3,11, 3,47(masqué) : 2H ; 2,32(s), 2,59(s) : 6H ; 2,81(s) : N-CH3 ; 3,27(d), 3,41 (masqué) : 2H ; 3,52(masqué) : 1H (ax) ; 4,15(sl) : 1H (éq) ; 6, 31 (s) : 1H ; 6,42(s) : 1H ; 6,62(s) : 1H ; 9,30 (sl), 11,17(sl), 13,22(s) : 3H(mobiles).

### Exemple 17 : trifluoroacétate de 2-[4-(diéthylamino)-phényl]-5,7-dihydroxy-8-[(3S,4R)-3-hydroxy-1-méthyl-4-piperidinyl]-4H-benzopyran-4-one

### Stade a) : 2-[4-(diéthylamino)-phény]-5,7-diméthoxy-8-[(3S,4R)-3-hydroxy-1-méthyl-4-piperidinyl]-4H-benzopyran-4-one

### 1) Condensation : 1-[4-(diéthylamino)-phény]-3-[2-hydroxy-4,6-diméthoxy-3-[(3S,4R)-3-hydroxy-1-méthyl-4-piperidinyl] phényl]-1,3-propanedione

Dans un tube sec de 30 cm3, sous Ar, on introduit 310 mg de NaH à 50 % dans l'huile (M=24) (6.5 mmol-4eq.), 10 cm3 de THF anhydre, 500 mg d'Acétoflocinopipéridol (1.6 mmol) par fractions, 2 gouttes d'EtOH et 12 mg de Dibenzo-18-Crown-6 (M=360.41) (0.03 mmol-2 % molaire). On agite 1h à température ambiante, puis introduit 1.00 g de 4-(diéthylamino)- benzoate de méthyle (M=207.27) (4.8 mmol-3eq.) On maintient sous agitation à température ambiante, pendant 19h puis on porte à reflux pendant 22h30. On revient ensuite à température ambiante.

### 2) Cyclisation : 2-[4-(diéthylamino)-phény]-5,7-diméthoxy-8-[(3S,4R)-3-hydroxy-1-méthyl-4-piperidinyl]-4H-benzopyran-4-one

On ajoute au milieu réactionnel obtenu ci-dessus en 1), 10 cm3 de HCl àw 36 % (116-mmol-72eq.) et porte alors à 60°C pendant 2h. On revient à température ambiante, verse dans 100 cm3 de NaOH (2N), extrait par 2x200 cm3 de CH2Cl2/MeOH : 8/2, lave par une solution saturée en NaCl, sèche sur Na2SO4 anhydre et porte à sec sous vide. On obtient 1.28 g d'une huile marron que l'on chromatographie sur silice 0,04-0,06 mm dans CH2Cl2/MeOH : 90/10. On isole le produit attendu amorphe jaune de 152 mg.

### Analyses :

Spectre IR :
-OH complexe : 3520 cm-1 ; -C=O : 1655 cm-1 ; Système conjugué + aromatique : 1621, 1596, 1568, 1494 cm-1.
Spectre ¹H RMN : CDCl3 δ (ppm)
1,23(t) : 6H ; 3,45(q) : 4H ; 1, 61 (d1) , 3,12(masqué) : 2H ; 2,16(tl), 3,09 (masqué) : 2H ; 2,33(dl), 3,15(masqué) : 2H ; 3,64(ddd) : 1H(axial) ; 2,41(s) : CH3-N ; 3,96(s), 3,99(s) : 6H ; 2,81(s) : N-CH3 ; 4,04(s1) : 1H(éq) ; 6,43(s) : 1H ; 6,53(s) : 1H ; 6,73, 7,66 AA' BB' : 4H.

### Stade b) : trifluoroacétate de 2-[4-(diéthylamino)-phényl]-5,7-dihydroxy-8-[(3S,4R)-3-hydroxy-1-méthyl-4-piperidinyl]-4H-benzopyran-4-one

Dans un ballon de 30 cm3 avec réfrigérant à reflux, sous N2, on introduit 140 mg du diméthoxy obtenu au stade a) ci-dessus (0.30 mmol), 3 cm3 de DMF anhydre et 750 mg de Reillex-pyridine -HI (≈ 4 mmol/g) (3.0 mmol-10 eq.) obtenu comme indiqué à la préparation 2) ci-dessus. On porte à 130°C pendant 37h et à 150°C pendant 8h. On revient ensuite à température ambiante, dilue par CH3CN, filtre sur Iéna, rince par CH3CN puis ajoute 2.3 g de résine PTBD (2.6 mmol/g) (6 mmol-20eq.). On agite 1h à température ambiante, filtre sur Iéna, lave par CH3CN puis décroche par 20 cm3 de CH3CN/TFA : 95/05 sous agitation. On filtre sur Iéna, rince par CH3CN/TFA : 95/05 puis porte à sec sous vide. On récupère 304 mg d'un produit amorphe orange que l'on reprend par 4 cm3 d'éluant HPLC : CH3CN/H2O/TFA : 30/70/0.1. On filtre sur toupie-0.45 µm puis injecte en 4 fois en HPLC préparative Kromasil C18 - 10 *µ*m - 500x22 mm - λ= 225 nm -15 ml/min. On regroupe les fractions propres que l'on concentre sous vide puis lyophilise. On isole le produit attendu sous forme de solide jaune de poids 109 mg.

### Analyses :

Spectre ¹H RMN : DMSO D⁶ δ (ppm)
1,15(t) : 6H ; 3,45 (masqué) : 4H ; 1, 81 (d) , 3,21 (masqué) : 2H ; 3,21, 3,43 (masqué) : 2H ; 3,42(m) : 2H ; 3,52 (masqué) : 1H (ax) ; 4,10 (s) : 1H (éq) ; 2,80 (s) : N-CH3 ; 6,29 (s) : 1H ; 6,69 (s) : 1H ; 6,80, 7,91 AA'BB' : 4H ; 6,02 (s1), 9,41(s1), 10,48(s1), 13, 47 (s1) : 4H (mobiles).

### Exemple 18 : trifluoroacétate de 5,7-dihydroxy-8-[(3S, 4R)-3-hydroxy-1-méthyl-4-piperidinyl]-2-[3-(trifluorométhoxy)-phényl]-4H-benzopyran-4-one

### Stade a) : 5,7-diméthoxy-8-[(3S, 4R)-3-hydroxy-1-méthyl-4-piperidinyl]-2-[3-(trifluorométhoxy)-phényl]-4H-benzopyran-4-one

### 1) Condensation : 1-[2-hydroxy-4,6-diméthoxy-3-[(3S,4R)-3-hydroxy-1-méthyl-4-piperidinyl]phényl]-3-[3-(trifluorométhoxy)-phényl]-1,3-propanedione

Dans un ballon sec de 50 cm3, sous N2, on introduit 310 mg de NaH à 50 % dans l'huile (M=24) (6.5 mmol-4eq.), 10 cm3 de THF anhydre, 500 mg d'Acétoflocinopipéridol (1.6 mmol) par fractions, 2 gouttes d'EtOH et 12 mg de Dibenzo-18-Crown-6 (M=360.41) (0.03 mmol-2 % molaire). On agite ½h à température ambiante, puis introduit 1.1 g de 3-(trifluorométhoxy)-benzoate de méthyle (M=220.15) (5.0 mmol-3eq.). On maintient sous agitation à température ambiante pendant 18h.

### 2) Cyclisation : 5,7-diméthoxy-8-[(3S,4R)-3-hydroxy-1-méthyl-4-piperidinyl]-2-[3-(trifluorométhoxy)-phényl]-4H-benzopyran-4-one

On ajoute au milieu réactionnel obtenu ci-dessus en 1) 10 cm3 de HCl à 36 % (116 mmol-72eq.). On porte alors à 55°C pendant 2h. On revient à température ambiante, verse dans 100 cm3 de NaOH(2N), extrait par 2x200 cm3 de CH2Cl2/MeOH : 8/2, lave par une solution saturée en NaCl, sèche sur Na2SO4 anhydre et porte à sec sous vide. On obtient 1.33 g d'une huile orange que l'on chromatographie sur silice 0,04-0,06 mm dans CH2Cl2/MeOH/NH4OH : 90/10/1. On isole le produit attendu sous forme de solide crème de 384 mg.

### Analyses :

Spectre IR : - Absorption région OH/NH
-C=O : 1645 cm-1 ; -C=C + aromatique : 1622, 1596, 1568 cm-1. Spectre ¹H RMN : DMSO D⁶ δ (ppm)
1,46(dl), 3,07(m) : 2H ; 1,93(tl), 2,88(masqué) : 2H ; 2,16(masqué), 2,90(masqué) : 2H ; 2,20(s) : CH3-N ; 3,33 (masqué) : 1H (ax) ; 3,77 (sl) : 1H(éq) ; 3,89(s), 3,93(s) : 6H ; 4,32(dl) : 1H(OH) ; 6,65(s) : 1H ; 6, 82 (s) : 1H ; 7,59 (d1) : 1H ; 8,15(d1) : 1H ; 7,71 (t) : 1H ; 8, 13 (s1) : 1H.

### Stade b) : trifluoroacétate de 5,7-dihydroxy-8-[(3S,4R)-3-hydroxy-1-méthyl-4-piperidinyl)-2-[3-(trifluorométhoxy)-phényl]-4H-benzopyran-4-one

Dans un ballon de 30 cm3 avec réfrigérant à reflux, sous N2, on introduit 106 mg du diméthoxy obtenu au stade a) ci-dessus (0.22 mmol), 2.5 cm3 de DMF anhydre et 538 mg de Reillex-pyridine -HI (≈ 4 mmol/g) (2.15 mmol-10 eq.) obtenu comme indiqué à la préparation 2) ci-dessus. On porte à 130°C pendant 18h et à 150°C pendant 7h. On revient ensuite à température ambiante, dilue par CH3CN, filtre sur Iéna, rince par CH3CN puis ajoute 1.7 g de résine PTBD (2.6 mmol/g) (4.4 mmol-20eq.). On agite 2h à température ambiante, filtre sur Iéna, lave par CH3CN puis décroche par 20 cm3 de CH3CN/TFA : 95/05 sous agitation. On filtre sur Iéna, rince par CH3CN/TFA : 95/05 puis porte à sec sous vide. On récupère 152 mg d'une résine jaune que l'on reprend par 2 cm3 d'éluant HPLC : CH3CN/H2O/TFA : 40/60/0.1. On filtre sur toupie-0.45 µm puis injecte en 2 fois en HPLC préparative : Kromasil C18 - 10 µm - 500x22 mm λ= 225 nm.- 15 ml/min. On regroupe les fractions propres que l'on concentre sous vide puis lyophilise. On isole le produit attendu sous forme de solide jaune de poids 88 mg.

### Analyses :

Spectre ¹H RMN : DMSO D⁶ δ (ppm)
1,87(dl), 3,15(m) : 2H ; 2,79(sl) : CH3-N ; 3,19(m), 3,45(m) : 2H ; 3,38(m) : 2H ; 3,55 (masqué) : 1H(ax) ; 4,11(sl) : 1H(éq) ; 6,37(s) : 1H ; 7,05(s) : 1H ; 5,99(sl) : 1H (OH) ; 7,67(dl) : 1H ; 7, 77 (tl) : 1H ; 8,05(sl) : 1H ; 8,17(dl) : 1H ; 9,43(sl), 11,26(sl), 13,08(s) : 3H (mobiles).

### Exemple 19 : trifluoroacétate de 2-[3,5-bis(trifluorométhyl) -phény]-5,7-dihydroxy-8-[(3S,4R)-3-hydroxy-1-méthyl-4-piperidinyl]- 4H-benzopyran-4-one

### Stade a) : 2-[3,5-bis(trifluorométhyl)-phényl]-5,7-diméthoxy-8-[(3S,4R)-3-hydroxy-1-méthyl-4-piperidinyl]-4H-benzopyran-4-one

### 1) Condensation : 1-[3,5-bis(trifluorométhyl)-phény]-3-[2-hydroxy-4,6-diméthoxy-3-[(3S,4R)-3-hydroxy-1-méthyl-4-piperidinyl) phényl)-1,3-propanedione

Dans un ballon sec de 50 cm3, sous N2, on introduit 310 mg de NaH à 50 % dans l'huile (M=24) (6.5 mmol-4eq.), 10 cm3 de THF anhydre, 500 mg d'Acétoflocinopipéridol (1.6 mmol) par fractions, 2 gouttes d'EtOH et 12 mg de Dibenzo-18-Crown-6 (M=360.41) (0.03 mmol-2 % molaire). On agite ½h à température ambiante, puis introduit 1.3 g de 3,5-bis(trifluorométhyl)-benzoate de méthyle(M=272.15) (4.8 mmol-3eq.) et maintient sous agitation à température ambiante pendant 18h.

### 2) Cyclisation : 2-[3,5-bis(trifluorométhyl)-phényl]-5,7-diméthoxy-8-[(3S,4R)-3-hydroxy-1-méthyl-4-piperidinyl]-4H-benzopyran-4-one

On ajoute au milieu réactionnel obtenu ci-dessus en 1), 10 cm3 de HCl à 36 % (116 mmol-72eq.) et porte alors à 55°C pendant 2h. On revient à température ambiante, verse dans 100 cm3 de NaOH (2N), extrait par 2x200 cm3 de CH2Cl2/MeOH : 8/2, lave par une solution saturée en NaCl, sèche sur Na2SO4 anhydre et porte à sec sous vide. On obtient 985 mg d'un solide orange que l'on empâte par 15 cm3 d'éther isopropylique au reflux, laisse revenir à température ambiante, filtre puis sèche sous vide. On isole le produit attendu sous forme de solide jaune de poids 503 mg.

### Analyses :

Spectre IR : - Absorption région OH/NH
-C=O : 1660 cm-1 ; -C=C + aromatique : 1632, 1622, 1573, 1565 cm-1.

### Analyses :

Spectre ¹H RMN : DMSO D⁶ δ (ppm)
1,49(d1), 3,05(ddt) . 2H ; 1,93(tl), 2,89(s1) . 2H ; 2,16 (masqué), 2,88(sl) : 2H ; 2,20(s) : CH3-N ; 3,35(masqué) : 1H (ax) ; 3,78 (s1) : 1H (éq) ; 3, 90 (s), 3,93(s) : 6H ; 4,29(d1) : 1H (OH) ; 6, 66 (s) . 1H ; 7,15(s) : 1H ; 8, 31 (s1), 8,76(s1) : 3H.

### Stade b) : trifluoroacétate de 2-[3,5-bis(trifluorométhyl)-phényl-5,7-dihydroxy-8-[(3S,4R)-3-hydroxy-1-méthyl-4-piperidinyl]-4H-benzopyran-4-one

Dans un ballon de 20 cm3 avec réfrigérant à reflux, sous N2, on introduit 117 mg du diméthoxy obtenu au stade a) ci-dessus (0.22 mmol), 2.5 cm3 de DMF anhydre et 538 mg de Reillex-pyridine -HI (≈ 4 mmol/g) (2.15 mmol-10 eq.) obtenu comme indiqué à la préparation 2) ci-dessus. On porte à 150°C pendant 6h et à 130°C pendant 19h. On revient ensuite à température ambiante, dilue par CH3CN, filtre sur Iéna, rince par CH3CN puis ajoute 1.7 g de résine PTBD (2.6 mmol/g) (4.4 mmol-20eq.). On agite 2h à température ambiante, filtre sur Iéna, lave par CH3CN puis décroche par 20 cm3 de CH3CN/TFA : 95/05 sous agitation. On filtre sur Iéna, rince par CH3CN/TFA : 95/05 puis porte à sec sous vide. On récupère 158 mg d'une résine jaune que l'on reprend par 2 cm3 d'éluant HPLC : CH3CN/H2O/TFA : 45/55/0.1. On filtre sur toupie-0.45 µm puis injecte en 2 fois en HPLC préparative : Kromasil C18 - 10 *µ*m - 500x22 mm - λ= 225 nm -15 ml/min. On regroupe les fractions propres que l'on concentre sous vide puis lyophilise. On isole le produit attendu sous forme de solide légèrement jaune de poids 41 mg.

### Analyses :

Spectre ¹H RMN : DMSO D⁶ δ(ppm)
2,05(dl), 3,15(m) : 2H ; 2,76(sl) : CH3-N ; 3,14(m), 3,40(masqué) : 2H ; 3,21(m), 3,40 (masqué) : 2H ; 3,59(d1) : 1H(ax) ; 4,18(s1) : 1H (éq) ; 6,38 (s) : 1H ; 7,30 (s) : 1H ; 8,43(s) : 1H ; 8,62(s) : 1H 5, 96 (s1), 9, 46 (s1), 11, 25 (s1), 12,93 (s1) : 4H(mobiles).

### Exemple 20 : trifluoroacétate de 2-(2,6-dichloro-4-pyridinyl) -5,7-dihydroxy-8-[(3S,4R)-3-hydroxy-1-méthyl-4-piperidinyl]-4H-benzopyran-4-one

### Stade a) : 2-(2,6-dichloro-4-pyridinyl)-5,7-diméthoxy-8-[(3S,4R)-3-hydroxy-1-méthyl-4-piperidinyl]-4H-benzopyran-4-one

### 1) Condensation : 1-(2,6-dichloro-4-pyridinyl)-3-[2-hydroxy-4,6-diméthoxy-3-[(3S,4R)-3-hydroxy-1-méthyl-4-piperidinyl] phényl]-1,3-propanedione

Dans un ballon de 30 cm3, sous N2, on introduit 310 mg de NaH à 50 % dans l'huile (M=24) (6.5 mmol-4eq.), 10 cm3 de THF anhydre, 500 mg d'Acétoflocinopipéridol (1.6 mmol) par fractions, 2 gouttes d'EtOH et 12 mg de Dibenzo-18-Crown-6 (M=360.41) (0.03 mmol-2 % molaire). On agite ½h à température ambiante, puis introduit 1.00 g de 2,6-dichloro-4-pyridinecarboxylate de méthyle (M=360.41) (4.8 mmol-3eq.). On maintient sous agitation à température ambiante pendant 18h.

### 2) Cyclisation : 2-(2,6-dichloro-4-pyridinyl)-5,7-diméthoxy-8-[(3S,4R)-3-hydroxy-1-méthyl-4-piperidinyl]-4H-benzopyran-4-one

On ajoute au milieu réactionnel obtenu ci-dessus en 1), 10 cm3 de HCl à 36 % (116 mmol-72eq.) et porte alors à 50°C pendant 2h. On revient à température ambiante, verse dans 100 cm3 de NaOH(2N), extrait par 2x200 cm3 de CH2Cl2/MeOH : 8/2, lave par une solution saturée en NaCl, sèche sur Na2SO4 anhydre et porte à sec sous vide. On obtient 820 mg d'un solide jaune que l'on chromatographie sur silice 0,04-0,06 mm dans CH2Cl2/MeOH/NH4OH : 90/10/1. On isole le produit attendu sous forme de solide jaune pâle de poids 384 mg.

### Analyses :

Spectre IR : - Absorption région OH/NH
-C=O : 1658 cm-1 ; Système conjugué + aromatique : 1632, 1595, 1585, 1568, 1528 cm-1.
Spectre ¹H RMN : DMSO D⁶ δ (ppm)
1,53(dl), 3,06(masqué) : 2H ; 2,09(tl), 2,97(masqué) : 2H ; 2,31(masqué), 2,97 (masqué) : 2H ; 3,83 (masqué) : 1H (éq) ; 3,37(dl) : 1H ; 2,29 (sl) : CH3-N ; 3,90(s), 3,93(s) : 6H ; 6,66 (s) : 1H ; 6,98 (s) : 1H ; 8,16 (s) : 2H.

### Stade b) : trifluoroacétate de 2-(2,6-dichloro-4-pyridinyl)-5,7-dihydroxy-8-[(3S,4R)-3-hydroxy-1-méthyl-4-piperidinyl]-4H-benzopyran-4-one

Dans un ballon de 50 cm3 avec réfrigérant à reflux, sous N2, on introduit 150 mg du diméthoxy obtenu au stade a) ci-dessus (0.32 mmol), 3.5 cm3 de DMF anhydre et 806 mg de Reillex-pyridine -HI (= 4 mmol/g) (3.22 mmol-10 eq.) obtenu comme indiqué à la préparation 2) ci-dessus. On porte à 130°C pendant 33h et à 150°C pendant 12h30. On revient ensuite à température ambiante, dilue par CH3CN, filtre sur Iéna, rince par CH3CN puis ajoute 2.5 g de résine PTBD (2.6 mmol/g) (6.5 mmol-20eq.). On agite 1h30 à température ambiante, filtre sur Iéna, lave par CH3CN puis décroche par 40 cm3 de CH3CN/TFA : 95/05 sous agitation. On filtre sur Iéna, rince par CH3CN/ TFA : 95/05 puis porte à sec sous vide. On récupère 211 mg d'une résine jaune que l'on reprend par 5 cm3 d'éluant HPLC : CH3CN/H2O/TFA : 33/67/0.1. On filtre sur toupie-0.45 µm puis injecte en 5 fois en HPLC préparative : Kromasil C18 - 10 µm - 500x22 mm λ= 225 nm- 15 ml/min. On regroupe les fractions propres que l'on concentre sous vide puis lyophilise. On isole le produit attendu sous forme de solide jaune de poids 39 mg.

### Analyses :

Spectre ¹H RMN : DMSO D⁶ δ (ppm)
1,91(dl), 3,11(m) : 2H ; 3,20(m), 3,44(masqué) : 2H ; 3,31(m), 3,39(masqué) : 2H ; 2,79(sl) . CH3-N ; 3,56 (dl) : 1H(ax) ; 4,15(sl) : 1H(éq) ; 6,38(s) : 1H ; 7,25(s) : 1H ; 8,11(sl) : 2H ; 9,37 (sl), 11,39 (sl), 12,82(s), 6,04 (sl) : 4H (mobiles).

### Exemple 21 : trifluoroacétate de 2-[5-bromo-2-furanyl]-5,7-dihydroxy-8-[(3S,4R)-3-hydroxy-1-méthyl-4-piperidinyl]-4H-benzopyran-4-one

### Stade a) : 2-[5-bromo-2-furanyl]-5,7-diméthoxy-8-[(3S,4R)-3-hydroxy-1-méthyl-4-piperidinyl]-4H-benzopyran-4-one

### 1) Condensation : 1-[5-bromo-2-furanyl]-3-[2-hydroxy-4,6-diméthoxy-3-[(3S,4R)-3-hydroxy-1-méthyl-4-piperidinyl] phényl]-1,3-propanedione

Dans un ballon de 30 cm3, sous N2, on introduit 310 mg de NaH à 50 % dans l'huile (M=24) (6.5 mmol-4eq.), 10 cm3 de THF anhydre, 500 mg d'Acétoflocinopipéridol (1.6 mmol) par fractions, 2 gouttes d'EtOH et 12 mg de Dibenzo-18-Crown-6 (M=360.41) (0.03 mmol-2 % molaire). On agite 10 min à température ambiante, puis introduit 994 mg de 5-bromo-2-furancarboxylate de méthyle (M=201.05) (4.85 mmol-3eq.). On maintient sous agitation à température ambiante pendant 18h.

### 2) Cyclisation : 2-[5-bromo-2-furanyl]-5,7-diméthoxy-8-[(3S,4R)-3-hydroxy-1-méthyl-4-piperidinyl]-4H-benzopyran-4-one

On ajoute au milieu réactionnel obtenu ci-dessus en 1) 10 cm3 de HCl à 36 % (116 mmol-72eq.) et porte alors à 50°C pendant 1h30. On revient à température ambiante, verse dans 100 cm3 de NaOH (2N), extrait par 2x100 cm3 de CH2Cl2/MeOH : 8/2, lave par une solution saturée en NaCl, sèche sur Na2SO4 anhydre et porte à sec sous vide. On obtient 1.48 g d'une huile marron que l'on chromatographie sur silice 0,04-0,06 mm dans CH2Cl2/MeOH : 90/10. On isole le produit attendu amorphe jaune de poids 321 mg

### Analyses :

Spectre IR : Absorption région OH/NH
-C=O : 1655 cm-1 ; Système conjugué + aromatique : 1621, 1596, 1568, 1494 cm-1.
Spectre ¹H RMN : DMSO D⁶ δ(ppm)
1,53(d1), 3,10(d1) : 2H ; 2,37(masqué), 2,97(d1) : 2H ; 2,15(t1), 3,01(d1) : 2H ; 2,30(s) : CH3-N ; 3,30(masqué) : 1H(ax) ; 3,76(s1) : 1H(éq) ; 3,88(s), 3,91 (s) : 6H ; 4,36(s1) : 1H (OH) ; 6,33 (s) : 1H ; 6, 63 (s) : 1H ; 6, 95 (d), 7,37(d) : 2H.

### Stade b) : trifluoroacétate de 2-[5-bromo-2-furanyl]-5,7-dihydroxy-8-[(3S,4R)-3-hydroxy-1-méthyl-4-piperidinyl]-4H-benzopyran-4-one

Dans un ballon de 30 cm3 avec réfrigérant à reflux, sous N2, on introduit 140 mg du diméthoxy obtenu au stade a) ci-dessus (0.3 mmol), 3 cm3 de DMF anhydre et 754 mg de Reillex-pyridine -HI (≈ 4 mmol/g) (3 mmol-10 eq.) obtenu comme indiqué à la préparation 2) ci-dessus. On porte à 130°C pendant 40h30 et à 150°C pendant 2h. On revient ensuite à température ambiante, dilue par CH3CN, filtre sur Iéna, rince par CH3CN puis ajoute 2.3 g de résine PTBD (2.6 mmol/g) (6.0 mmol-20 eq.). On agite 1h30 à température ambiante, filtre sur Iéna, lave par CH3CN puis décroche par 10 cm3 de CH3CN/TFA : 95/05 sous agitation. On filtre sur Iéna, rince par CH3CN/TFA : 95/05 puis porte à sec sous vide. On récupère 287 mg d'un produit amorphe marron que l'on reprend par 4 cm3 d'éluant HPLC : CH3CN/H2O/TFA : 30/70/0.1. On filtre sur toupie-0.45 µm puis injecte en 2 fois en HPLC préparative : Kromasil C18 - 10 µm - 500x22 mm - λ= 225 nm - 15 ml/min. On regroupe les fractions propres que l'on concentre sous vide puis lyophilise. On isole le produit attendu sous forme de solide jaune de poids 44 mg.

### Analyses :

Spectre ¹H RMN : DMSO D⁶ δ (ppm)
1,80 (m), 3,17 (masqué) : 2H ; 2,81 (d) : NH⁺-CH3 ; 3,18(m), 3,47 (m) : 2H ; 3,39 (m) : 2H ; 3,48 (masqué) : 1H (ax) ; 4,04 (s) : 1H (éq) ; 6,34 (s), 6,52 (s) : 2H ; 7,03 (d), 7, 56 (d) . 2H ; 5,79(s), 9,39(s), 11,24(s), 13,05 (s) : 4H(mobiles).

### Exemple 22 : trifluoroacétate de 8-[(3S,4R)-3-acétyloxy-1-méthyl-4-piperidinyl]- 2-(5-bromo-2-furanyl) 5,7-dihydroxy-4H-benzopyran-4-one

Dans un ballon avec réfrigérant à reflux, sous N2, on introduit 140 mg du produit obtenu au stade a) de l'exemple 21 (0.30 mmol), 3 cm3 de DMA et 625 mg de Pyridine-HI (M=207.01) (3.0 mmol-10 eq.) obtenu comme indiqué à la préparation 1) ci-dessus. On porte à 130°C pendant 40h et à 150°C pendant 5h30. On revient ensuite à température ambiante, dilue par CH3CN, puis ajoute 2.3 g de résine PTBD (2.6 mmol/g) (6.0 mmol-20eq.). On agite 1h20 à température ambiante, filtre sur Iéna, lave par CH3CN puis décroche par 50 cm3 de CH3CN/TFA : 95/05 puis porte à sec sous vide. On récupère 241 mg d'une résine rouge que l'on reprend par 8 cm3 d'éluant HPLC CH3CN/H2O/TFA : 35/65/0.1. décrit ci-après. On filtre sur toupie-0.45 µm puis injecte en 4 fois en HPLC préparative : Kromasil C18 - 10 *µ*m - 500x22 mm - À= 225 nm - 15 ml/min. On regroupe les fractions propres que l'on porte à sec sous vide, reprend à l'eau puis lyophilise. On isole le produit attendu sous forme de solide crème de poids 47 mg.

### Analyses :

Spectre ¹H RMN : DMSO D⁶ δ (ppm)
1,94(d1), 3,21(m) : 2H ; 2,85(s) : CH3-N ; 3,26(masqué), 3,59(m) : 2H ; 3,59 (m) : 2H ; 3,68(t1) : 1H(ax) ; 5,09 (s1) : 1H (éq) ; 6,33(s), 6,52(s) : 2H ; 7, 03 (d), 7,56(d) : 2H ; 9,82(s1) : N⁺H ; 11,38(s), 13,06(s) : 2H ; 1,78(s) : 3H.

### Exemple 23 : trifluoroacétate de 2-cyclohexyl-5,7-dihydroxy-8-[(3S,4R)-3-hydroxy-1-méthyl-4-piperidinyl]-4H-benzopyran-4-one

### Stade a) : 2- cyclohexyl-5,7-diméthoxy-8-[(3S,4R)-3-hydroxy-1-méthyl-4-piperidinyl]-4H-benzopyran-4-one

### 1) Condensation : 1-cyclohexyl-3-[2-hydroxy-4,6-diméthoxy-3-[(3S,4R)-3-hydroxy-1-méthyl-4-piperidinyl]phényl]-1,3-propanedione

Dans un ballon sec de 50 cm3, sous N2, on introduit 370 mg de NaH à 50 % dans l'huile (M=24) (7.7 mmol-4eq.), 20 cm3 de DMSO/NK20 et 600 mg d'Acétoflocinopipéridol (1.9 mmol). On agite 1h à température ambiante, puis introduit 0.85 cm3 de cyclohexanecarboxylate de méthyle (M=142.2-d=0.995) (5.95 mmol-3eq.) On maintient sous agitation pendant 22h.

### 2) Cyclisation : 2-cyclohexyl-5,7-diméthoxy-8-[(3S,4R)-3-hydroxy-1-méthyl-4-piperidinyl]-4H-benzopyran-4-one

On ajoute au milieu réactionnel obtenu ci-dessus en 1), 13 cm3 de HCl à 36 % (151 mmol-78eq.). On porte alors à 50°C pendant 2h15. On revient à température ambiante, verse dans 200 cm3 de NaOH(N), extrait par 200 cm3 de CH2C12 puis 200 cm3 de CH2Cl2/MeOH : 9/1, lave par une solution saturée en NaCl, sèche sur Na2SO4 anhydre et porte à sec sous vide. On obtient 1.173 g d'une huile marron que l'on chromatographie sur silice 0,04-0,06 mm dans CH2Cl2/MeOH : 90/10. On isole le produit attendu sous forme de solide jaune de poids 511 mg.

### Analyses :

Spectre IR :
-OH : 3535 cm-1 ; -C=O : 1652 cm-1 ; -C=C + aromatiques : 1619, 1597, 1495 cm-1.
Spectre ¹H RMN : CDCl3 δ(ppm)
1,25(m), 1,77(m) : 2H ; 1,86(m), 1,42(m) : 4H ; 1,41(m), 2,50(m) : 4H ; 2,49(tt) : 1H ; 1,62(m), 3,13(m) : 2H ; 2,17(m), 3,11(m) : 2H ; 2,32(d), 3,13(m) : 2H ; 2,41(s) : N-CH3 ; 3,94(s), 3,96(s) : 6H ; 3,43(ddd) : 1H (ax) ; 3,90(masqué) : 1H (éq) ; 6,00(s) : 1H ; 6,39(s) : 1H.

### Stade b) : trifluoroacétate de 2-cyclohexyl-5,7-dihydroxy-8-[(3S,4R)-3-hydroxy-1-méthyl-4-piperidinyl]-4H-benzopyran-4-one

Dans un ballon de 20 cm3 avec réfrigérant à reflux, sous N2, on introduit 100 mg du diméthoxy obtenu au stade a) à ci-dessus (0.25 mmol) et 1.3 cm3 de HI à 57 %. On porte à 130°C pendant 1h. On refroidit à 0°C par un bain de glace puis basifie par 2 cm3 de NaOH (≈ 6 M) dans MeOH. On injecte en 2 fois en HPLC préparative : Kromasil C18 - 10 *µ*m - 500x22 mm λ= 225 nm - 15 ml/min. avec pour éluant MeOH/H2O/TFA : 55/45/0.1. On regroupe les fractions propres que l'on porte à sec sous vide, reprend par 1 cm3 de MeOH + 100 cm3 d'eau puis lyophilise. On isole ainsi le produit attendu sous forme de solide blanc de poids 29.4 mg.

### Analyses :

Spectre ¹H RMN : DMSO D⁶ δ (ppm)
1,75, 1,42 : 2H ; 1,36, 1,85 : 4H ; 1,95, 1,52 : 4H ; 2,61 : 1H ; 2,82 : CH3-N ; 3,39 : 2H ; 4,05 : 1H ; 3,40 : 1H ; 1,83, 3,48 : 2H ; 5,80 : 1H(OH) ; 6,16(s) : 1H ; 6,33(s) : 1H ; 13,15, 11,14 : 2H(mobiles).

### Exemple 24 : trifluoroacétate de 5,7-dihydroxy-8-[(3S,4R)-3-hydroxy-1-méthyl-4-piperidinyl]-2-[4-(trifluorométhoxy)-phényl]-4H-benzopyran-4-one

### Stade a) : 5,7-diméthoxy-8-[(3S,4R)-3-hydroxy-1-méthyl-4-piperidinyl]-2-[4-(trifluorométhoxy)-phényl]-4H-benzopyran-4-one

### 1) Condensation : 1-[2-hydroxy-4,6-diméthoxy-3-[(3S,4R)-3-hydroxy-1-méthyl-4-piperidinyl]phényl]-3-[4-(trifluorométhoxy)-phényl]-1,3-propanedione

Dans un ballon sec de 50 cm3, sous N2, on introduit 310 mg de NaH à 50 % dans l'huile (M=24) (6.5 mmol-4eq.), 16 cm3 de DMSO/NK20 et 500 mg d'Acétoflocinopipéridol (1.6 mmol) par fractions. On agite 1h à température ambiante, puis introduit 1.07 g de 4-(trifluorométhoxy)-benzoate de méthyle (M=220.16) (4.9 mmol-3eq.) On maintient sous agitation pendant 17h à température ambiante, puis on chauffe à 50°C pendant 2 h.

### 2) Cyclisation : 5,7-diméthoxy-8-[(3S,4R)-3-hydroxy-1-méthyl-4-piperidinyl]-2-[4-(trifluorométhoxy)-phényl]-4H-benzopyran-4-one

On ajoute au milieu réactionnel obtenu ci-dessus en 1), 11 cm3 de HCl à 36 % (128 mmol-79eq.) et maintient à 50°C pendant 1h30. On revient à température ambiante, verse dans 70 cm3 de NaOH(2N), extrait par 2×100 cm3 de CH2Cl2/MeOH : 8/2, lave par une solution saturée en NaCl, sèche sur Na2SO4 anhydre et porte à sec sous vide. On obtient 1.23 g d'une huile jaune que l'on chromatographie sur silice 0,04-0,06 mm dans CH2Cl2/MeOH : 90/10 puis 80/20. On isole ainsi le produit attendu sous forme de résine jaune de poids 242 mg.

### Analyses:

Spectre IR : - Absorption région OH/NH
-C=O : 1647 cm-1 ; -C=C + aromatique : 1597, 1583, 1570, 1508, 1498 cm-1.
Spectre ¹H RMN : DMSO D⁶ δ (ppm)
1, 51 (d1), 3,09 (d1) : 2H ; 2,98(d1), 2,15(t1) : 2H ; 2,98(d1), 2,39 (masqué) : 2H ; 3,90(s), 3,94(s) : 6H ; 2,32(s) : CH3-N ; 3,37(dl) : 1H (ax) ; 3,82(s) : 1H(éq) ; 6,66(s) : 1H ; 6,73(s) : 1H ; 7,56, 8,23 AA'BB' : 4H ; 4,54 : 1H (OH).

### Stade b) : trifluoroacétate de 5,7-dihydroxy-8-[(3S,4R)-3-hydroxy-1-méthyl-4-piperidinyl]-2-[4-(trifluorométhoxy)-phényl]-4H-benzopyran-4-one

Dans un ballon de 20 cm3 avec réfrigérant à reflux sous N2, on introduit 144 mg du produit obtenu au stade a) ci-dessus (0.30 mmol), 3 cm3 de DMF anhydre et 450 mg de Reillex-pyridine -HI (= 4 mmol/g) (1.8 mmol-6 eq.) obtenu comme indiqué à la préparation 2) ci-dessus. On porte à 120°C pendant 19h et à 150°C pendant 24h puis revient à température ambiante. On filtre sur Iéna et lave par 10 cm3 de CH3CN + 3 cm3 de MeOH, puis ajoute 1.2 g de résine PTBD (2.6 mmol/g) (3.1 mmol-10eq.) dans le filtrat. On agite 1 nuit à température ambiante, filtre sur Iéna, lave par CH3CN puis décroche par CH3CN/TFA : 95/05 sous agitation. On filtre sur Iéna, rince par CH3CN/TFA : 95/05 puis porte à sec sous vide. On récupère 171 mg d'un vernis jaune que l'on reprend par 6 cm3 d'éluant HPLC décrit ci-après. On filtre sur toupie-0.45 µm puis injecte en 3 fois en HPLC préparative : Kromasil C18 - 10 *µ*m - 500x22 mm λ= 225 nm - Eluant : CH3CN/H2O/TFA : 35/65/0.1 - 15 ml/min. On regroupe les fractions propres que l'on concentre sous vide puis lyophilise. On isole ainsi le produit attendu sous forme de solide jaune pâle de poids 77 mg.

### Analyses :

Spectre ¹H RMN : DMSO D⁶ δ(ppm)
1,82(dl), 3,18(m) : 2H ; 3,19(m), 3,49(masqué) : 2H ; 3,42(s1) : 2H ; 3,55(masqué) : 1H (ax) ; 2,83(d) : CH3-N ; 4,09(s1) : 1H(éq) ; 6,37(s) : 1H ; 7,00(s) : 1H ; 7,58, 8,27 AA' BB' : 4H ; 9,43, 11,27, 13,11, 5, 98 (sl) : 4H(mobiles).

### Exemple 25 : trifluoroacétate de l'acide 4-[5,7-dihydroxy-8-[(3S,4R)-3-hydroxy-1-méthyl-4-piperidinyl]-4-oxo-4H-benzopyran-2-yl]-benzoique

### Stade a) : 4-[5,7-diméthoxy-8-[(3S,4R)-3-hydroxy-1-méthyl-4-piperidinyl]-4-oxo-4H-benzopyran-2-yl]-benzoate de méthyle

### 1) Condensation : 4-[3-[2-hydroxy-4,6-diméthoxy-3-[(3S,4R)-3-hydroxy-1-méthyl-4-piperidinyl]phényl]1,3-dioxopropyl]-benzoate de méthyle

Dans un ballon de 100 cm3, sous N2, on introduit 196 mg de NaH à 50 % dans l'huile (M=24) (8 mmol-4 eq.), 5 cm3 de DMSO/NK20 et 309 mg d'Acétoflocinopipéridol (1 mmol). On agite 1h à température ambiante, puis introduit 582 mg de de 1,4-benzenedicarboxylate de diméthyle(M=194.1) (3 mmol-3eq.) et maintient sous agitation pendant 20h.

### 2) Cyclisation : 4-[5,7-diméthoxy-8-[(3S,4R)-3-hydroxy-1-méthyl-4-piperidinyl]-4-oxo-4H-benzopyran-2-yl]-benzoate de méthyle

On ajoute au milieu réactionnel obtenu en 1) ci-dessus 2.5 cm3 de HCl à 36 % (0.03 mmol-30eq.). On porte alors à 50°C pendant 2.5 h. On laisse pendant 48 h à t. amb. On traite en diluant le milieu réactionnel par 5 cm3 d'eau. On ajoute goutte à goutte 2.5 cm3 de NaOH à 33 %, extrait par 60 cm3 de CH2Cl2/MeOH : 9/1, lave à l'eau, sèche sur Na2SO4 anhydre et porte à sec sous vide. On obtient 314 mg d'une huile marron que l'on chromatographie sur silice 0,04-0,06 mm dans CH2Cl2/MeOH : 80/20. On isole ainsi le produit attendu sous forme de solide jaune de poids 220 mg.

### Analyses :

Spectre IR : .- Absorption région OH/NH
-C=O : 1722 cm-1 ; 1647 cm-1 ; Système conjugué + aromatique : 1619, 1596, 1574, 1494 cm-1.
Spectre ¹H RMN : CDCl3 δ (ppm)
1,63(m), 3,11 : 2H ; 2,19, 3,21 : 2H ; 2,39, 3,21 : 2H ; 2,44(s) : N-CH3 ; 3,56 (ddd) : 1H (ax) ; 4,05 (1) : 1H (éq) ; 3,45 : 1H (OH) ; 3,96(s), 3,97(s), 4, 50 (s) : 9H ; 6,44(s), 6,69(s) : 2 ; 7,89, 8,18 : 4H.

### Stade b) : trifluoroacétate de l'acide 4-[5,7-dihydroxy-8-[(3S,4R)-3-hydroxy-1-méthyl-4-piperidinyl]-4-oxo-4H-benzopyran-2-yl]-benzoique

Dans un ballon de 30 cm3 avec réfrigérant à reflux, sous N2, on introduit 90 mg du diméthoxy obtenu au stade a) ci-dessus (0.198 mmol), 1.9 cm3 de DMF anhydre et 1238 mg de Reillex-pyridine -HI (= 4 mmol/g) (4,87 mmol-25eq.) obtenu comme indiqué ci-dessus à la préparation 2). On porte à 130°C pendant environ 60h puis à 140°C pendant 5 h. On revient ensuite à température ambiante, dilue par MeOH + DMF, filtre sur Iéna, rince par DMF, le filtrat est amené à sec sous vide au rotavapor. Le résidu huileux est repris par 4 cm3 de l'éluant HPLC : MeOH/H2O/TFA : 55/45/0.1 - 15 ml/min. On filtre sur toupie-0.45 µm puis injecte en 4 fois en HPLC préparative : Kromasil C18 - 10 µm - 500x22 mm - λ= 225 nm. On regroupe les fractions propres que l'on concentre sous vide puis lyophilise. On isole ainsi le produit attendu sous forme de solide jaune de poids 20 mg.

### Analyses :

Spectre ¹H RMN : DMSO D⁶ δ (ppm)
3,41 : 2H ; 4,09 : 1H ; 3,54 : 1H ; 1,80, 3,18 : 2H ; 3,18, 3,46 : 2H ; 2,83 : N-CH3 ; 7,04 : 1H ; 6,37 : 1H ; 8,14, 8,24 : 4H ; 5,97, 9,34, 11,26, 13,08 : 4H (mobiles).

### Exemple 26 : trifluoroacétate de 2-[3-[(4-bromo-3,5-diméthyl-1H-pyrazol-1-yl)méthyl]-phényl]-5,7-dihydroxy-8-[(3S,4R)-3-hydroxy-1-méthyl-4-piperidinyl]-4H-benzopyran-4-one

### Stade a) : 2-[3-[(4-bromo-3,5-diméthyl-1H-pyrazol-1-yl) méthyl] -phényl] -5, 7-diméthoxy-8- [(3S, 4R) -3-hydroxy-1-méthyl-4-piperidinyl]-4H-benzopyran-4-one

### 1) Condensation : 1-[3-[(4-bromo-3,5-diméthyl-1H-pyrazol-1-yl)méthyl]-phényl]-3-[2-hydroxy-4,6-diméthoxy-3-[(3S,4R)-3-hydroxy-1-méthyl-4-piperidinyl]phényl]-1,3-propanedione

Dans un ballon, sous N2, on introduit 250 mg de NaH à 50 % dans l'huile (M=24) (5.2 mmol-2.7eq.), 6.5 cm3 de DMSO/NK20 et 600 mg d'Acétoflocinopipéridol (1.9 mmol). On agite 1h à température ambiante, puis introduit 1.460 g de 3-[(4-bromo-3,5-diméthyl-1H-pyrazol-1-yl)méthyl]-benzoate de méthyle (M=323.19) (4.5 mmol-2,3eq.) et maintient sous agitation pendant 22h à température ambiante, puis chauffe à 75°C avant de revenir à température ambiante pendant 18h.

### 2) Cyclisation : 2-[3-[(4-bromo-3,5-diméthyl-1H-pyrazol-1-yl)méthyl]-phényl]-5,7-diméthoxy-8-[(3S,4R)-3-hydroxy-1-méthyl-4-piperidinyl]-4H-benzopyran-4-one

On ajoute au milieu réactionnel obtenu ci-dessus en 1), 2 cm3 de HCl à 36 % (24 mmol-12eq.). On porte alors à 50°C pendant 1h40 puis on rajoute 2 cm3 de HCl à 36 % (24 mmol-12eq.). On maintient encore 2h à 50°C puis on revient à température ambiante, verse dans 60 cm3 de NaOH(N), extrait par 2x100 cm3 de CH2Cl2/MeOH : 95/5, lave par une solution saturée en NaCl, sèche sur Na2SO4 anhydre et porte à sec sous vide. On obtient 1.23 g d'une résine jaune que l'on chromatographie sur silice 0,04-0,06 mm dans CH2Cl2/MeOH : 90/10. On isole ainsi le produit attendu sous forme de mousse jaune de poids 526 mg.

### Analyses :

Spectre ¹H RMN : CDCl3 δ(ppm)
1,66(d), 3,22(masqué) : 2H ; 2,33(tl), 3,19(masqué) . 2H ; 2,52(masqué), 3,28(masqué) : 2H ; 3,57 : 1H(ax) ; 4,09(s1) : 1H(éq) ; 2,21(s), 2,24(s) : 6H ; 2,52(s) : N-CH3 ; 3,98(s), 4,00 (s) : 6H ; 6,45 (s) : 1H ; 6,55 (s) : 1H ; 5,29 (s) : 2H ; 7,16(d) . 1H ; 7,48(t) : 1H ; 7,61(s1) : 1H ; 7,78(d) : 1H.

### Stade b) : trifluoroacétate de 2-[3-[(4-bromo-3,5-diméthyl-1H-pyrazol-1-yl)méthyl]-phényl]-5,7-dihydroxy-8-[(3S,4R)-3-hydroxy-1-méthyl-4-piperidinyl)-4H-benzopyran-4-one

Dans un ballon de 20 cm3 avec réfrigérant à reflux sous N2, on introduit 150 mg du diméthoxy (0.26 mmol) obtenu au stade a) ci-dessus 2.6 cm3 de DMF anhydre et 710 mg de Reillex-pyridine -HI (≈ 4 mmol/g) (2.8 mmol-11 eq.) obtenu comme indiqué ci-dessus à la préparation 2). On porte à 130°C pendant 30h. On revient ensuite à température ambiante, dilue par 10 cm3 MeOH, amène le pH à 8 par une solution saturée de NaOH dans le MeOH, filtre la Reillex sur cartouche de filtration 20 µm + toupie 0.5 µm, porte à sec sous vide poussé. On récupère 532 mg d'un solide crème que l'on reprend par l'éluant HPLC : MeOH/H2O/TFA : 65/35/0.1. On filtre sur toupie-0.45 µm puis injecte en HPLC préparative : Kromasil C18 - 10 *µ*m - 500x22 mm - λ= 225 nm - 15 ml/min. On regroupe les fractions propres que l'on concentre sous vide puis lyophilise. On isole ainsi le produit attendu sous forme de solide jaune de poids 38 mg.

### Analyses :

Spectre ¹H RMN : DMSO D⁶ δ (ppm)
2,81(s) : N-CH3 ; 2,11(s), 2,24(s) : 6H ; 1,86, 3,18 : 2H ; 3,49, 3,20 : 2H ; 3,38 : 2H ; 4,11(sl) : 1H(ax) ; 3,52 : 1H (éq) ; 5,39 : 2H ; 6,37(s) : 1H ; 6,89(s) : 1H ; 7,31(d) : 1H ; 7,59(t) : 1H ; 7,93(sl) : 1H ; 8,03(d) : 1H ; 13,11, 11,2, 9,44, 6,0 : 4H (mobiles).

### Exemple 27 : trifluoroacétate de 5,7-dihydroxy-2-[3-[(3,5-diméthyl-1H-pyrazol-1-yl)méthyl] -phényl] -8- [(3S, 4R) -3-hydroxy-1-méthyl-4-piperidinyl]- 4H-benzopyran-4-one

On procède comme à l'exemple 26 et obtient en même temps le produit de l'exemple 27. On isole ainsi le produit attendu sous forme de solide jaune de poids 74 mg.

### Analyses :

Spectre ¹H RMN : DMSO D⁶ δ (ppm)
2,81(s) : N-CH3 ; 2,10(s), 2,21(s) : 6H ; 3,19(t1), 1,86(d1) : 2H ; 3,51(tl), 3,20 : 2H ; 3,40 : 2H ; 4,10 (masqué) : 1H(ax) ; 3,53(t1) : 1H(éq) ; 5,32 : 2H ; 6,36(s) : 1H ; 6,86(s) : 1H ; 7,27(d) : 1H ; 7,57(t) : 1H ; 7,88(s) : 1H ; 8,01(d) : 1H ; 13,11, 11,20, 9,42, 5,96 : 4H (mobiles).

### Exemple 28 : trifluoroacétate de 2-[2,5-bis(2,2,2-trifluoroéthoxy)-phényl]-5,7-dihydroxy-8-[(3S,4R)-3-hydroxy-1-méthyl-4-piperidinyl]-4H-benzopyran-4-one

### Stade a) : 2-[2,5-bis(2,2,2-trifluoroéthoxy)-phényl]-5,7-diméthoxy-8-[(3S,4R)-3-hydroxy-1-méthyl-4-piperidinyl]-4H-benzopyran-4-one

### 1) Condensation : 1-[2,5-bis(2,2,2-trifluoroéthoxy)-phényl]-3-[2-hydroxy-4,6-diméthoxy-3-((3S,4R)-3-hydroxy-1-méthyl-4-piperidinyl]phényl]-1,3-propanedione

Dans un ballon de 30 cm3, sous N2, on introduit 196 mg de NaH à 50 % dans l'huile (M=24) (4 mmol-4eq.), 5 cm3 de DMSO/NK20 et 309 mg d'Acétoflocinopipéridol (1 mmol). On agite 1h à température ambiante, puis introduit 996 mg de 2,5-bis(2,2,2-trifluoroéthoxy)-benzoate de méthyle (M=332.2) (3 mmol 3eq.). On maintient sous agitation pendant 20h.

### 2) Cyclisation : 2-[2,5-bis(2,2,2-trifluoroéthoxy)-phényl]-5,7-diméthoxy-8-[(3S,4R)-3-hydroxy-1-méthyl-4-piperidinyl]-4H-benzopyran-4-one

On ajoute au milieu réactionnel obtenu ci-dessus en 1), 2.5 cm3 de HCl à 36 % (24 mmol-24eq.). On porte alors à 50°C pendant 3h30. On revient à température ambiante, ajoute 3 cm3 de NaOH conc, extrait par 3 fois 20 cm3 de CH2Cl2/MeOH : 9/1, lave à l'eau, sèche sur MgSO4 anhydre et porte à sec sous vide. On obtient 1 g d'une huile marron que l'on chromatographie sur silice 0,04-0,06 mm dans CH2Cl2/MeOH : 80/20. On isole ainsi le produit attendu sous forme de solide jaune de poids 146 mg.

### Analyses :

Spectre ¹H RMN : CDCl3 δ(ppm)
1,61 (m), 3,26 (m) : 2H ; 2,23 (m), 3,14(m) : 2H ; 2,42 (s1) : N-CH3 ; 2,44(masqué), 3,19 (m) : 2H ; 3,41(1) : 1H (OH) ; 3,51 (ddd) : 1H (ax) ; 4,03(s1) : 1H(éq) ; 3,97 (s) : 6H ; 4,39 (qd), 4,44(qd), 4,67 (m) : 4H ; 6,43 (s) : 1H ; 6,75 (s) : 1H ; 6,99 (d) : 1H ; 7,07 (dd) : 1H ; 7,64 (d1) : 1H.

### Stade b) : trifluoroacétate de 2-[2,5-bis(2,2,2-trifluoroéthoxy)-phényl]-5,7-dihydroxy-8-[(3S,4R)-3-hydroxy-1-méthyl-4-piperidinyl]-4H-benzopyran-4-one

Dans un ballon de 30 cm3 avec réfrigérant à reflux, sous N2, on introduit 130 mg du diméthoxy obtenu ci-dessus en a) (0.25 mmol), 15 cm3 de DMF anhydre et 682 mg de Reillex-pyridine -HI Pyridine (≈ 4 mmol/g) (2.72 mmol-11 eq.) obtenu comme indiqué ci-dessus à la préparation 2). On porte à 150°C pendant 36h. On revient ensuite à température ambiante, dilue par CH3CN, filtre sur Iéna, rince par CH3CN puis amène à sec sous vide. On obtient un produit brut de poids : 650 mg. Le produit brut obtenu est remis en solution dans 10 cm3 de CH3CN, 10 cm3 de MeOH et 1.9 g de résine PTBD (2.6 mmol/g) (5 mmol-20eq.). On agite 20h à température ambiante, filtre sur Iéna, lave par CH3CN puis décroche par 12 cm3 de CH3CN/TFA : 95/05 sous agitation. On filtre sur Iéna, rince par CH3CN/TFA : 95/05 puis porte à sec sous vide. On récupère 150 mg d'un produit amorphe orange que l'on reprend par 7.5 cm3 d'éluant HPLC CH3CN/H2O/TFA : 42/58/0.1. On filtre sur toupie-0.45 µm puis injecte en 5 fois en HPLC préparative : Kromasil C18 - 10 µm - 500x22 mm - λ= 225 nm - 15 ml/min. On regroupe les fractions propres que l'on concentre sous vide puis lyophilise. On isole ainsi le produit attendu sous forme de solide jaune de poids 45 mg.

### Analyses :

Spectre ¹H RMN : DMSO D⁶ δ (ppm)
1, 91 (d1), 3,07(m) : 2H ; 3,12(masqué), 3,36(m) : 2H ; 3, 24 (m), 3,32 (masqué) : 2H ; 2,74(s1) : N-CH3 ; 4, 12 (s1), 4,04 (sl) : 1H (éq) ; 3,47(masqué) : 1H (ax) ; 4,89 (q) : 4H ; 6,10(sl), 5,83 (sl) : 1H (OH) ; 6,36(s) : 1H ; 6,65(s) : 1H ; 7,37(masqué), 7,46(d) : 3H ; 9,32, 11,22(sl), 13,05(1), 13,14(s) : 4H (mobiles).

### Exemple 29 : 5,7-dihydroxy-8-[(3S,4R)-3-hydroxy-1-méthyl-4-piperidinyl]-2-(3-isoquinolinyl)-4H-benzopyran-4-one

### Stade a) : 5,7-diméthoxy-8-[(3S,4R)-3-hydroxy-1-méthyl-4-piperidinyl]-2-(3-isoquinolinyl)-4H-benzopyran-4-one

### 1) Condensation : 1-[2-hydroxy-4,6-diméthoxy-3-[(3S,4R)-3-hydroxy-1-méthyl-4-piperidinyl]phényl]-3-(3-isoquinolinyl) 1,3-propanedione

Dans un tube de 30 cm3, sous Ar, on introduit 124 mg de NaH à 50 % dans l'huile (M=24) (2.6 mmol-4eq.), 6.5 cm3 de DMSO/NK20 et 200 mg d'Acétoflocinopipéridol (0.65 mmol) par fractions. On agite 1h à température ambiante, puis introduit 363 mg de 3-isoquinolinecarboxylate de méthyle (M=187.2) (11.9 mmol-3eq.) et maintient sous agitation pendant 18h.

### 2) Cyclisation : 5,7-diméthoxy-8-[(3S,4R)-3-hydroxy-1-méthyl-4-piperidinyl]-2-(3-isoquinolinyl)-4H-benzopyran-4-one

On ajoute au milieu réactionnel obtenu ci-dessus en 1), 4.4 cm3 de HCl à 36 % (51 mmol-79eq.) et porte alors à 50°C pendant 3h30. On revient à température ambiante, verse dans 60 cm3 de NaOH(N), extrait par 2x50 cm3 de CH2Cl2/MeOH : 9/1, lave par 50 cm3 H2O, sèche sur Na2SO4 anhydre et porte à sec sous vide. On obtient 533 mg d'un solide jaune pâle que l'on chromatographie sur silice 0,04-0,06 mm dans CH2Cl2/MeOH : 90/10. On isole ainsi le produit attendu sous forme de solide jaune pâle de poids 294 mg.

### Analyses :

Spectre IR : absorption région OH/NH
-C=O : 1642 cm-1 ; Système conjugué + aromatiques : 1623, 1615, 1596, 1575, 1563, 1492 cm-1.
Spectre ¹H RMN : DMSO D⁶ δ (ppm)
1,90, 3,43 : 2H ; 2,83(sl) : N-CH3 ; 3,42 : 2H ; 3,44 : 2H ; 3,71(dl) : 1H(ax) ; 4,16 (sl) : 1H (éq) ; 3,92(s), 3,96 (s) : 6H ; 5,47(1) : 1H (OH) ; 6,70(s) : 1H ; 7,07(s) : 1H ; 7,84(ddd) : 1H ; 7,94(ddd) : 1H ; 8,26(d) : 1H ; 8,35 (dl) : 1H ; 8,54(s) : 1H ; 9,47(s) : 1H.

### Stade b) : 5,7-dihydroxy-8-[(3S,4R)-3-hydroxy-1-méthyl-4-piperidinyl]-2-(3-isoquinolinyl)-4H-benzopyran-4-one

Dans un ballon avec réfrigérant à reflux, sous Ar, on introduit 168 mg du diméthoxy obtenu ci-dessus en a) (0.38 mmol), 4 cm3 de DMF anhydre et 470 mg de Reillex-pyridine -HI (≈ 4 mmol/g) (1.9 mmol-5 eq.) obtenu comme indiqué ci-dessus à la préparation 2). On porte à 150°C pendant 16h30. On revient ensuite à température ambiante, filtre sur toupie-0.45 µm puis amène à pH=8-9 par 0.5 cm3 de NaOH(N), dilue par 20 cm3 H2O, agite 1h à température ambiante, filtre sur Iéna, lave par 10 cm3 H2O. On redisperse le solide dans H2O puis on dilue le milieu par 15 cm3 de DMF. On porte au reflux puis laisse revenir à température ambiante avant de placer le milieu au réfrigérateur 4 jours pour cristallisation. Après retour à température ambiante, on filtre sur Iéna, lave à l'eau et sèche sous vide à 60°C. On isole ainsi le produit attendu sous forme de solide jaune de poids 56 mg.

### Analyses :

Spectre ¹H RMN : DMSO D⁶ δ (ppm)
1,98, 3,32 : 2H ; 2,89 (d) : N-CH3 ; 3,47 : 2H ; 3,38, 3,58 : 2H ; 3,67 (dl) : 1H(ax) ; 4,22 (s1) : 1H(éq) ; 6,38 (s) : 1H ; 7,26 (s) : 1H ; 7,88 (t1) : 1H ; 7, 98 (t1) : 1H ; 8,28(d1) : 1H ; 8,37 (d1) : 1H ; 8,60 (s) : 1H ; 9,50 (s) : 1H ; 11,23, 13,11 : 3H ; 9,48 (d) : 1H.

### Exemple 30 : trifluoroacétate de 2-(2-chlorophényl)-5,7-dihydroxy-6-[(3S,4R)-3-hydroxy-1-méthyl-4-piperidinyl]-4H-benzopyran-4-one

Dans un ballon de 10 cm3 avec réfrigérant à reflux, sous N2, on introduit 466 mg de chlorhydrate de 2-(2-chlorophényl)-5,7-diméthoxy-8-[(3S,4R)-3-hydroxy-1-méthyl-4-piperidinyl]-4H-benzopyran-4-one préparé comme indiqué dans la demande de brevet numéro FR 9807677 (10 mmol), 1.1 cm3 de HBr à 48 % (d=1.49) (10 mmol-leq.), 2.4 cm3 d'H2O et porte cette suspension à 140°C pendant 21h et à 160°C pendant 2h30. On rajoute ensuite 1.2 cm3 de HBr à 48 % (10 eq.) et chauffe à 160°C encore 31 h. On revient ensuite à température ambiante, dilue par H2O, redissout le précipité par CH3CN, puis ajoute 7.7 g de résine PTBD (2.6 mmol/g) (20 mmol-20eq.). On agite 2h30 à température ambiante, puis rajoute 2 g de résine PTBD (2.6 mmol/g) (5.2 mmol-5eq.). On agite encore 1h30 à température ambiante, filtre sur Iéna, lave par CH3CN puis décroche par 40 cm3 de CH3CN/TFA : 95/05 sous agitation. On filtre sur Iéna, rince par CH3CN/TFA : 95/05 puis porte à sec sous vide. On récupère 535 mg d'une résine jaune que l'on reprend par 10 cm3 d'éluant HPLC CH3CN/H2O/ TFA : 35/65/0.1. On filtre sur toupie-0.45 µm puis injecte en 10 fois en HPLC préparative Kromasil C18 - 10 µm - 500x22 mm - λ= 225 nm - 20 ml/min. On regroupe les fractions propres que l'on concentre sous vide puis lyophilise. On isole ainsi le produit attendu sous forme de solide jaune de poids 68.3 mg.

### Analyses :

Spectre ¹H RMN : DMSO D⁶ δ (ppm)
2,76(s) : N-CH3 ; 1,73, 3,12 : 2H ; 3,10, 3,40 : 2H ; 3,32 : 2H ; 3,40 : 1H ; 4,11(sl) : 1H(éq) ; 6,51(s) : 1H ; 6,59(s) : 1H ; 7,52(td), 7,61 (td) : 2H ; 7,67(dd), 7,77(dd) : 2H ; 13,46 : 1H (OH) ; 11,37 : 1H (OH) ; 6,10, 9,40 : 2H (mobiles).

### Exemple 31 : trifluoroacétate de 2-(2-chlorophényl)-5,7-dihydroxy-8-[(3S,4R)-3-hydroxy-1-méthyl-4-piperidinyl]-4H-benzopyran-4-one

### Exemple de déprotection de fonctions hydroxyle en utilisant Reillex-HI-Pyridine dans DMF

Dans un ballon de 30 cm3 avec réfrigérant à reflux, sous N2, on introduit 100 mg de chlorhydrate de 2-(2-chlorophényl)-5,7-diméthoxy-8-[(3S,4R)-3-hydroxy-1-méthyl-4-piperidinyl]-4H-benzopyran-4-one préparé comme indiqué dans la demande de brevet numéro FR 9807677 (0.21 mmol), 2 cm3 de DMF anhydre et 581 mg de Reillex-pyridine -HI Pyridine (≈ 4 mmol/g) (2.2 mmol-11 eq.) obtenu comme indiqué ci-dessus à la préparation 2). On porte à 130°C pendant 48 h et à 150°C pendant 1 h. On revient ensuite à température ambiante, dilue par CH3CN, filtre sur Iéna, rince par CH3CN puis ajoute 1.5 g de résine PTBD (2.6 mmol/g) (4.2 mmol-20eq.). On agite 20h à température ambiante, filtre sur Iéna, lave par CH3CN puis décroche par 10 cm3 de CH3CN/TFA : 95/05 sous agitation pendant 1 h. On filtre sur Iéna, rince par CH3CN/TFA : 95/05 puis porte à sec sous vide. On récupère 130 mg d'un produit amorphe orange que l'on reprend par 3 cm3 d'éluant HPLC : CH3CN/H2O/TFA : 35/65/0.1. On filtre sur toupie-0.45 µm puis injecte en 4 fois en HPLC préparative : Kromasil C18 - 10 µm - 500x22 mm - λ= 225 nm - 15 ml/min. On regroupe les fractions propres que l'on concentre sous vide puis lyophilise. On isole ainsi le produit attendu sous forme de solide jaune de poids 50 mg.

### Analyses :

SM/ESP : MH⁺=402⁺

### Exemple 32 : trifluoroacétate de 8-[(3S,4R)-3-acétyloxy-1-méthyl-4-piperidinyl] -2-(chlorophényl) 5,7-dihydroxy-4H-benzopyran-4-one

### Exemple de déprotection de fonctions hydroxyle en utilisant HI-Pyridine dans DMA.

Dans un ballon de 30 cm3 avec réfrigérant à reflux, sous N2, on introduit 115 mg (0.24 mmol) de chlorhydrate de 2-(2-chlorophényl)-5,7-diméthoxy-8-[(3S, 4R)-3-hydroxy-1-méthyl-4-piperidinyl]-4H-benzopyran-4-one préparé comme indiqué dans la demande de brevet numéro FR 9807677, 2.6 cm3 de DMA anhydre et 553 mg de HI-Pyridine (M=207.1) (2.64 mmol-11eq.) obtenu comme indiqué à la préparation 1) ci-dessus. On porte à 130°C pendant 20h. On revient ensuite à température ambiante, dilue par CH3CN, puis ajoute 1.9 g de résine PTBD (2.6 mmol/g) (4,9 mmol-20eq.). On agite 20h à température ambiante, filtre sur Iéna, lave par CH3CN puis décroche par 10 cm3 de CH3CN/TFA : 95/05 sous agitation. On filtre sur Iéna, rince par CH3CN/TFA : 95/05 puis porte à sec sous vide.

On récupère 330 mg d'un produit amorphe orange que l'on reprend par 6 cm3 d'éluant HPLC : CH3CN/H2O/TFA : 35/65/0.1.

On filtre sur toupie-0.45 µm puis injecte en 6 fois en HPLC préparative : Kromasil C18 - 10 µm - 500x22 mm - λ= 225 nm - 15 ml/min. On regroupe les fractions propres que l'on concentre sous vide puis lyophilise. On isole ainsi le produit attendu sous forme de solide jaune de poids 90 mg.

### Analyses :

SM/ESP : MH⁺=444⁺

### Exemple 33 : COMPOSITION PHARMACEUTIQUE :

On a préparé des comprimés répondant à la formule suivante :

| | |
|---|---|
| Produit de l'exemple 2 | 0,2 g |
| Excipient pour un comprimé terminé à | 1 g |

(détail de l'excipient : lactose, talc, amidon,
stéarate de magnésium).

## Revendications

1. Produits de formule (I) : 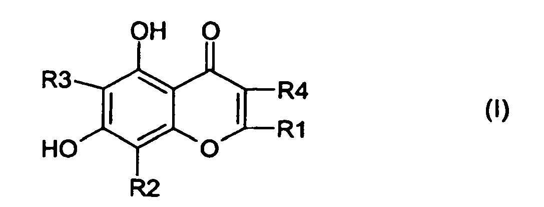 dans laquelle :
R2 et R3 sont tels que l'un représente un atome d'hydrogène et l'autre représente un radical pipéridinyle éventuellement substitué par un ou plusieurs radicaux hydroxyle et alkyle, R2 et R3 pouvant prendre alternativement les mêmes valeurs pour donner les isomères correspondants,
R4 représente un atome d'hydrogène, un radical alkyle ou phényle éventuellement substitués par un ou plusieurs atomes d'halogène,
R1 représente un radical phényle, éventuellement substitué par un ou plusieurs radicaux choisis parmi les atomes d'halogène ; le radical cyclohexyle ; cyano ; nitro ; hydroxyle ; carboxy libre, salifié ou estérifié ; tétrazolyle ; -NH2, -NH(alkyl), -N(alkyl)(alkyl) ; SO2-NH-CO-NHR5 dans lequel R5 représente un radical alkyle ou phényle ; phényle ; alkyle, alcoxy ou phénoxy ; CF3 ; OCF3 ; pyrazolinyle lui-même éventuellement substitué par un ou plusieurs radicaux choisis parmi les radicaux alkyle et les atomes d'halogène, étant entendu que dans les radicaux ci-dessus, les radicaux alkyle et alcoxy sont linéaires ou ramifiés et renferment au plus 4 atomes de carbone,
**caractérisés en ce que** ces produits de formule (I) sont les suivants :
- le trifluoroacétate de 2-(2-chloro-4-fluorophényl)-5,7-dihydroxy-8-[(3S,4R)-3-hydroxy-1-méthyl-4-pipéridinyl]- 4H-benzopyran-4-one
- le trifluoroacétate de 2-(4-cyclohexylphényl)-5,7-dihydroxy-8-[(3S,4R)-3-hydroxy-1-méthyl-4-pipéridinyl]-4H-benzopyran-4-one
- le trifluoroacétate de 4-[5,7-dihydroxy-8-[(3S,4R)-3-hydroxy-1-méthyl-4-pipéridinyl]-4-oxo-4H-benzopyran-2-yl]-benzonitrile
- le trifluoroacétate de 5,7-dihydroxy-8-[(3S,4R)-3-hydroxy-1-méthyl-4-pipéridinyl]-2-[4-(1H-tetrazol-5-yl)-phényl]-4H-benzopyran-4-one
- le trifluoroacétate de 5,7-dihydroxy-8-[(3S,4R)-3-hydroxy-1-méthyl-4-pipéridinyl]-2-[3-(phénoxy)-phényl]-4H-benzopyran-4-one
- le trifluoroacétate de 5,7-dihydroxy-6-[(3S,4R)-3-hydroxy-1-méthyl-4-pipéridinyl]-2-[3-(phénoxy)-phényl]-4H-benzopyran-4-one
- le 5,7-dihydroxy-8-[(3S,4R)-3-hydroxy-1-méthyl-4-pipéridinyl]-2-(3-nitrophényl)-4H-benzopyran-4-one
- le trifluoroacétate de 5,7-dihydroxy-2-[4-fluoro-3-(trifluorométhyl)phényl]-8-[(3S,4R)-3-hydroxy-1-méthyl-4-pipéridinyl]- 4H-benzopyran-4-one
- le trifluoroacétate de l'acide 4-[8-[(3S,4R)-3-acétyloxy-1-méthyl-4-pipéridinyl]- 5,7-dihydroxy-4-oxo-4H-benzopyran-2-yl]-2,5-dichloro-benzoique
- le chlorhydrate de 2,5-dichloro-4-[5,7-dihydroxy-8-[(3S,4R)-3-hydroxy-1-méthyl-4-pipéridinyl]-4-oxo-4H-benzopyran-2-yl]-benzoate de méthyle
- le trifluoroacétate de 2-[4-(diéthylamino)-phényl]-5,7-dihydroxy-8-[(3S,4R)-3-hydroxy-1-méthyl-4-pipéridinyl]- 4H-benzopyran-4-one
- le trifluoroacétate de 5,7-dihydroxy-8-[(3S,4R)-3-hydroxy-1-méthyl-4-pipéridinyl]-2-[3-(trifluorométhoxy)-phényl)-4H-benzopyran-4-one
- le trifluoroacétate de 2-[3,5-bis(trifluorométhyl)-phényl]-5,7-dihydroxy-8-[(3S,4R)-3-hydroxy-1-méthyl-4-pipéridinyl]- 4H-benzopyran-4-one
- le trifluoroacétate de 5,7-dihydroxy-8-[(3S,4R)-3-hydroxy-1-méthyl-4-pipéridinyl]-2-[4-(trifluorométhoxy)-phényl]-4H-benzopyran-4-one
- le trifluoroacétate de l'acide 4-[5,7-dihydroxy-8-[(3S,4R)-3-hydroxy-1-méthyl-4-pipéridinyl]-4-oxo-4H-benzopyran-2-yl]-benzoique
- le trifluoroacétate de 2-(3-[(4-bromo-3,5-diméthyl-1H-pyrazol-1-yl)méthyl]-phényl]-5,7-dihydroxy-8-[(3S,4R)-3-hydroxy-1-méthyl-4-pipéridinyl]-4H-benzopyran-4-one
- le trifluoroacétate de 5,7-dihydroxy-2-[3-[(3,5-diméthyl-1H-pyrazol-1-yl)méthyl]-phényl]-8-[(3S,4R)-3-hydroxy-1-méthyl-4-pipéridinyl]- 4H-benzopyran-4-one
- le trifluoroacétate de 2-[2,5-bis(2,2,2-trifluoroéthoxy)-phényl]-5,7-dihydroxy-8-[(3S,4R)-3-hydroxy-1-méthyl-4-pipéridinyl]-4H-benzopyran-4-one
- le trifluoroacétate de 2-(2-chlorophényl)-5,7-dihydroxy-6-[(3S,4R)-3-hydroxy-1-méthyl-4-pipéridinyl]-4H-benzopyran-4-one
lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).

2. Procédé de préparation des produits de formule (I), telle que définie à la revendication 1, **caractérisé en ce que** l'on soumet le composé de formule (II) : 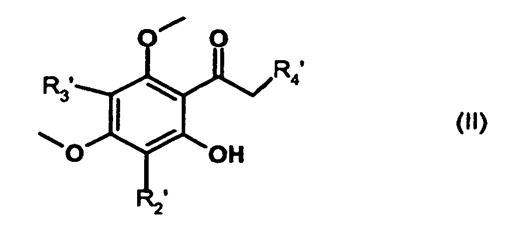 dans laquelle R2', R3' et R4' ont les significations indiquées à la revendication 1 respectivement pour R2, R3 et R4, dans lesquelles les éventuelles fonctions réactives sont éventuellement protégées par des groupements protecteurs à une réaction avec un composé de formule (III) :
R1'COX (III)
dans laquelle R1' a la signification indiquée à la revendication 1 pour R1, dans laquelle les éventuelles fonctions réactives sont éventuellement protégées par des groupements protecteurs et X représente un atome d'halogène ou un radical alcoxy renfermant au plus 6 atomes de carbone, pour obtenir le produit de formule (IV) : 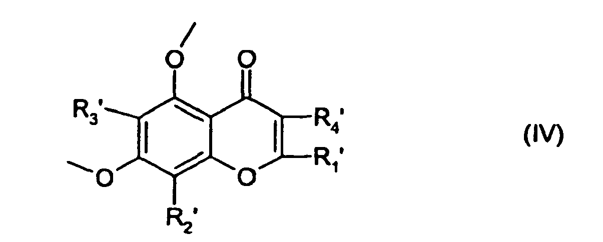 dans laquelle R1', R2', R3' et R4' ont les significations indiquées ci-dessus,
produit de formule (IV) que l'on soumet à une réaction de déprotection des radicaux hydroxyle
pour obtenir un produit de formule (I') : 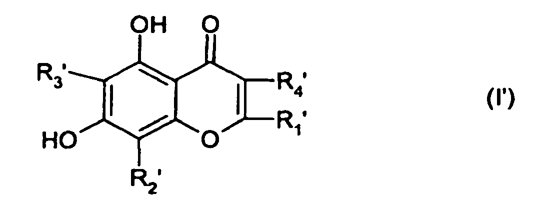 dans laquelle R1', R2', R3' et R4' ont les significations indiquées ci-dessus,
produits de formule (I') qui peuvent être des produits de formule (I) et que, pour obtenir des ou d'autres produits de formule (I), l'on peut soumettre, si désiré et si nécessaire, à l'une ou plusieurs des réactions de transformations suivantes, dans un ordre quelconque :
a) une réaction d'estérification de fonction acide,
b) une réaction de saponification de fonction ester en fonction acide,
c) une réaction d'oxydation de groupement alkylthio en sulfoxyde ou sulfone correspondant,
d) une réaction de transformation de fonction cétone en fonction oxime,
e) une réaction de réduction de la fonction carboxy libre ou estérifié en fonction alcool,
f) une réaction de transformation de fonction alcoxy en fonction hydroxyle, ou encore de fonction hydroxyle en fonction alcoxy,
g) une réaction d'oxydation de fonction alcool en fonction aldéhyde, acide ou cétone,
h) une réaction de transformation de radical nitrile en tétrazolyle,
i) une réaction d'élimination des groupements protecteurs que peuvent porter les fonctions réactives protégées,
j) une réaction de salification par un acide minéral ou organique ou par une base pour obtenir le sel correspondant,
k) une réaction de dédoublement des formes racémiques en produits dédoublés,
lesdits produits de formule (I) ainsi obtenus étant sous toutes les formes isomères possibles racémiques, énantiomères et diastéréoisomères.

3. Procédé de déprotection des fonctions hydroxyle d'un produit de formule (IV) telle que définie à la revendication 2 : 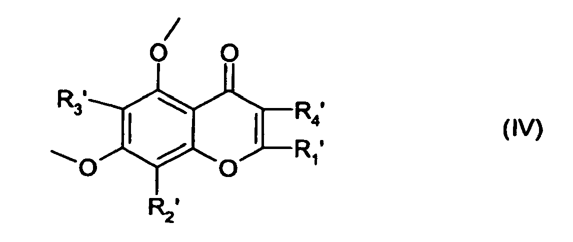 dans laquelle R1', R2', R3' et R4' ont les significations indiquées à la revendication 2,
**caractérisé en ce que** l'on soumet le produit de formule (IV) soit à l'action de l'acide iodhydrique supporté par une résine (telle que par exemple le Reillex-pyridine-TM-402 polymer) dans un solvant polaire anhydre,
soit à l'action d'un sel de l'acide iodhydrique tel que HI-Py dans un solvant polaire anhydre,
soit à l'action de l'acide iodhydrique concentré, pour obtenir un produit de formule (I') : 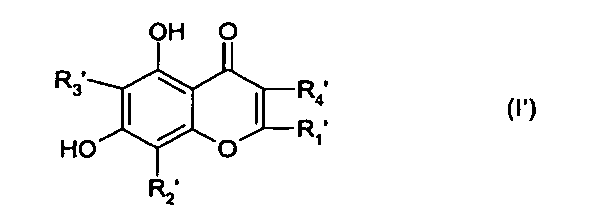 dans laquelle R1', R2', R3' et R4' ont les significations indiquées à la revendication 2,
produits de formule (I') qui peuvent être des produits de formule (I) et que, pour obtenir des ou d'autres produits de formule (I), l'on peut soumettre, si désiré et si nécessaire, à l'une ou plusieurs des réactions de transformations suivantes, dans un ordre quelconque :
a) une réaction d'estérification de fonction acide,
b) une réaction de saponification de fonction ester en fonction acide,
c) une réaction d'oxydation de groupement alkylthio en sulfoxyde ou sulfone correspondant, correspondant,
d) une réaction de transformation de fonction cétone en fonction oxime,
e) une réaction de réduction de la fonction carboxy libre ou estérifié en fonction alcool,
f) une réaction de transformation de fonction alcoxy en fonction hydroxyle, ou encore de fonction hydroxyle en fonction alcoxy,
g) une réaction d'oxydation de fonction alcool en fonction aldéhyde, acide ou cétone,
h) une réaction de transformation de radical nitrile en tétrazolyle,
i) une réaction d'élimination des groupements protecteurs que peuvent porter les fonctions réactives protégées,
j) une réaction de salification par un acide minéral ou organique ou par une base pour obtenir le sel correspondant,
k) une réaction de dédoublement des formes racémiques en produits dédoublés,
lesdits produits de formule (I) ainsi obtenus étant sous toutes les formes isomères possibles racémiques, énantiomères et diastéréoisomères.

4. A titre de médicaments, les produits de formule (I) telle que définie à la revendication 1, ainsi que les sels d'addition avec les acides minéraux et organiques ou avec les bases minérales et organiques pharmaceutiquement acceptables desdits produits de formule (I).

5. Les compositions pharmaceutiques contenant à titre de principe actif, l'un au moins des médicaments tels que définis à la revendication 4.

6. Compositions pharmaceutiques selon la revendication 5 **caractérisées en ce qu'**elles sont utilisées comme médicaments antimitotiques, en particulier pour la chimiothérapie de cancers ou encore pour le traitement de psoriasis, de parasitoses telles que celles dues à des champignons ou à des protistes ou de la maladie d'Alzheimer.

7. Compositions pharmaceutiques selon la revendication 5 **caractérisées en ce qu'**elles sont utilisées comme médicaments antineurodégénératifs notamment anti-apoptose neuronale.

8. Utilisation des produits de formule (I) telle que définie à la revendication 1 pour la préparation de médicaments destinés à la prévention ou au traitement de maladies liées à un dérèglement de la sécrétion et/ou de l'activité de protéines tyrosines kinases.

9. Utilisation des produits de formule (I) telle que définie à la revendication 1, pour la préparation de médicaments destinés à la chimiothérapie de cancers, au traitement de psoriasis, de parasitoses telles que celles dues à des champignons ou à des protistes, au traitement de la maladie d'Alzheimer ou au traitement d'affections neurodégénératives notamment l'apoptose neuronale.

10. A titre de produits industriels nouveaux, les composés de formule (IV) : 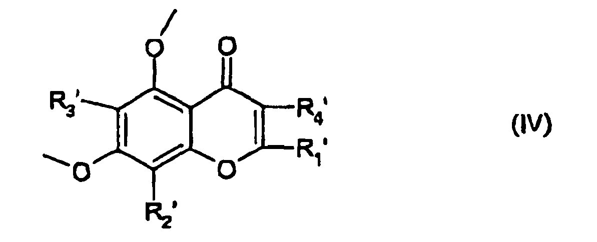 dans laquelle R1', R2', R3' et R4' ont les significations indiquées à la revendication 1 respectivement pour R1, R2, R3 et R4, dans lesquelles les éventuelles fonctions réactives sont éventuellement protégées par des groupements protecteurs ainsi qu'il est défini aux revendications 2 et 3.
Procédé de préparation des produits de formule (I) :

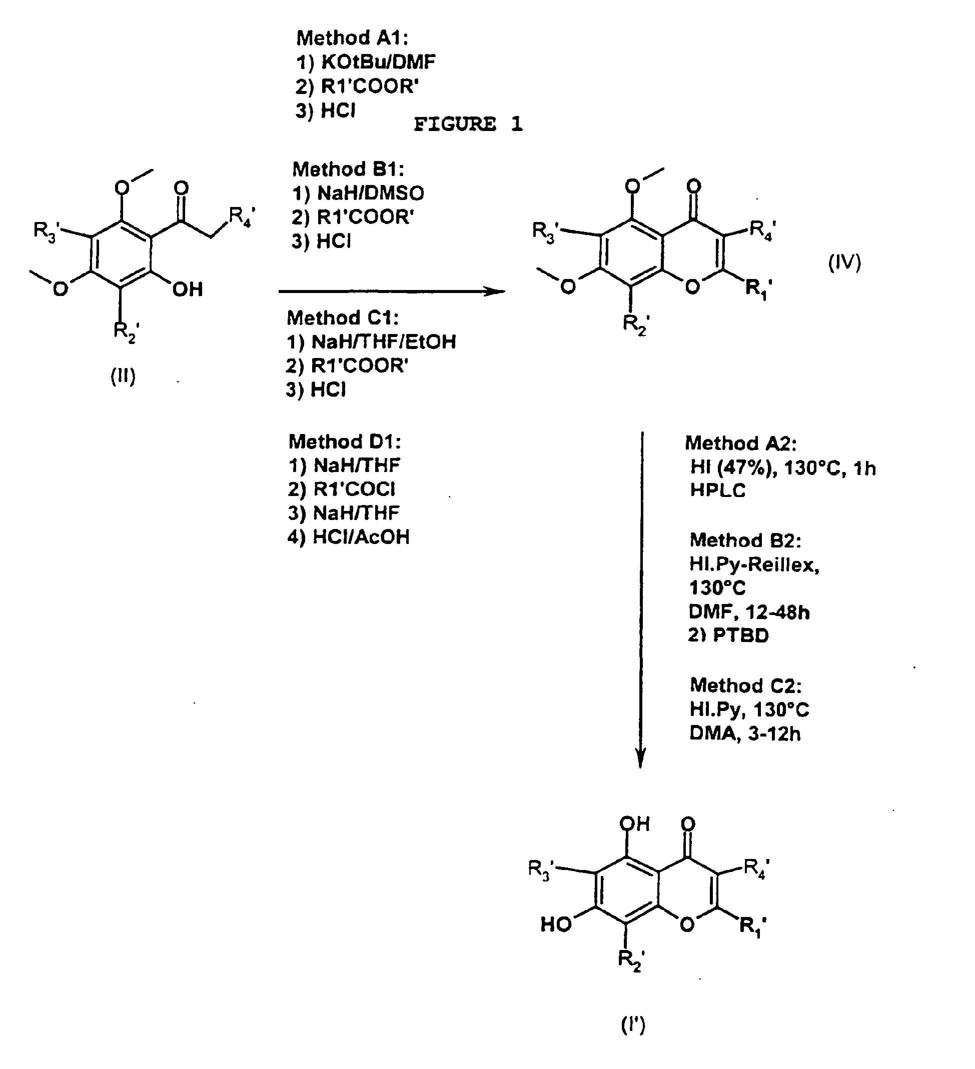

## Claims

1. Products of formula (I): 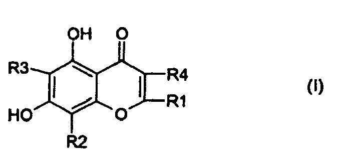 in which:
R2 and R3 are such that one represents a hydrogen atom and the other represents a piperidyl radical optionally substituted with one or more hydroxyl and alkyl radicals, R2 and R3 possibly alternatively taking the same values to give the corresponding isomers,
R4 represents a hydrogen atom or an alkyl or phenyl radical optionally substituted with one or more halogen atoms,
R1 represents a phenyl radical, optionally substituted with one or more radicals chosen from halogen atoms; cyclohexyl; cyano; nitro; hydroxyl; free, salified or esterified carboxyl; tetrazolyl; -NH2, -NH(alkyl), -N(alkyl)(alkyl); SO2-NH-CO-NHR5 in which R5 represents an alkyl or phenyl radical; phenyl; alkyl, alkoxy or phenoxy; CF3; OCF3; pyrazolinyl, itself optionally substituted with one or more radicals chosen from alkyl radicals and halogen atoms, it being understood that in the above radicals, the alkyl and alkoxy radicals are linear or branched and contain up to 4 carbon atoms,
**characterized in that** these products of formula (I) are the following:
- 2-(2-chloro-4-fluorophenyl)-5,7-dihydroxy-8-[(3S,4R)-3-hydroxy-1-methyl-4-piperidyl]-4H-benzopyran-4-one trifluoroacetate
- 2-(4-cyclohexylphenyl)-5,7-dihydroxy-8-[(3S,4R)-3-hydroxy-1-methyl-4-piperidyl]-4H-benzopyran-4-one trifluoroacetate
- 4-[5,7-dihydroxy-8-[(3S,4R)-3-hydroxy-1-methyl-4-piperidyl]-4-oxo-4H-benzopyran-2-yl]benzonitrile trifluoroacetate
- 5,7-dihydroxy-8-[(3S,4R)-3-hydroxy-1-methyl-4-piperidyl]-2-[4-(1H-tetrazol-5-yl)phenyl]-4H-benzopyran-4-one trifluoroacetate
- 5,7-dihydroxy-8-[(3S,4R)-3-hydroxy-1-methyl-4-piperidyl]-2-[3-(phenoxy)phenyl]-4H-benzopyran-4-one trifluoroacetate
- 5,7-dihydroxy-6-[(3S,4R)-3-hydroxy-1-methyl-4-piperidyl]-2-[3-(phenoxy)phenyl]-4H-benzopyran-4-one trifluoroacetate
- 5,7-dihydroxy-8-[(3S,4R)-3-hydroxy-1-methyl-4-piperidyl]-2-(3-nitrophenyl)-4H-benzopyran-4-one trifluoroacetate
- 5,7-dihydroxy-2-[4-fluoro-3-(trifluoromethyl)phenyl]-8-[(3S,4R)-3-hydroxy-1-methyl-4-piperidyl]-4H-benzopyran-4-one trifluoroacetate
- 4-[8-[(3S,4R)-3-acetyloxy-1-methyl-4-piperidyl]-5,7-dihydroxy-4-oxo-4H-benzopyran-2-yl]-2,5-dichlorobenzoic acid trifluoroacetate
- methyl 2,5-dichloro-4-[5,7-dihydroxy-8-[(3S,4R)-3-hydroxy-1-methyl-4-piperidyl]-4-oxo-4H-benzopyran-2-yl]benzoate hydrochloride
- 2-[4-(diethylamino)phenyl]-5,7-dihydroxy-8-[(3S,4R)-3-hydroxy-1-methyl-4-piperidyl]-4H-benzopyran-4-one trifluoroacetate
- 5,7-dihydroxy-8-[(3S,4R)-3-hydroxy-1-methyl-4-piperidyl]-2-[3-(trifluoromethoxy)phenyl]-4H-benzopyran-4-one trifluoroacetate
- 2-[3,5-bis(trifluoromethyl)phenyl]-5,7-dihydroxy-8-[(3S,4R)-3-hydroxy-1-methyl-4-piperidyl]-4H-benzopyran-4-one trifluoroacetate
- 5,7-dihydroxy-8-[(3S,4R)-3-hydroxy-1-methyl-4-piperidyl]-2-[4-(trifluoromethoxy)phenyl]-4H-benzopyran-4-one trifluoroacetate
- 4-[5,7-dihydroxy-8-[(3S,4R)-3-hydroxy-1-methyl-4-piperidyl]-4-oxo-4H-benzopyran-2-yl]benzoic acid trifluoroacetate
- 2-[3-[(4-bromo-3,5-dimethyl-1H-pyrazol-1-yl)methyl]phenyl]-5,7-dihydroxy-8-[(3S,4R)-3-hydroxy-1-methyl-4-piperidyl]-4H-benzopyran-4-one trifluoroacetate
- 5,7-dihydroxy-2-[3-[(3,5-dimethyl-1H-pyrazol-1-yl)methyl]phenyl]-8-[(3S,4R)-3-hydroxy-1-methyl-4-piperidyl]-4H-benzopyran-4-one trifluoroacetate
- 2-[2,5-bis(2,2,2-trifluoroethoxy)phenyl]-5,7-dihydroxy-8-[(3S,4R)-3-hydroxy-1-methyl-4-piperidyl]-4H-benzopyran-4-one trifluoroacetate
- 2-(2-chlorophenyl)-5,7-dihydroxy-6-[(3S,4R)-3-hydroxy-1-methyl-4-piperidyl]-4H-benzopyran-4-one trifluoroacetate
the said products of formula (I) being in any of the possible racemic, enantiomeric and diastereoisomeric isomer forms, and also the addition salts with mineral and organic acids or with mineral and organic bases of the said products of formula (I).

2. Process for preparing the products of formula (I), as defined in Claim 1, **characterized in that** the compound of formula (II): 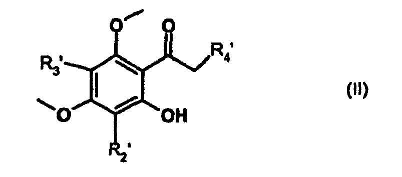 in which R2', R3' and R4' have the meanings given in Claim 1 for R2, R3 and R4, respectively, in which the optional reactive functions are optionally protected with protecting groups, is subjected to a reaction with a compound of formula (III):
R1'COX (III)
in which R1' has the meaning given in Claim 1 for R1, in which the optional reactive functions are optionally protected with protecting groups and X represents a halogen atom or an alkoxy radical containing up to 6 carbon atoms,
to obtain the product of formula (IV): 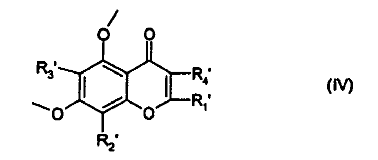 in which R1' , R2', R3' and R4' have the meanings given above,
which product of formula (IV) is subjected to a reaction for deprotection of the hydroxyl radicals, to obtain a product of formula (I'): 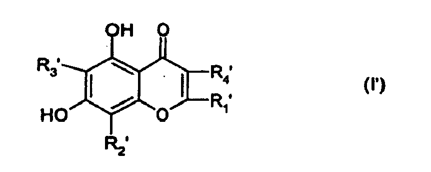 in which R1' , R2', R3' and R4' have the meanings given
above,
which products of formula (I') may be products of formula (I) and which, in order to obtain products or other products of formula (I), may be subjected, if desired and if necessary, to one or more of the following conversion reactions, in any order:
a) a reaction for esterification of the acid function,
b) a reaction for saponification of the ester function to an acid function,
c) a reaction for oxidation of the alkylthio group to a corresponding sulfoxide or sulfone group,
d) a reaction for conversion of the ketone function to an oxime function,
e) a reaction for reduction of the free or esterified carboxyl function to an alcohol function,
f) a reaction for conversion of the alkoxy function to a hydroxyl function, or of the hydroxyl function to an alkoxy function,
g) a reaction for oxidation of the alcohol function to an aldehyde, acid or ketone function,
h) a reaction for conversion of the nitrile radical to tetrazolyl,
i) a reaction for removal of the protecting groups possibly borne by the protected reactive functions,
j) a reaction for salification with a mineral or organic acid or with a base to obtain the corresponding salt,
k) a reaction for resolution of the racemic forms into resolved products,
the said products of formula (I) thus obtained being in any of the possible racemic, enantiomeric and diastereoisomeric isomer forms.

3. Process for deprotecting the hydroxyl functions of a product of formula (IV) as defined in Claim 2: 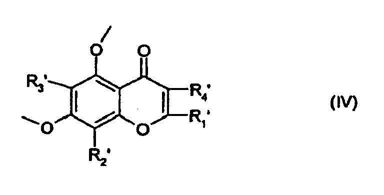 in which R1', R2', R3' and R4' have the meanings given in Claim 2,
**characterized in that** the product of formula (IV) is subjected either to the action of hydriodic acid supported on a resin (for instance Reillex-pyridine-TM-402 polymer) in an anhydrous polar solvent,
or to the action of a hydriodic acid salt such as HI-Py in an anhydrous polar solvent,
or to the action of concentrated hydriodic acid, to obtain a product of formula (I'): 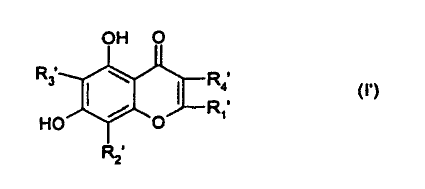 in which R1' , R2', R3' and R4' have the meanings given in Claim 2,
which products of formula (I') may be products of formula (I) and which, in order to obtain products or other products of formula (I), may be subjected, if desired and if necessary, to one or more of the following conversion reactions, in any order:
a) a reaction for esterification of the acid function,
b) a reaction for saponification of the ester function to an acid function,
c) a reaction for oxidation of the alkylthio group to a corresponding sulfoxide or sulfone group,
d) a reaction for conversion of the ketone function to an oxime function,
e) a reaction for reduction of the free or esterified carboxyl function to an alcohol function,
f) a reaction for conversion of the alkoxy function to a hydroxyl function, or of the hydroxyl function to an alkoxy function,
g) a reaction for oxidation of the alcohol function to an aldehyde, acid or ketone function,
h) a reaction for conversion of the nitrile radical to tetrazolyl,
i) a reaction for removal of the protecting groups possibly borne by the protected reactive functions,
j) a reaction for salification with a mineral or organic acid or with a base to obtain the corresponding salt,
k) a reaction for resolution of the racemic forms into resolved products,
the said products of formula (I) thus obtained being in any of the possible racemic, enantiomeric and diastereoisomeric isomer forms.

4. As medicaments, the products of formula (I) as defined in Claim 1, and also the addition salts with pharmaceutically acceptable mineral and organic acids or with pharmaceutically acceptable mineral and organic bases of the said products of formula (I).

5. Pharmaceutical compositions containing, as active principle, at least one of the medicaments as defined in Claim 4.

6. Pharmaceutical compositions according to Claim 5, **characterized in that** they are used as antimitotic medicaments, in particular for cancer chemotherapy or for treating psoriasis, parasitoses such as those caused by fungi or protists, or Alzheimer's disease.

7. Pharmaceutical compositions according to Claim 5, **characterized in that** they are used as antineurodegenerative medicaments and especially as medicaments for combating neuronal apoptosis.

8. Use of the products of formula (I) as defined in Claim 1 for the preparation of medicaments for preventing or treating diseases associated with a deregulation of the secretion and/or activity of protein tyrosine kinases.

9. Use of the products of formula (I) as defined in Claim 1 for the preparation of medicaments for cancer chemotherapy, for treating psoriasis or parasitoses such as those caused by fungi or protists, for treating Alzheimer's disease or for treating neurodegenerative complaints, especially neuronal apoptosis.

10. As novel industrial products, the compounds of formula (IV): 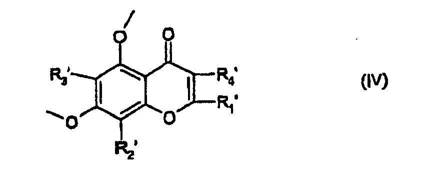 in which R1', R2', R3' and R4' have the meanings given in Claim 1 for R1, R2, R3 and R4, respectively, in which the optional reactive functions are optionally protected with protecting groups as defined in Claims 2 and 3.

## Patentansprüche

1. Produkte der Formel (I) 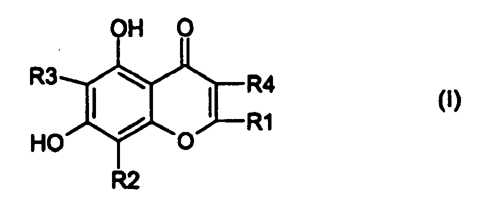 in der
R2 und R3 derart sind, daß eines ein Wasserstoffatom darstellt und das andere einen Rest Piperidinyl darstellt, gegebenenfalls substituiert durch einen oder mehrere Reste Hydroxyl und Alkyl, wobei R2 und R3 alternativ die gleichen Werte annehmen können, um die entsprechenden Isomere zu ergeben,
R4 ein Wasserstoffatom, einen Rest Alkyl oder Phenyl darstellt, gegebenenfalls substituiert durch ein oder mehrere Halogenatome, R1 einen Rest Phenyl darstellt, gegebenenfalls substituiert durch einen oder mehrere Reste, ausgewählt unter den Halogenatomen ; dem Rest Cyclohexyl ; Cyano ; Nitro ; Hydroxyl ; Carboxy, frei, in Salz überführt oder verestert ; Tetrazolyl ; NH2, -NH-(Alkyl) ; -N-(Alkyl)-(alkyl) ; SO2-NH-CO-NHR5, worin R5 einen Rest Alkyl oder Phenyl darstellt ; Phenyl ; Alkyl ; Alkoxy oder Phenoxy ; CF3 ; OCF3 ; Pyrazolinyl, das selbst gegebenenfalls substituiert ist durch einen oder mehrere Reste, ausgewählt unter den Resten Alkyl und den Halogenatomen, mit der Maßgabe, daß in den obengenannten Resten die Reste Alkyl und Alkoxy linear oder verzweigt sind und höchstens 4 Kohlenstoffatome umfassen,
**dadurch gekennzeichnet, daß** diese Produkte der Formel (I) die folgenden sind:
- Trifluoracetat von 2-(2-Chlor-4-fluorphenyl)-5,7-dihydroxy-8-[(3S,4R)-3-hydroxy-1-methyl-4-piperidinyl]-4H-benzopyran-4-on
- Trifluoracetat von 2-(4-Cyclohexylphenyl)-5,7-dihydroxy-8-[(3S,4R)-3-hydroxy-1-methyl-4-piperidinyl]-4H-benzopyran-4-on
- Trifluoracetat von 4-[5,7-Dihydroxy-8-[(3S,4R)-3-hydroxy-1-methyl-4-piperidinyl]-4-oxo-4H-benzopyran-2-yl]-benzonitril
- Trifluoracetat von 5,7-Dihydroxy-8-[(3S,4R)-3-hydroxy-1-methyl-4-piperidinyl]-2-[4-(1H-tetrazol-5-yl)-phenyl]-4H-benzopyran-4-on
- Trifluoracetat von 5,7-Dihydroxy-8-[(3S,4R)-3-hydroxy-1-methyl-4-piperidinyl]-2-[3-(phenoxy)-phenyl]-4H-benzopyran-4-on
- Trifluoracetat von 5,7-Dihydroxy-6-[(3S,4R)-3-hydroxy-1-methyl-4-piperidinyl]-2-[3-(phenoxy)-phenyl]-4H-benzopyran-4-on
- 5,7-Dihydroxy-8-[(3S,4R)-3-hydroxy-1-methyl-4-piperidinyl]-2-(3-nitrophenyl)-4H-benzopyran-4-on
- Trifluoracetat von 5,7-Dihydroxy-2-[4-fluor-3-(trifluormethyl)-phenyl]-8-(3S,4R)-3-hydroxy-1-methyl-4-piperidinyl]-4H-benzopyran-4-on
- Trifluoracetat von 4-{8-[(3S,4R)-3-Acetyloxy-1-methyl-4-piperidinyl]-5,7-dihydroxy-4-oxo-4H-benzopyran-2-yl}-2,5-dichlor-benzoesäure
- Hydrochlorid von 2,5-Dichlor-4-{5,7-dihydroxy-8-[(3S,4R)-3-hydroxy-1-methyl-4-piperidinyl]-4-oxo-4H-benzopyran-2-yl}-benzoesäure-methylester
- Trifluoracetat von 2-[4-(Diethylamino)-phenyl]-5,7-dihydroxy-8-[(3S,4R)-3-hydroxy-1-methyl-4-piperidinyl]-4H-benzopyran-4-on
- Trifluoracetat von 5,7-Dihydroxy-8-[(3S,4R)-3-hydroxy-1-methyl-4-piperidinyl]-2-[3-(trifluormethoxy)-phenyl]-4H-benzopyran-4-on
- Trifluoracetat von 2-[3,5-Bis-(Trifluormethyl)-phenyl]-5,7-dihydroxy-8-[(3S,4R)-3-hydroxy-1-methyl-4-piperidinyl]-4H-benzopyran-4-on
- Trifluoracetat von 5,7-Dihydroxy-8-[(3S,4R)-3-hydroxy-1-methyl-4-piperidinyl]-2-[4-(trifluormethoxy)-phenyl]-4H-benzopyran-4-on
- Trifluoracetat von 4-[5,7-Dihydroxy-8-[(3S,4R)-3-hydroxy-1-methyl-4-piperidinyl]-4-oxo-4H-benzopyran-2-yl]-benzoesäure
- Trifluoracetat von 2-{3-[(4-Brom-3,5-dimethyl-1H-pyrazol-1-yl)-methyl]-phenyl}-5,7-dihydroxy-8-[(3S,4R)-3-hydroxy-1-methyl-4-piperidinyl]-4H-benzopyran-4-on
- Trifluoracetat von 5,7-Dihydroxy-2-{3-[(3,5-dimethyl-1H-pyrazol-1-yl)-methyl]-phenyl}-8-[(3S,4R)-3-hydroxy-1-methyl-4-piperidinyl]-4H-benzopyran-4-on
- Trifluoracetat von 2-[2,5-Bis-(2,2,2-Trifluorethoxy)-phenyl]-5,7-dihydroxy-8-[(3S,4R)-3-hydroxy-1-methyl-4-piperidinyl]-4H-benzopyran-4-on
- Trifluoracetat von 2-(2-Chlorphenyl)-5,7-dihydroxy-6-[(3S,4R)-3-hydroxy-1-methyl-4-piperidinyl]-4H-benzopyran-4-on
wobei die genannten Produkte der Formel (I) in allen ihren möglichen racemischen isomeren Formen, Enantiomeren und Diastereoisomeren vorliegen können, sowie die Additionssalze der genannten Produkte der Formel (I) mit mineralischen und organischen Säuren oder mit mineralischen und organischen Basen.

2. Verfahren zur Herstellung der Produkte der Formel (I) wie in Anspruch 1 definiert, **dadurch gekennzeichnet, daß** man die Verbindung der Formel (II) 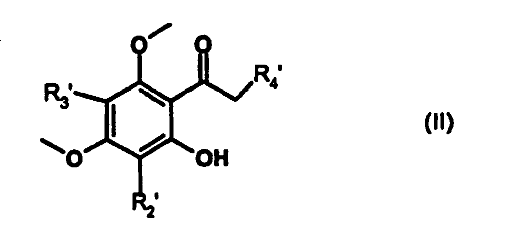 in der R2', R3' und R4' die in Anspruch 1 jeweils für R2, R3 und R4 angegebenen Bedeutungen besitzen, in denen die eventuellen reaktiven Funktionen gegebenenfalls durch Schutzgruppen geschützt sind, einer Reaktion mit einer Verbindung der Formel (III)
R1'COX (III)
unterzieht, in der R1' die in Anspruch 1 für R1 angegebene Bedeutung besitzt, worin die eventuellen reaktiven Funktionen gegebenenfalls durch Schutzgruppen geschützt sind und X ein Halogenatom oder einen Rest Alkoxy mit höchstens 6 Kohlenstoffatomen darstellt, um das Produkt der Formel (IV) 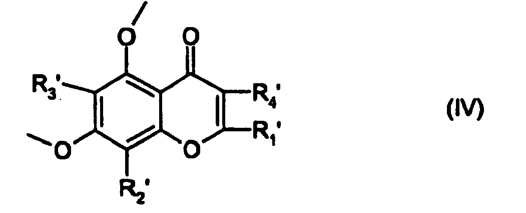 zu erhalten, in der R1', R2', R3' und R4' die oben angegebenen Bedeutungen besitzen,
und man das Produkt der Formel (IV) einer Reaktion zur Abspaltung der Schutzgruppen von den Hydroxylresten unterzieht, um ein Produkt der Formel (I') 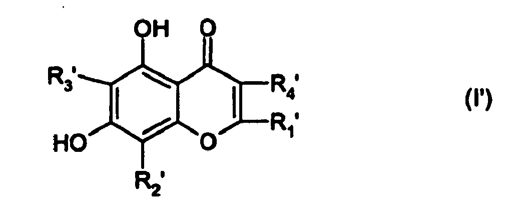 zu erhalten, in der R1', R2', R3' und R4' die oben angegebenen Bedeutungen besitzen,
und man die Produkte der Formel (I'), die Produkte der Formel (I) sein können, um diese oder andere Produkte der Formel (I) zu erhalten, wenn erwünscht und wenn erforderlich, einer oder mehreren der folgenden Umwandlungsreaktionen in irgendeiner Reihenfolge unterziehen kann:
a) einer Reaktion zur Veresterung der Säurefunktion,
b) einer Reaktion zur Verseifung der Esterfunktion zur Säurefunktion,
c) einer Reaktion zur Oxidation der Gruppe Alkylthio zum entsprechenden Sulfoxid oder Sulfon,
d) einer Reaktion zur Umwandlung der Ketonfunktion zur Oximfunktion,
e) einer Reaktion zur Reduktion der freien oder veresterten Carboxyfunktion zur Alkoholfunktion,
f) einer Reaktion zur Umwandlung der Alkoxyfunktion zur Hydroxylfunktion, oder auch der Hydroxylfunktion zur Alkoxyfunktion,
g) einer Reaktion zur Oxidation der Alkoholfunktion zur Aldehydfunktion, Säurefunktion oder Ketonfunktion,
h) einer Reaktion zur Umwandlung des Restes Nitril zum Rest Tetrazolyl,
i) einer Reaktion zur Entfernung der Schutzgruppen, welche die geschützten reaktiven Funktionen tragen können,
j) einer Reaktion zur Salzbildung durch eine Mineralsäure oder organische Säure oder durch eine Base, um das entsprechende Salz zu erhalten,
k) einer Reaktion zur Aufspaltung der racemischen Formen in die Spaltprodukte,
wobei die genannten Produkte der Formel (I), die auf diese Weise erhalten wurden, in allen ihren möglichen racemischen isomeren Formen, Enantiomeren und Diastereoisomeren vorliegen können.

3. Verfahren zum Abspalten der Schutzgruppen von den Hydroxylfunktionen bei einem Produkt der Formel (IV) wie in Anspruch 2 definiert: 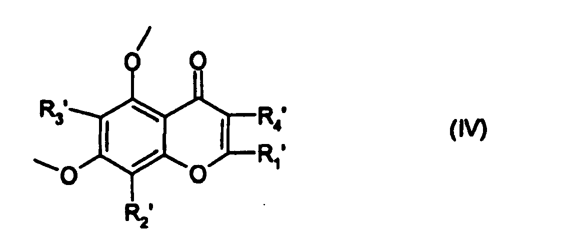 in der R1', R2', R3' und R4' die in Anspruch 2 angegebenen Bedeutungen besitzen,
**dadurch gekennzeichnet, daß** man das Produkt der Formel (IV) entweder der Einwirkung von Iodwasserstoffsäure, getragen von einem Harz (wie beispielsweise Reillex-Pyridin-TM-402 Polymer), in einem wasserfreien polaren Lösungsmittel,
oder der Einwirkung eines Salzes der Iodwasserstoffsäure wie HI-Py in einem wasserfreien polaren Lösungsmittel,
oder der Einwirkung von konzentrierter Iodwasserstoffsäure unterzieht, um ein Produkt der Formel (I') 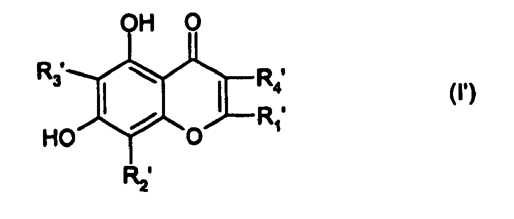 zu erhalten, in der R1', R2', R3' und R4' die in Anspruch 2 angegebenen Bedeutungen besitzen,
und man die Produkte der Formel (I'), die Produkte der Formel (I) sein können, um diese oder andere Produkte der Formel (I) zu erhalten, wenn erwünscht und wenn erforderlich, einer oder mehreren der folgenden Umwandlungsreaktionen in irgendeiner Reihenfolge unterziehen kann:
a) einer Reaktion zur Veresterung der Säurefunktion,
b) einer Reaktion zur Verseifung der Esterfunktion zur Säurefunktion,
c) einer Reaktion zur Oxidation der Gruppe Alkylthio zum entsprechenden Sulfoxid oder Sulfon,
d) einer Reaktion zur Umwandlung der Ketonfunktion zur Oximfunktion,
e) einer Reaktion zur Reduktion der freien oder veresterten Carboxyfunktion zur Alkoholfunktion,
f) einer Reaktion zur Umwandlung der Alkoxyfunktion zur Hydroxylfunktion, oder auch der Hydroxylfunktion zur Alkoxyfunktion,
g) einer Reaktion zur Oxidation der Alkoholfunktion zur Aldehydfunktion, Säurefunktion oder Ketonfunktion,
h) einer Reaktion zur Umwandlung des Restes Nitril zum Rest Tetrazolyl,
i) einer Reaktion zur Entfernung der Schutzgruppen, welche die geschützten reaktiven Funktionen tragen können,
j) einer Reaktion zur Salzbildung durch eine Mineralsäure oder organische Säure oder durch eine Base, um das entsprechende Salz zu erhalten,
k) einer Reaktion zur Aufspaltung der racemischen Formen in die Spaltprodukte,
wobei die genannten Produkte der Formel (I), die auf diese Weise erhalten wurden, in allen ihren möglichen racemischen isomeren Formen, Enantiomeren und Diastereoisomeren vorliegen können.

4. Als Arzneimittel die Produkte der Formel (I) wie in Anspruch 1 definiert sowie die Additionssalze der Produkte der Formel (I) mit pharmazeutisch akzeptablen Mineralsäuren und organischen Säuren oder mit Mineralbasen und organischen Basen.

5. Pharmazeutische Zusammensetzungen, enthaltend als Wirkstoff mindestens eines der Arzneimittel wie in Anspruch 4 definiert.

6. Pharmazeutische Zusammensetzungen nach Anspruch 5, **dadurch gekennzeichnet, daß** sie als antimitotische Arzneimittel, insbesondere bei der Chemotherapie von Krebszuständen, oder auch bei der Behandlung von Psoriasis, Parasitosen wie solchen, die mit Pilzen oder Protisten einhergehen oder der Alzheimer-Krankheit verwendet werden.

7. Pharmazeutische Zusammensetzungen nach Anspruch 5, **dadurch gekennzeichnet, daß** sie als antineurodegenerative, insbesondere als neuronale Anti-Apoptose-Arzneimittel verwendet werden.

8. Verwendung der Produkte der Formel (I) wie in Anspruch 1 definiert zur Herstellung von Arzneimitteln, die zur Vorbeugung oder Behandlung von Erkrankungen vorgesehen sind, die mit einer Unregelmäßigkeit der Sekretion und/oder der Aktivität von Tyrosin Kinasen Proteinen verbunden sind.

9. Verwendung der Produkte der Formel (I) wie in Anspruch 1 definiert zur Herstellung von Arzneimitteln, die zur Chemotherapie von Krebszuständen, zur Behandlung von Psoriasis, von Parasitosen wie solchen, die mit Pilzen oder Protisten einhergehen oder der Alzheimer-Krankheit, oder auch zur Behandlung von neurodegenerativen Erkrankungen, insbesondere neuronaler Apoptosis, vorgesehen sind.

10. Als neue industrielle Produkte die Verbindungen der Formel (IV) 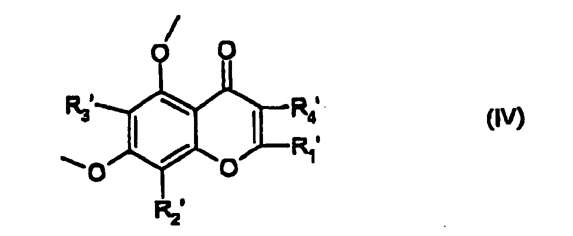 in der R1', R2', R3' und R4' die in Anspruch 1 jeweils für R1, R2, R3 und R4 angegebenen Bedeutungen besitzen, in denen die eventuellen reaktiven Funktionen gegebenenfalls durch Schutzgruppen geschützt sind, wie in Anspruch 2 und 3 definiert.
